(19) Europäisches Patentamt
European Patent Office
Office européen des brevets

(11) Veröffentlichungsnummer: **0 124 476**
**B1**

(12) # EUROPÄISCHE PATENTSCHRIFT

(45) Veröffentlichungstag der Patentschrift: **22.06.88**

(51) Int. Cl.⁴: **C 07 D 211/22**, C 07 D 211/18, C 07 D 211/32, C 07 D 409/06, A 61 K 31/445

(21) Anmeldenummer: **84810109.3**

(22) Anmeldetag: **05.03.84**

(54) **Carboxamide, Verfahren zur Herstellung und pharmazeutische Präparate, die diese enthalten.**

(30) Priorität: **09.03.83 CH 1258/83**
**18.07.83 CH 3925/83**

(43) Veröffentlichungstag der Anmeldung:
**07.11.84 Patentblatt 84/45**

(45) Bekanntmachung des Hinweises auf die Patenterteilung:
**22.06.88 Patentblatt 88/25**

(84) Benannte Vertragsstaaten:
**AT BE CH DE FR GB IT LI LU NL SE**

(56) Entgegenhaltungen:
**EP - A - 0 013 612**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Storni, Angelo, Dr., Im Feuerbusch 3, CH-4310 Rheinfelden (CH)**

**EP 0 124 476 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft pharmazeutische Präparate, enthaltend N-(Piperidinyl-alkyl)-carboxamide und deren Salze, Verfahren zu ihrer Herstellung, die Verwendung dieser Verbindungen, neue N-(Piperidinyl-alkyl)-carboxamide sowie Verfahren zu ihrer Herstellung.

Die Erfindung betrifft insbesondere pharmazeutische Präparate, enthaltend N-(Piperidinyl-alkyl)-carboxamide der allgemeinen Formel I

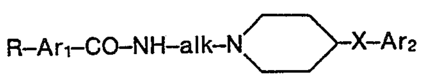

(I)

worin

R Hydroxy, Niederalkoxy, Niederalkenyloxy oder Halogen bedeutet, $Ar_1$ für jeweils unsubstituiertes oder ein- oder mehrfach zusätzlich durch Niederalkyl, Niederalkenyl, Niederalkadienyl, Halogenniederalkyl, Hydroxy, Niederalkanoyloxy, Halogen, Niederalkoxy, Niederalkenyloxy, Halogenniederalkoxy, Niederalkanoyl, Nitro, Cyano, Carbamoyl, N-Niederalkyl-carbamoyl, N,N-Diniederalkyl-carbamoyl, Carboxy, Niederalkoxycarbonyl, Amino, N-Niederalkyl-amino, N,N-Diniederalkyl-amino, Sulfamoyl, N-Niederalkylsulfamoyl, N,N-Diniederalkyl-sulfamoyl, Niederalkylthio, Halogenniederalkylthio, Niederalkansulfinyl, Halogenniederalkansulfinyl, Niederalkansulfonyl und/oder Halogenniederalkansulfonyl substituiertes Phenylen bzw. über ein C-Atom gebundenes, monocyclisches Azaarylen mit bis und mit 3 Stickstoffatomen steht, alk die beiden N-Atome durch 2 bis 3 C-Atome trennendes Alkylen mit 2 bis und mit 7 C-Atomen darstellt, X für Carbonyl, Diniederalkoxymethylen, Niederalkylendioxymethylen, Hydroxymethylen, Niederalkanoyloxymethylen oder Methylen steht, und $Ar_2$ einen jeweils unsubstituierten oder ein- oder mehrfach durch Niederalkyl, Niederalkenyl, Niederalkadienyl, Halogenniederalkyl, Hydroxy, Niederalkanoyloxy, Halogen, Niederalkoxy, Niederalkenyloxy, Halogenniederalkoxy, Niederalkanoyl, Nitro, Cyano, Carbamoyl, N-Niederalkyl-carbamoyl, N,N-Diniederalkyl-carbamoyl, Carboxy, Niederalkoxycarbonyl, Amino, N-Niederalkyl-amino, N,N-Diniederalkyl-amino, Sulfamoyl, N-Niederalkylsulfamoyl, N,N-Diniederalkyl-sulfamoyl, Niederalkylthio, Halogenniederalkylthio, Niederalkansulfinyl, Halogenniederalkansulfinyl, Niederalkansulfonyl und/oder Halogenniederalkansulfonyl substituierten Phenylrest bzw. jeweils monocyclischen Monooxa-, Monoaza- oder Monothiaarylrest darstellt, und ihre Salze,

Verbindungen der Formel I und ihre Salze zur Anwendung bei der prophylaktischen oder therapeutischen Behandlung des menschlichen oder tierischen Körpers,

neue Verbindungen der Formel I und ihre Salze, worin R, $Ar_1$, alk, X und $Ar_2$ die angegebenen Bedeutungen haben, mit der Massgabe, dass die Gruppierung R–$Ar_1$– von dem Rest der Formel Ia

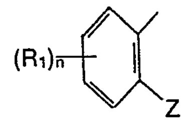

(Ia)

verschieden ist, worin $R_1$ ausgewählt ist aus der Gruppe bestehend aus Niederalkyl, Trifluormethyl, Halogen, Niederalkoxy, Nitro und Cyano und mindestens einer der Reste $R_1$ Halogen oder Niederalkoxy bedeutet, n für eine ganze Zahl von 1 bis 3 steht und Z Nitro, Amino oder Niederalkylamino darstellt, wenn alk und X die angegebenen Bedeutungen haben und $Ar_2$ unsubstituiertes Phenyl, Thienyl bzw. Pyridyl oder substituiertes Phenyl mit einem, zwei oder drei Substituenten ausgewählt aus der Gruppe bestehend aus Halogen, Niederalkyl, Niederalkoxy, Trifluormethyl und Amino bedeutet, wobei unter mit «Nieder» bezeichneten Resten solche zu verstehen sind, die bis und mit 7 C-Atome aufweisen, sowie Verfahren zu ihrer Herstellung.

Die Gruppe der ausgenommenen Verbindungen sind in EP-A-13 612 als Auszugsprodukte beschrieben.

Ein monocyclischer Arylenrest $Ar_1$ ist in erster Linie unsubstituiertes oder ein- oder mehrfach zusätzlich zu dem Rest R und der CO-Gruppe substituiertes Phenylen, insbesondere 1,2-Phenylen, ferner 1,3- oder 1,4-Phenylen.

Ein über ein C-Atom gebundenes, monocyclisches Azaarylen mit bis und mit 3 Stichstoffatomen ist in erster Linie unsubstituiertes oder ein- oder mehrfach zusätzlich substituiertes Pyridylen, insbesondere solches, worin die Carbamoylgruppe in Position 3 und der Rest R in Position 2 des Pyridylenrings gebunden ist, wie 2,3-Pyridylen, ferner 3,2-, 2,4-, 2,5-, 2,6-, 3,4- oder 3,5-Pyridylen. Im Phenylen- bzw. Pyridylenring ist bevorzugt die Carbamoylgruppe an ein dem Rest R benachbartes C-Atom gebunden.

Eine die beiden N-Atome durch 2 bis 3 C-Atome trennendes Alkylen mit 2 bis und mit 7 weist insbesondere 2 oder 3 C-Atome auf.

Ein monocyclischer Monooxa-, Monoaza- oder Monothiaarylrest $Ar_2$ bedeutet in erster Linie unsubstituiertes oder ein- oder mehrfach substituiertes Furyl, Pyridyl bzw. Thienyl.

Als Substituenten von Aryl- bzw. Heteroarylresten $Ar_2$ kommen beispielsweise die für $Ar_1$ genannten, in erster Linie Halogen, ferner Niederalkoxy, in Frage.

Die vor- und nachstehend verwendeten Allgemeindefinitionen haben, sofern nicht abweichend definiert, in erster Linie die folgenden Bedeutungen.

Der Ausdruck «nieder» bedeutet, dass entsprechend bezeichnete organische Gruppen oder Verbindungen bis und mit 7, vor allem bis und mit 4 Kohlenstoffatome, enthalten.

Halogen ist insbesondere Halogen mit Atomnummer bis und mit 35, wie Fluor, Chlor oder Brom, und umfasst ferner Iod.

Niederalkoxy ist z.B. Methoxy, Ethoxy, n-Propyloxy, Isopropyloxy, n-Butyloxy, Isobutyloxy, sek.-Butyloxy, tert.-Butyloxy und umfasst ferner entsprechende Pentyloxy-, Hexyloxy- und Heptyloxyreste.

Niederalkenyloxy ist z.B. Allyloxy oder But-2-en- oder But-3-enyloxy.

Niederalkyl ist z.B. Methyl, Ethyl, n-Propyl, Isopropyl, n-Butyl, Isobutyl, sek.-Butyl, tert.-Butyl und umfasst ferner entsprechende Pentyl-, Hexyl- und Heptylreste.

Niederalkenyl ist z.B. 2-Propenyl oder 1-, 2- oder 3-Butenyl und Niederalkadienyl z.B. Butadien-1,3-yl.

Halogenniederalkyl ist z.B. Trifluormethyl, 1,1,2-Trifluor-2-chlorethyl oder Chlormethyl.

Niederalkanoyloxy ist z.B. Acetyloxy, Proprionyloxy, Butyryloxy, Isobutyryloxy oder Pivaloyloxy.

Halogenniederalkoxy ist z.B. Difluormethoxy oder 1,1,2-Trifluor-2-chlor-ethoxy.

Niederalkanoyl ist z.B. Formyl, Acetyl, Propionyl, Butyryl, Isobutyryl oder Pivaloyl.

Niederalkoxycarbonyl ist z.B. Methoxy-, Ethoxy-, Propyloxy- oder Pivalyloxy-carbonyl.

N-Niederalkyl-carbamoyl ist z.B. N-Methyl-, N-Ethyl-, N-(n-Propyl)- oder N-Isopropyl-carbamoyl, während N,N-Diniederalkyl-carbamoyl z.B. N,N-Dimethyl- oder N,N-Diethyl-carbamoyl bedeutet.

N-Niederalkyl-amino ist z.B. N-Methyl-, N-Ethyl-, N-(n-Propyl)- und N-Isopropyl-amino, während N,N-Diniederalkyl-amino z.B. N,N-Dimethyl- oder N,N-Diethyl-amino darstellt.

N-Niederalkyl-sulfamoyl ist z.B. N-Methyl- oder N-Ethylsulfamoyl und N,N-Diniederalkyl-sulfamoyl z.B. N,N-Dimethyl- oder N,N-Diethylsulfamoyl.

Niederalkylthio ist z.B. Methyl-, Ethyl-, n-Propyl-, Isopropyl-, n-Butyl-, Isobutyl-, sek-Butyl- oder tert-Butylthio.

Halogenniederalkylthio ist z.B. Chlormethyl-, Trifluormethyl- oder 1,1,2-Trifluor-2-chlor-ethylthio.

Niederalkansulfinyl bzw. -sulfonyl ist z.B. Methan-, Ethan-, n-Propan- oder Isopropan-sulfinyl bzw. -sulfonyl.

Halogenniederalkansulfinyl bzw. -sulfonyl ist z.B. Chlormethan-, Trifluormethan- oder 1,1,2-Trifluor-2-chlor-ethan-sulfinyl bzw. -sulfonyl.

2- bis 3-gliedriges Alkylen mit 2 bis 7, vor allem 2 bis 3, C-Atomen ist z.B. Ethylen oder 1,3-Propylen, ferner 1,2-Propylen, sowie 2-Methyl-1,2-propylen.

Niederalkylendioxy ist z.B. Ethylendioxy oder 1,3-Propylendioxy. Thienyl ist z.B. 2-Thienyl, ferner 3-Thienyl und Furyl z.B. 2-Furyl, ferner 3-Furyl, während Pyridyl z.B. 2- oder 3-Pyridyl, ferner 4-Pyridyl bedeutet.

Salze von Verbindungen der Formel (I) sind vorzugsweise pharmazeutisch verwendbare Salze. Verbindungen der Formel (I) können infolge ihrer basischen Gruppe(n) z.B. Säureadditionssalze bilden. Diese werden beispielsweise mit starken anorganischen Säuren, wie Mineralsäuren, z.B. Schwefelsäure, Phosphorsäuren oder Halogenwasserstoffsäuren, oder mit starken organischen Säuren, wie Niederalkancarbonsäuren, z.B. Essigsäure, gegebenenfalls ungesättigten Dicarbonsäuren, z.B. Oxal-, Malon-, Malein- oder Fumarsäure, Hydroxycarbonsäuren, z.B. Weinsäure oder Citronensäure, oder Arylcarbonsäuren, z.B. Benzoesäure, gebildet. Ebenso sind für die Bildung von Säureadditionssalzen beispielsweise Sulfonsäuren, wie Niederalkansulfonsäuren, z.B. Methansulfonsäure, oder gegebenenfalls substituierte Benzolsulfonsäuren, z.B. p-Toluolsulfonsäure, geeignet. Umfasst sind ferner für pharmazeutische Verwendungen ungeeignete Salze, da diese beispielsweise für die Isolierung bzw. Reinigung von entsprechenden freien Verbindungen der Formel (I) sowie derer pharmazeutisch verwendbarer Salze verwendet werden können.

Die erfindungsgemässen Verbindungen können als Strukturisomere vorliegen. Weisen z.B. Verbindungen der Formel (I) chirale C-Atome auf, z.B. sofern X der Formel (I) für freies oder mit einer organischen Carbonsäure verestertes Hydroxymethylen steht, können sie z.B. als reine Enantiomere oder Enantiomerengemische, wie Racemate, und, sofern noch mindestens ein weiteres chirales Zentrum vorhanden ist, als Diastereomere oder Diastereomerengemische vorliegen.

Die Verbindungen der Formel (I) und ihre pharmazeutisch verwendbaren Salze können beispielsweise als Arzneimittel, z.B. als Antipsychotika, oder in einem Verfahren zur prophylaktischen oder therapeutischen Behandlung des menschlichen, ferner des tierischen, Körpers verwendet werden. So weisen die Verbindungen der Formel (I) insbesondere wertvolle pharmakologische Eigenschaften auf. Sie besitzen vor allem eine ausgeprägte antipsychotische Wirkung. Beispielsweise antagonisieren die Verbindungen der Formel (I), wie Untersuchungen analog B. Costall et al., Brain Research 123, 89–111 (1977) zeigen, ab einer Dosis von etwa 1 mg/kg bei intraperitonealer Verabreichung die durch Amphetamin bedingte Stereotypie der Ratte.

Wie Untersuchungen an der Ratte in Anlehnung an die Testordnung von P.C. Waldmeier, Experientia 36, 1092–4 (1980), im Dosisbereich von etwa 0,1 bis etwa 100 mg/kg bei intraperitonealer Applikation der Wirksubstanzen zeigen, besitzen die Verbindungen der Formel (I) eine augeprägte antidopaminerge Aktivität. Diese wird durch die Bildungsrate von Metaboliten des Neurotransmitters Dopamin (DA) ermittelt, da die Steigerung des DA-Umsatzes als Mass für die Dopamin-Rezeptorblockade gilt.

Die DA-Rezeptorblockade durch Verbindungen der Formel (I) kann auch direkt durch die in-vivo-Hemmung der [$^3$H] Spiperone-Bindung in der Hippocampus-Region der Ratte festgestellt werden. In dem Radiorezeptor-Assay gemäss S. Bischoff et al., European J. Pharmacol., 68, 305–315 (1980), wurde eine starke Dopamin-Rezeptorblockade-Wirkung in einem Dosisbereich von 0,01 bis 100 mg/kg nach intraperitonealer Verabreichung an der Ratte nachgewiesen.

Entsprechend können Verbindungen der Formel (I) und ihre Salze, z.B. infolge ihrer starken antidopaminergen Aktivität, beispielsweise als Antipsychotika, insbesondere mit Wirkung auf das hippocampale dopaminerge System, verwendet werden. Besonders vorteilhaft ist die Tatsache, dass bei Verwendung von Verbindungen der Formel (I) geringe oder keine extrapyramidale Nebenwirkungen zu beobachten sind.

Bei der Verwendung von Verbindungen der Formel I kann neben der therapeutischen bzw. prophylaktischen Anwendung auch die gewerbsmässige Herrichtung der Wirksubstanz eingeschlossen sein.

Die unter den Disclaimer fallenden Verbindungen werden in der EP-A-13 612 als Ausgangsstoffe beschrieben. Es werden keine Angaben hinsichtlich etwaiger pharmakologischer Eigenschaften gemacht.

Die Erfindung betrifft insbesondere pharmazeutische Präparate, enthaltend Verbindungen der Formel (I), worin R Hydroxy, Niederalkoxy, Niederalkenyloxy oder Halogen bedeutet, $Ar_1$ für jeweils unsubstituiertes oder ein- oder mehrfach zusätzlich durch
Niederalkyl, Niederalkenyl, Niederalkadienyl,
Halogenniederalkyl, Hydroxy, Niederalkanoyloxy,
Halogen, Niederalkoxy, Niederalkenyloxy,
Halogenniederalkoxy, Niederalkanoyl, Nitro,
Cyano, Carbamoyl, N-Niederalkyl-carbamoyl,
N,N-Diniederalkyl-carbamoyl, Amino,
N-Niederalkylamino, N,N-Diniederalkyl-amino,
Sulfamoyl, N-Niederalkyl-sulfamoyl,
N,N-Diniederalkylsulfamoyl, Niederalkylthio,
Halogenniederalkylthio, Niederalkansulfinyl,
Halogenniederalkansulfinyl, Niederalkansulfonyl
und/oder Halogenniederalkansulfonyl substituiertes Phenylen bzw. Pyridylen steht, alk die beiden N-Atome durch 2 bis 3 C-Atome trennendes Alkylen mit 2 bis und mit 7 C-Atomen darstellt, X für Carbonyl, Diniederalkoxymethylen, Niederalkylendioxymethylen, Hydroxymethylen, Niederalkanoyloxymethylen oder Methylen steht und $Ar_2$ einen jeweils unsubstituierten oder ein- oder mehrfach durch
Niederalkyl, Niederalkenyl, Halogenniederalkyl,
Hydroxy, Niederalkanoyloxy, Halogen,
Niederalkoxy, Niederalkenyloxy,
Halogenniederalkoxy, Niederalkanoyl, Nitro,
Cyano, Carbamoyl, N-Niederalkyl-carbamoyl,
N,N-Diniederalkylcarbamoyl, Amino,
N-Niederalkyl-amino, N,N-Diniederalkyl-amino,
Sulfamoyl, N-Niederalkyl-sulfamoyl,
N,N-Diniederalkylsulfamoyl, Niederalkylthio,
Halogenniederalkylthio, Niederalkansulfinyl,
Halogenniederalkansulfinyl, Niederalkansulfonyl
und/oder Halogenniederalkansulfonyl substituierten Phenyl-, Thienyl-, Furyl- bzw. Pyridylrest darstellt, und ihre Salze, die Verbindungen der Formel (I) und ihrer Salze zur Verwendung, neue Verbindungen der Formel (I) und ihre Salze, worin R, $Ar_1$, alk, X und $Ar_2$ die angegebenen Bedeutungen haben, mit der Massgabe, dass die Gruppierung $R-Ar_1-$ von dem Rest der Formel (Ia) verschieden ist, worin $R_1$ ausgewählt ist aus der Gruppe bestehend aus Niederalkyl, Trifluormethyl, Halogen, Niederalkoxy, Nitro und Cyano und mindestens einer der Reste $R_1$ Halogen oder Niederalkoxy bedeutet, n für eine ganze Zahl von 1 bis 3 steht, und Z Nitro, Amino oder Niederalkylamino darstellt, wenn alk und X die angegebenen Bedeutungen haben und $Ar_2$ unsubstituiertes Phenyl, Thienyl bzw. Pyridyl oder substituiertes Phenyl mit einem, zwei oder drei Substituenten ausgewählt aus der Gruppe bestehend aus Niederalkyl, Trifluormethyl, Halogen, Niederalkoxy und Amino bedeutet, sowie Verfahren zu ihrer Herstellung.

Die Erfindung betrifft insbesondere pharmazeutische Präparate, enthaltend Verbindungen der Formel (I), worin R Niederalkoxy, z.B. mit bis und mit 4 C-Atomen, wie Methoxy, bedeutet, $Ar_1$ für unsubstituiertes oder jeweils ein- oder mehrfach zusätzlich durch Niederalkyl, z.B. mit bis und mit 4 C-Atomen, wie Methyl, Halogenniederalkyl, z.B. mit bis und mit 4 C-Atomen, wie Trifluormethyl, Halogen, z.B. mit Atomnummer bis und mit 35, wie Fluor, Chlor oder Brom, Niederalkoxy, z.B. mit bis und mit 4 C-Atomen, wie Methoxy, Cyano, Carbamoyl, Amino, N-Niederalkylamino, z.B. mit bis und mit 4 C-Atomen, wie N-Methylamino, N,N-Diniederalkylamino, z.B. jeweils mit bis und mit 4 C-Atomen im Niederalkylteil, wie N,N-Dimethylamino, Sulfamoyl, N,N-Diniederalkyl-sulfamoyl, z.B. mit bis und mit 4 C-Atomen jeweils im Alkylteil, wie N,N-Dimethylsulfamoyl, Halogenniederalkylthio, z.B. mit bis und mit 4 C-Atomen, wie Trifluormethylthio, Niederalkansulfonyl, z.B. mit bis und mit 4 C-Atomen, wie Methansulfonyl, und/oder Halogenniederalkansulfonyl, z.B. mit bis und mit 4 C-Atomen, wie Trifluormethansulfonyl, substituiertes Phenylen, z.B. 1,2-Phenylen, bzw. Pyridylen, z.B. 3,2-Pyridylen, steht, alk die beiden N-Atome durch 2 bis 3 C-Atome trennendes Alkylen mit 2 bis und mit 3 C-Atomen, wie Ethylen oder 1,3-Propylen, darstellt, X für Carbonyl, Hydroxymethylen oder Methylen steht, und $Ar_2$ durch Halogen, z.B. mit Atomnummer bis und mit 35, wie Fluor, substituiertes Phenyl oder unsubstituiertes Thienyl darstellt, und ihre Salze, die Verwendung dieser Verbindungen der Formel (I) und ihrer Salze, neue Verbindungen der Formel (I) und ihre Salze, worin R, $Ar_1$, alk, X und $Ar_2$ die angegebenen Bedeutungen haben, mit der Massgabe, dass die Gruppierung $R-Ar_1-$ von dem Rest der Formel (Ia) verschieden ist, worin $R_1$ ausgewählt ist aus der Gruppe bestehend aus Niederalkyl, Trifluormethyl, Halogen, Niederalkoxy und Cyano und mindestens einer der Reste $R_1$ Halogen oder Niederalkoxy bedeutet, n für eine ganze Zahl von 1 bis und mit 3 steht, und Z Amino oder Niederalkylamino darstellt, wenn alk und X die angegebenen Bedeutungen haben und $Ar_2$ unsubstituiertes Thienyl oder ein-, zwei- oder dreifach durch Halogen substituiertes Phenyl bedeutet, sowie Verfahren zu ihrer Herstellung.

Die Erfindung betrifft insbesondere pharmazeutische Präparate, enthaltend Verbindungen der Formel (I), worin einerseits R Niederalkoxy, insbesondere mit bis und mit 4 C-Atomen, wie Methoxy,

oder Halogen, insbesondere mit Atomnummer bis und mit 35, wie Fluor, bedeutet und $Ar_1$ für unsubstituiertes oder zusätzlich ein- oder mehrfach durch Niederalkyl, insbesondere mit bis und mit 4 C-Atomen, wie Methyl, Halogenniederalkyl, insbesondere mit Atomnummer bis und mit 35 und mit bis und mit 4 C-Atomen, wie Trifluormethyl, Hydroxy, Halogen, insbesondere mit Atomnummer bis und mit 35, wie Brom, Niederalkoxy, insbesondere mit bis und mit 4 C-Atomen, wie Methoxy, Cyano, Carbamoyl, Niederalkoxycarbonyl, insbesondere mit 2 bis und mit 5 C-Atomen, wie Methoxycarbonyl, Amino, Niederalkylamino, insbesondere mit bis und mit 4 C-Atomen, wie Methylamino, Diniederalkylamino, insbesondere jeweils mit bis und mit 4 C-Atomen im Niederalkylteil, wie Dimethylamino, Sulfamoyl, N,N-Diniederalkylsulfamoyl, insbesondere jeweils mit bis und mit 4 C-Atomen im Niederalkylteil, wie N,N-Dimethylsulfamoyl, Niederalkylthio, insbesondere mit bis und mit 4 C-Atomen, wie Methylthio, Niederalkansulfinyl, insbesondere mit bis und mit 4 C-Atomen, wie Methansulfinyl, und/oder Niederalkansulfonyl, insbesondere mit bis und mit 4 C-Atomen, wie Methansulfonyl, substituiertes Phenylen steht, oder worin andererseits R Niederalkoxy, insbesondere mit bis und mit 4 C-Atomen, wie Methoxy, bedeutet, und $Ar_1$ für unsubstituiertes oder durch Niederalkoxy, insbesondere mit bis und mit 4 C-Atomen, wie Methoxy, substituiertes Pyridylen, insbesondere 3,2-Pyridylen, steht, und jeweils alk die beiden N-Atome durch 2 bis 3 C-Atome trennendes Alkylen mit 2 bis und mit 7 C-Atomen darstellt, X für Carbonyl, Hydroxymethylen oder Methylen steht und $Ar_2$ durch Halogen, insbesondere mit Atomnummer bis und mit 35, wie Fluor, substituiertes Phenyl oder unsubstituiertes Thienyl darstellt, und ihre Salze, die Verwendung dieser Verbindungen der Formel I und ihrer Salze, neue Verbindungen der Formel (I) und ihre Salze, worin R, $Ar_1$, alk, X und $Ar_2$ die angegebenen Bedeutungen haben, mit der Massgabe, dass die Gruppierung $R{-}Ar_1{-}$ von dem Rest der Formel (Ia) verschieden ist, worin $R_1$ ausgewählt ist aus der Gruppe bestehend aus Niederalkyl, Trifluormethyl, Halogen, Niederalkoxy und Cyano und mindestens einer der Reste $R_1$ Halogen oder Niederalkoxy bedeutet, n für eine ganze Zahl von 1 bis und mit 3 steht, und Z Amino oder Niederalkylamino darstellt, wenn alk und X die angegebenen Bedeutungen haben und $Ar_2$ unsubstituiertes Thienyl oder ein-, zwei- oder dreifach durch Halogen substituiertes Phenyl bedeutet, sowie Verfahren zu ihrer Herstellung.

Die Erfindung betrifft vor allem pharmazeutische Präparate, enthaltend Verbindungen der Formel (I), worin R jeweils Niederalkoxy, z.B. mit bis und mit 4 C-Atomen, wie Methoxy bedeutet, $Ar_1$ einerseits für einen unsubstituierten oder ein- oder mehrfach zusätzlich durch Halogenniederalkyl, z.B. mit bis und mit 4 C-Atomen, wie Trifluormethyl, Halogen, z.B. mit Atomnummer bis und mit 35, wie Fluor, Chlor oder Brom, Niederalkoxy, z.B. mit bis und mit 4 C-Atomen, wie Methoxy, Cyano, Carbamoyl, N-Niederalkylamino, z.B. mit bis und mit 4 C-Atomen, wie Methylamino, Sulfamoyl, N,N-Diniederalkyl-sulfamoyl, z.B. mit bis und mit 4 C-Atomen, wie Dimethylsulfamoyl, Halogenniederalkylthio, z.B. mit bis und mit 4 C-Atomen, wie Trifluormethylthio, und/oder Halogenniederalkan-sulfonyl, z.B. mit bis und mit 4 C-Atomen, wie Trifluormethansulfonyl, substituierten Phenylrest, andererseits für einen ein- oder mehrfach zusätzlich durch Niederalkoxy, z.B. mit bis und mit 4 C-Atomen, wie Methoxy, und/oder Halogen, z.B. mit Atomnummer bis und mit 35, wie Chlor, substituierten Pyridylenrest, wie 3,2-Pyridylen, steht, alk Ethylen oder 1,3-Propylen darstellt, X für Carbonyl, Hydroxymethylen oder Methylen steht, und $Ar_2$ einen durch Halogen, z.B. bis und mit Atomnummer 35, wie Fluor, substituierten Phenylrest darstellt, und ihre Salze, die Verwendung dieser Verbindungen und ihrer Salze, neue Verbindungen der Formel (I) und ihrer Salze, worin R, $Ar_1$, alk, X und $Ar_2$ die angegebenen Bedeutungen haben, mit der Massgabe, dass die Gruppierung $R{-}Ar_1{-}$ von dem Rest der Formel (Ia) verschieden ist, worin $R_1$ ausgewählt ist aus der Gruppe bestehend aus Trifluormethyl, Halogen, Niederalkoxy und Cyano und mindestens einer der Reste $R_1$ Halogen oder Niederalkoxy bedeutet, n für eine ganze Zahl von 1 bis und mit 3 steht, und Z Niederalkylamino darstellt, wenn alk und X die angegebenen Bedeutungen haben und $Ar_2$ ein-, zwei- oder dreifach durch Halogen substituiertes Phenyl bedeutet, sowie Verfahren zu ihrer Herstellung.

Die Erfindung betrifft vor allem pharmazeutische Präparate, enthaltend Verbindungen der Formel (I), worin die Gruppierung $R{-}Ar_1$ für das Strukturelement der Formel Ib

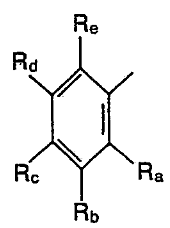

(Ib)

steht, in dem einer der Reste $R_a$ und $R_c$ den Rest R und dieser Niederalkoxy, insbesondere mit bis und mit 4 C-Atomen, wie Methoxy, oder Halogen, insbesondere mit Atomnummer bis und mit 35, wie Fluor, bedeutet und der andere Wasserstoff, Niederalkyl, insbesondere mit bis und mit 4 C-Atomen, wie Methyl, Halogen, insbesondere mit Atomnummer bis und mit 35, wie Brom, oder Niederalkylamino, insbesondere mit bis und mit 4 C-Atomen, wie Methylamino, darstellt, und die Reste $R_b$, $R_d$ und $R_e$ unabhängig voneinander für Wasserstoff, Niederalkyl, insbesondere mit bis und mit 4 C-Atomen, wie Methyl, Halogenniederalkyl, insbesondere mit Atomnummer bis und mit 35 und mit bis und mit 4 C-Atomen, wie Trifluormethyl, Hydroxy, Halogen, insbesondere mit Atomnummer bis und mit 35, wie Chlor, Niederalkoxy, insbesondere mit bis und mit 4 C-Atomen, wie Methoxy, Cyano, Carbamoyl, Niederalkoxycarbonyl, insbesondere mit 2 bis und mit 5 C-Atomen,

wie Methoxycarbonyl, Amino, Sulfamoyl, N,N-Diniederalkylsulfamoyl, insbesondere mit bis und mit 4 C-Atomen jeweils im Alkylteil, wie Dimethylsulfamoyl, Niederalkylthio, insbesondere mit bis und mit 4 C-Atomen, wie Methylthio, Niederalkansulfinyl, insbesondere mit bis und mit 4 C-Atomen, wie Methansulfinyl, und/oder Niederalkansulfonyl, insbesondere mit bis und mit 4 C-Atomen, wie Methansulfonyl, bedeuten, und worin alk für die beiden N-Atome durch 2 bis 3 C-Atome trennendes Alkylen mit 2 bis und mit 4 C-Atomen steht, X für Carbonyl, Hydroxymethylen oder Methylen steht und $Ar_2$ durch Halogen, insbesondere mit Atomnummer bis und mit 35, wie Fluor, substituiertes Phenyl oder unsubstituiertes Thienyl darstellt, und ihre Salze, die Verwendung dieser Verbindungen und ihrer Salze, neue Verbindungen der Formel (I) und ihre Salze, worin R, $Ar_1$, alk, X und $Ar_2$ die angegebenen Bedeutungen haben, mit der Massgabe, dass die Gruppierung R–$Ar_1$– von dem Rest der Formel (Ib) verschieden ist, worin $R_a$ oder $R_e$ Amino oder Niederalkylamino bedeutet und einer, zwei oder drei der verbleibenden Reste $R_a$, $R_b$, $R_c$, $R_d$ und $R_e$ ausgewählt sind aus der Gruppe bestehend aus Niederalkyl, Trifluormethyl, Halogen, Niederalkoxy und Cyano und die anderen Wasserstoff sind, wobei mindestens einer dieser Reste Halogen oder Niederalkoxy darstellt, wenn alk und X die angegebenen Bedeutungen haben und $Ar_2$ unsubstituiertes Thienyl oder ein-, zwei- oder dreifach durch Halogen substituiertes Phenyl bedeutet, sowie Verfahren zu ihrer Herstellung.

Die Erfindung betrifft vor allem pharmazeutische Präparate, enthaltend Verbindungen der Formel (I), worin die Gruppierung R–$Ar_1$– für das Strukturelement der Formel Ib steht, in dem einerseits der Rest $R_a$ den Rest R und dieser Niederalkoxy mit bis und mit 4 C-Atomen, wie Methoxy, bedeutet und $R_c$ Wasserstoff, Halogen mit Atomnummer bis und mit 35, wie Fluor, oder Niederalkylamino mit bis und mit 4 C-Atomen, wie Methylamino, darstellt, bzw. $R_a$ Wasserstoff ist und $R_c$ den Rest R und dieser Niederalkoxy mit bis und mit 4 C-Atomen bedeutet, oder in dem andererseits der Rest $R_c$ den Rest R und dieser Halogen mit Atomnummer bis und mit 35, wie Fluor, bedeutet und $R_a$ Wasserstoff oder Halogen mit Atomnummer bis und mit 35, wie Brom, darstellt und einer der Reste $R_b$ und $R_d$ für Wasserstoff, Niederalkyl mit bis und mit 4 C-Atomen, wie Methyl, Halogenniederalkyl mit Atomnummer bis und mit 35 und mit bis und mit 4 C-Atomen, wie Trifluormethyl, Halogen mit Atomnummer bis und mit 35, wie Brom, Niederalkoxy mit bis und mit 4 C-Atomen, wie Methoxy, Cyano, Carbamoyl, Niederalkoxycarbonyl mit 2 bis und mit 5 C-Atomen, wie Methoxycarbonyl, Niederalkylthio mit bis und mit 4 C-Atomen, wie Methylthio, Niederalkansulfinyl mit bis und mit 4 C-Atomen, wie Methansulfinyl, oder Niederalkansulfonyl mit bis und mit 4 C-Atomen, wie Methansulfonyl, steht, und der andere sowie $R_e$ Wasserstoff sind, und worin jeweils alk für die beiden N-Atome durch 2 bis 3 C-Atome trennendes Alkylen mit 2 bis und mit 4 C-Atomen steht, wie Ethylen, mit 2 bis und mit 4 C-Atomen steht, X für Carbonyl oder Hydroxymethylen steht und $Ar_2$ in p-Stellung durch Halogen mit Atomnummer bis und mit 35, wie Fluor, substituiertes Phenyl darstellt, und ihre Salze, die Verwendung dieser Verbindungen und ihrer Salze, neue Verbindungen der Formel (I) und ihre Salze, worin R, $Ar_1$, alk, X und $Ar_2$ die angegebenen Bedeutungen haben, sowie Verfahren zu ihrer Herstellung.

Die Erfindung betrifft vor allem pharmazeutische Präparate, enthaltend Verbindungen der Formel (I), worin $Ar_1$ einerseits ein- oder mehrfach zusätzlich durch Halogen mit Atomnummer bis und mit 35, wie Chlor, Cyano und/oder N-Niederalkylamino mit bis und mit 4 C-Atomen im Niederalkylteil, wie Methylamino, substituiertes Phenylen, wie 1,2-Phenylen, bedeutet oder andererseits Pyridylen, wie 3,2-Pyridylen, darstellt, und jeweils R Niederalkoxy bis und mit 4 C-Atomen, wie Methoxy, bedeutet, alk Ethylen ist, X Carbonyl darstellt, und $Ar_2$ für durch Halogen mit Atomnummer bis und mit 35, wie Fluor, substituiertes Phenyl steht, und ihre Salze, die Verwendung dieser Verbindungen der Formel (I) und ihrer Salze, neue Verbindungen der Formel I und ihre Salze, worin R, $Ar_1$, alk, X und $Ar_2$ die angegebenen Bedeutungen haben, mit der Massgabe, dass die Gruppierung R–$Ar_1$– von dem Rest der Formel (Ia) verschieden ist, worin $R_1$ ausgewählt ist aus der Gruppe bestehend aus Halogen mit Atomnummer bis und mit 35 und Niederalkoxy mit bis und mit 4 C-Atomen, n für eine ganze Zahl und 1 bis und mit 3 steht, und Z Niederalkylamin mit bis und mit 4 C-Atomen darstellt, sowie Verfahren zu ihrer Herstellung.

Die Erfindung betrifft in erster Linie pharmazeutische Präparate, enthaltend Verbindungen der Formel (I), worin R Niederalkoxy mit bis und mit 4 C-Atomen, wie Methoxy, bedeutet, $Ar_1$ für zusätzlich durch Cyano, insbesondere in p-Stellung zu R, substituiertes 1,2-Phenylen steht, alk Ethylen darstellt, X für Carbonyl steht, und $Ar_2$ durch Halogen mit Atomnummer bis und mit 35, wie Fluor, insbesondere in p-Stellung, substituiertes Phenyl darstellt, und ihre Salze, die Verwendung dieser Verbindungen und ihrer Salze, neue Verbindungen der Formel (I) und ihre Salze, worin R, $Ar_1$, alk, X und $Ar_2$ die angegebenen Bedeutungen haben, sowie Verfahren zu ihrer Herstellung.

Die Erfindung betrifft in erster Linie pharmazeutische Präparate, enthaltend Verbindungen der Formel (I), worin die Gruppierung R–$Ar_1$– für das Strukturelement der Formel Ib steht, in dem einerseits $R_a$ den Rest R und dieser Niederalkoxy mit bis und mit 4 C-Atomen, wie Methoxy, bedeutet, $R_b$ und $R_e$ Wasserstoff sind, $R_c$ Niederalkylamino mit bis und mit 4 C-Atomen, wie Methylamino, darstellt und $R_d$ Halogen mit Atomnummer bis und mit 35, wie Chlor, bedeutet oder $R_c$ Wasserstoff oder Halogen mit Atomnummer bis und mit 35, wie Chlor, darstellt und $R_d$ Cyano ist, oder in dem andererseits $R_a$, $R_d$ und $R_e$ Wasserstoff sind, $R_b$ Halogen mit Atomnummer bis und mit 35, wie Brom, und $R_c$ den Rest R und dieser Niederalkoxy mit bis und mit 4 C-Atomen, wie Methoxy, darstellt, und jeweils alk Ethylen bedeutet, X für Carbonyl oder ferner Hydroxymethylen steht, und $Ar_2$ 4-Fluorphenyl darstellt, und ihre Salze, die Verwendung dieser Verbindungen und ihrer Salze, neue Verbindungen der Formel (I) und ihre Salze, worin R, $Ar_1$, alk, X und $Ar_2$ die angegebenen Bedeutungen haben, sowie Verfahren zu ihrer Herstellung.

Die Erfindung betrifft in erster Linie pharmazeutische Präparate, enthaltend Verbindungen der Formel (I), worin die Gruppierung R–$Ar_1$– für das Strukturelement der Formel Ib steht, in dem einer der Reste $R_a$ und $R_b$ Wasserstoff oder Halogen mit Atomnummer bis und mit 35, wie Brom, darstellt, und der andere Wasserstoff bedeutet, $R_c$ den Rest R und dieser Halogen mit Atomnummer bis und mit 35, wie Fluor, darstellt und $R_d$ und $R_e$ jeweils Wasserstoff sind, und alk Ethylen ist, X für Carbonyl steht und $Ar_2$ p-Fluorphenyl bedeutet, und ihre Salze, die Verwendung dieser Verbindungen und ihrer Salze, neue Verbindungen der Formel (I) und ihre Salze, worin R, $Ar_1$, alk, X und $Ar_2$ die angegebenen Bedeutungen haben, sowie Verfahren zu ihrer Herstellung.

Die Erfindung betrifft in allererster Linie pharmazeutische Präparate, enthaltend Verbindungen der Formel I, worin die Gruppierung R–$Ar_1$– für das Strukturelement der Formel Ib steht, in dem $R_a$, $R_d$ und $R_e$ Wasserstoff sind, $R_b$ Wasserstoff oder Halogen mit Atomnummer bis und mit 35, wie Brom, bedeutet, und $R_c$ den Rest R und dieser Halogen mit Atomnummer bis und mit 35, wie Fluor, darstellt, alk Ethylen bedeutet, X für Carbonyl steht und $Ar_2$ 4-Fluorphenyl darstellt, und ihre Salze, die Verwendung dieser Verbindungen und ihrer Salze, neue Verbindungen der Formel (I) und ihre Salze, worin R, $Ar_1$, alk, X und $Ar_2$ die angegebenen Bedeutungen haben, sowie Verfahren zu ihrer Herstellung.

Die Erfindung betrifft in allererster Linie pharmazeutische Präparate, enthaltend Verbindungen der Formel I, worin die Gruppierung R–$Ar_1$– für das Strukturelement der Formel Ib steht, in dem $R_a$ den Rest R und dieser Niederalkoxy mit bis und mit 4 C-Atomen, wie Methoxy, bedeutet, $R_b$, $R_c$ und $R_e$ Wasserstoff darstellen und $R_d$ Cyano ist, alk Ethylen bedeutet, X für Carbonyl steht und $Ar_2$ 4-Fluorphenyl darstellt, und ihre Salze, die Verwendung dieser Verbindungen und ihrer Salze, neue Verbindungen der Formel (I) und ihre Salze, worin R, $Ar_1$, alk, X und $Ar_2$ die angegebenen Bedeutungen haben, sowie Verfahren zu ihrer Herstellung.

Die Erfindung betrifft insbesondere die in den Beispielen genannten Verbindungen, ihre Salze und Isomere.

Die Verbindungen der Formel (I) und ihre Salze werden nach an sich bekannten Methoden hergestellt, beispielsweise indem man

a) eine Verbindung der Formel IIa

$$R–Ar_1–X_1 \qquad (IIa)$$

worin $X_1$ Carboxy oder reaktionsfähiges funktionell abgewandeltes Carboxy bedeutet, mit einer Verbindung der Formel IIb

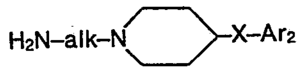

(IIb)

oder einem Salz davon kondensiert, oder

b) Verbindungen der Formeln IIIa und IIIb

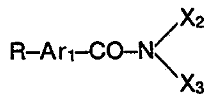

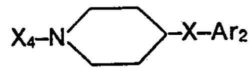

(IIIa) (IIIb)

worin $X_2$ für Wasserstoff steht, einer der Reste $X_3$ und $X_4$ Wasserstoff bedeutet und der andere Rest für eine Gruppe der Formel –alk–$X_5$ steht und $X_5$ reaktionsfähiges verestertes Hydroxy darstellt, oder Verbindungen der Formeln (IIIa) und (IIIb), worin $X_2$ und $X_3$ gemeinsam für alk′ stehen, wobei alk′ 2- bis 3-gliedriges Alkylen mit 2 bis und mit 7 C-Atomen darstellt, und $X_4$ Wasserstoff ist, oder deren Salze intermolekular kondensiert, oder eine Verbindung der Formel IIIc

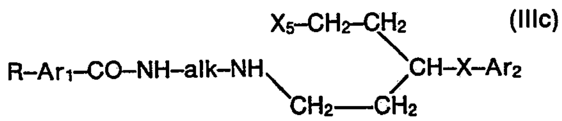

(IIIc)

worin $X_5$ reaktionsfähiges verestertes Hydroxy bedeutet, intramolekular kondensiert, oder

d) eine Verbindung der Formel IVa

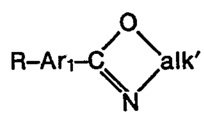

(IVa)

worin alk′ eine das C-Atom und das N-Atom durch 2 bis 3 C-Atome trennende Alkylengruppe mit 2 bis und mit 7 C-Atomen darstellt, mit einer Verbindung der Formel IVb

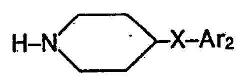

(IVb)

umsetzt, oder

e) in einer Verbindung der Formel V

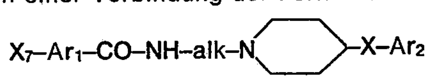

(V)

oder einem Salz davon, worin $X_7$ einen in R überführbaren Rest bedeutet, $X_7$ in R überführt, und gewünschtenfalls eine verfahrensgemäss oder auf andere Weise erhältliche Verbindung in eine andere erfindungsgemässe Verbindung überführt, und/oder ein verfahrensgemäss erhältliches Salz in die freie Verbindung oder in ein anderes Salz überführt, und/oder eine verfahrensgemäss erhältliche freie Verbindung mit salzbildenden Eigenschaften in ein Salz überführt und/oder ein verfahrensgemäss erhältliches Isomerengemisch in die einzelnen Isomeren auftrennt.

Zu Variante a):

Reaktionsfähiges funktionell abgewandeltes Carboxy $X_1$ bedeutet beispielsweise verestertes, in erster Linie reaktionsfähiges verestertes, Carboxy, anhydridisiertes Carboxy oder amidiertes Carboxy.

Verestertes Carboxy ist beispielsweise gegebenenfalls substituiertes Niederalkoxycarbonyl, wie Ethoxycarbonyl, vorzugsweise jedoch reaktionsfähiges verestertes Carboxy, beispielsweise gegebenenfalls, z. B. durch Niederalkoxy oder gegebenenfalls substituiertes Carbamoyl, zusätzlich aktiviertes Vinyloxycarbonyl, wie 1-Niederalkoxy-, z. B. 1-Ethoxyvinyloxycarbonyl, oder 2-(N-Niederalkyl-carbamoyl)-, z. B. 2-(N-Ethylcarbamoyl)-vinyloxycarbonyl, sowie gegebenenfalls, z. B. durch Nitro, Halogen, Niederalkansulfonyl oder Phenylazo, substituiertes Phenoxy- bzw. Thiophenoxycarbonyl, wie 4-Nitro-, 2,4,5-Trichlor-, Pentachlor-, 4-Methansulfonyl-, 4-Phenylazo-phenoxycarbonyl, Thiophenoxy- oder 4-Nitrothiophenoxycarbonyl, und ebenso aktiviertes, z. B. durch Cyano oder ferner gegebenenfalls verestertes Carboxy substituiertes Methoxycarbonyl, insbesondere Cyanomethoxycarbonyl. Reaktionsfähiges verestertes Carboxy kann ebenso 1,1- oder 1,3-disubstituiertes 2-Isoureidocarbonyl, wie 1,1-Diniederalkyl-, 1,1-Diaryl- oder 1,1-Diarylniederalkyl-2-isoureidocarbonyl, z. B. 1,1-Diethyl-, 1,1-Diphenyl- oder 1,1-Dibenzyl-2-isoureidocarbonyl, oder 1,3-Dicycloalkyl-, z. B. 1,3-Dicyclohexyl-2-isoureido-carbonyl, oder N-Alkylenaminooxycarbonyl, wie N-Piperidinyl-oxycarbonyl, sowie N-Imido-oxycarbonyl, z. B. N-Succinimido-oxy- oder N-Phthalimido-oxycarbonyl, sein.

Unter anhydridisiertem Carboxy ist beispielsweise gegebenenfalls verzweigtes Niederalkoxycarbonyloxycarbonyl, wie Ethoxy- oder Isobutyloxycarbonyloxycarbonyl, Halogencarbonyl, wie Chlorcarbonyl, Azidocarbonyl, Halogenphosphoryloxycarbonyl, wie Dichlorphosphoryloxycarbonyl, oder gegebenenfalls, z. B. durch Halogen oder Aryl, substituiertes Niederalkanoyloxycarbonyl, wie Pivaloyloxy-, Trifluoracetyloxy- oder Phenylacetoxycarbonyl, zu verstehen. Anhydridisiertes Carboxy kann ferner symmetrisch anhydridisiertes Carboxy der Formel R–$Ar_1$–CO–O–CO– sein.

Reaktionsfähiges amidiertes Carboxy ist beispielsweise gegebenenfalls, z. B. durch Niederalkyl, substituiertes 1-Imidazolyl- oder 1-Pyrazolylcarbonyl, wie 3,5-Dimethyl-pyrazolylcarbonyl.

Die verfahrensgemässe Kondensation (N-Acylierung) wird in an sich bekannter Weise durchgeführt, erforderlichenfalls in Gegenwart eines, insbesondere basischen, Kondensationsmittels. Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide, sowie carbocyclische Amine in Frage. Beispielhaft seien Natrium-hydroxid, -hydrid, -amid, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, oder Ethyl-diisopropylamin, N-Methyl-piperidin, Pyridin, Benzyltrimethylammoniumhydroxid, 1,5-Diazabicyclo[4.3.0]non-5-en (DBN) sowie 1,8-Diaza-bicyclo[5.4.0]undec-7-en (DBU) genannt, ferner Phosphine, wie Triphenylphosphin, oder Halogensilane, wie Tetrachlorsilan. Falls $X_1$ Carboxy bedeutet, werden primär die entsprechenden Ammoniumsalze gebildet, die durch Erwärmen oder Behandeln mit geeigneten Dehydratisierungsmitteln, wie Carbodiimiden, z. B. N,N′-Diniederalkyl- oder N,N′-Dicycloalkylcarbodiimid, wie N,N′-Diethyl-, N,N′-Diisopropyl- oder N,N′-Dicyclohexyl-carbodiimid, vorteilhaft unter Zusatz von N-Hydroxysuccinimid oder gegebenenfalls, z. B. durch Halogen, Niederalkoxy oder Niederalkyl, substituiertem 1-Hydroxy-benzotriazol oder N-Hydroxy-5-norbornen-2,3-dicarboxamid, sowie N,N-Carbonyldiimidazol, dehydratisiert werden können. Mit Carbodiimiden können intermediär z. B. auch die entsprechenden 1-Isoureidocarbonyl-Verbindungen gebildet werden. Als wasserbindende Kondensationsmittel können weiterhin N-Etoxycarbonyl-2-ethoxy-1,2-dihydrochinolin, Phosphorylcyanamide bzw. -azide, wie Diethylphosphorylcyanamid oder Diphenylphosphorylazid, Triphenylphosphin-disulfid oder 1-Niederalkyl-2-halogeno-piperidinium-halogenide, wie 1-Methyl-2-chlor-pyridinium-iodid, eingesetzt werden.

Die N-Acylierung wird in an sich bekannter Weise durchgeführt, beispielsweise in Ab- oder, üblicherweise, Anwesenheit eines geeigneten Lösungs- oder Verdünnungsmittels oder eines entsprechenden Gemisches, unter Kühlen, bei Raumtemperatur oder unter Erwärmen, z. B. in einem Temperaturbereich von −20 °C bis 150 °C, vorzugsweise von 0 °C bis 100 °C, und erforderlichenfalls in einem geschlossenen Gefäss, gegebenenfalls unter Druck und/oder in einer Inertgasatmosphäre.

Die in dieser Verfahrensvariante verwendeten Ausgangsstoffe sind z. T. bekannt bzw. können nach an sich bekannten Verfahren hergestellt werden.

Zur Herstellung von Verbindungen der Formel (IIa), worin $X_1$ gegebenenfalls substituiertes Niederalkoxycarbonyl bedeutet, kann man üblicherweise von der freien Säure ($X_1$ = Carboxy) oder einem Säureanhydrid ($X_1$ bedeutet z. B. Halogencarbonyl) ausgehen und setzt beispielsweise mit dem entsprechenden, erforderlichenfalls in reaktionsfähiger Form vorliegendem, Alkohol, z. B. einem Niederalkylhalogenid, um. Die Herstellung von Verbindungen der Formel (IIa), worin $X_1$ gegebenenfalls zusätzlich aktiviertes Vinyloxycarbonyl bedeutet, kann z. B. durch Umesterung eines Niederalkylesters mit Vinylacetat (Methode des aktivierten Vinylesters), durch Umsetzung der freien Säure von Verbindungen der Formel (IIa) mit Niederalkoxyacetylen (z. B. Ethoxyacetylen-Methode) oder, analog der Woodward-Methode, mit einem 1,2-Oxazoliumsalz erfolgen. Gegebenenfalls substituiertes Phenoxy- bzw. Thiophenoxycarbonyl aufweisende Verbindungen der Formel (IIa) können beispielsweise ausgehend von der freien Säure nach der Carbodiimid-Methode durch Umsetzen mit dem entsprechenden (Thio-)Phenol durchgeführt werden. Ebenfalls ausgehend von

der freien Säure der Formel (IIa) können Verbindungen der Formel (IIa), worin $X_1$ aktiviertes Methoxycarbonyl bzw. 1,1- oder 1,3-disubstituiertes 2-Isoureidocarbonyl darstellt, z. B. durch Umsetzung mit einem Halogen-, wie Chloracetonitril (Cyanmethylester-Methode) bzw. mit einem Carbodiimid oder Cyanamid (Carbodiimid- oder Cyamid-Methode) erhalten werden. Die Herstellung von N-Alkylenaminooxycarbonyl- bzw. N-Imidooxycarbonyl-Verbindungen der Formel (IIa) kann beispielsweise bei Verwendung der freien Säure der Formel (IIa) aus entsprechenden N-Hydroxyverbindungen mit Hilfe von Carbodiimiden nach der Methode der aktivierten N-Hydroxyester erfolgen. Zur Herstellung von Verbindungen der Formel (IIa), worin $X_1$ gegebenenfalls verzweigtes Niederalkoxycarbonyloxycarbonyl, Halogenphosphoryloxycarbonyl bzw. gegebenenfalls substituiertes Niederalkanoyloxycarbonyl bedeutet, kann beispielsweise von freier Säure der Formel (IIa) ausgegangen werden, die z. B. mit einem entsprechenden Halogenid, wie gegebenenfalls substituiertes Niederalkylkohlensäurehalogenid (Methode der gemischten O-Kohlensäureanhydride), Phosphoroxyhalogenid (z. B. Phosphoroxychlorid-Methode) oder gegebenenfalls substituiertes Niederalkanoylhalogenid (Methode der gemischten Carbonsäurehalogenide) behandelt werden kann. Azidocarbonylverbindungen der Formel (IIa) sind beispielsweise durch Behandeln entsprechender Hydrazide mit salpetriger Säure zugänglich (Azid-Methode). Zur Herstellung von Verbindungen der Formel (IIa), worin $X_1$ gegebenenfalls substituiertes 1-Imidazolyl-carbonyl bzw. 1-Pyrazolyl-carbonyl bedeutet, setzt man die freie Säure der Formel (IIa) z. B. mit Di-(1-imidazolyl)-carbonyl (Imidazolid-Methode) bzw. das betreffende Hydrazid z. B. mit einem entsprechenden 1,3-Diketon (Pyrazolid-Methode) um.

Das Ausgangsmaterial der Formel (IIb), worin X für die Carbonylgruppe steht, kann beispielsweise erhalten werden, indem man eine Verbindung der Formel $Ar_2$–H (IIc) mit der Piperidin-4-carbonsäure oder einem reaktionsfähigen Derivat davon analog einer Friedel-Crafts-Acylierung in Gegenwart einer Lewissäure acyliert. Die so erhältliche Verbindung der Formel IId

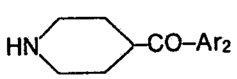 (IId)

lässt sich z. B. durch Behandeln mit einer Verbindung der Formel Hal–alk–$NH_2$ (IIe), worin Hal für Halogen, wie Brom, steht, z. B. in Gegenwart einer Base, in die entsprechende Verbindung der Formel (IIb) überführen. Die Reduktion der Carbonylgruppe X zur Hydroxymethylengruppe X kann, ausgehend von Verbindungen der Formel (IId) bzw. (IIb), beispielsweise mit Hilfe eines geeigneten Metallhydrides, z. B. Lithiumaluminiumhydrid oder Natriumborhydrid, und die reduktive Überführung der Carbonyl- über die Hydroxymethylengruppe in die Methylengruppe X beispielsweise durch katalytische Hydrierung in Gegenwart eines Hydrierungskatalysators erfolgen.

Ebenso kann man zu Verbindungen der Formel (IIb) gelangen, worin X für Methylen steht, indem man in einer Verbindung der Formel (IId) die Carbonylgruppe, z. B. durch katalytische Hydrierung, in die Methylengruppe überführt, die resultierende Verbindung mit einem Halogennitril, wie Chloracetonitril, in Gegenwart einer Base, wie Ethyldiisopropylamin, kondensiert und anschliessend die Cyanogruppe, z. B. durch katalytische Hydrierung, in die Aminomethylgruppe überführt.

Zu Variante b):

Reaktionsfähiges verestertes Hydroxy $X_5$ bedeutet insbesondere mit einer starken anorganischen Säure oder organischen Säure verestertes Hydroxy, beispielsweise Halogen, wie Chlor, Brom oder Iod, Sulfonyloxy, wie Hydroxysulfonyloxy, Halogensulfonyloxy, z. B. Fluorsulfonyloxy, gegebenenfalls, z. B. durch Halogen, substituiertes Niederalkansulfonyloxy, z. B. Methan- oder Trifluormethan-sulfonyloxy, Cycloalkansulfonyloxy, z. B. Cyclohexansulfonyloxy, oder gegebenenfalls, z. B. durch Niederalkyl oder Halogen, substituiertes Benzolsulfonyloxy, z. B. p-Bromphenyl- oder p-Toluolsulfonyloxy, gegebenenfalls, z. B. durch Halogen, substituiertes Niederalkanoyloxy, wie Acetyl- oder Trifluoracetyloxy, gegebenenfalls, z. B. durch Niederalkyl, Niederalkoxy, Halogen und/oder Nitro, substituiertes Benzoyloxy.

Die verfahrensgemässe Kondensation (N-Alkylierung) wird in an sich bekannter Weise durchgeführt, erforderlichenfalls in Gegenwart einer Base. Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide, sowie carbocyclische Amine in Frage. Beispielhaft seien Natrium-hydroxid, -hydrid, -amid, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, oder Ethyl-diisopropylamin, N-Methyl-piperidin, Pyridin, Benzyl-trimethylammoniumhydroxid, 1,5-Diaza-bicyclo[4.3.0]-non-5-en (DBN) sowie 1,8-Diaza-bicyclo[5.4.0]undec-7-en (DBU) genannt, ferner Phosphine, wie Triphenylphosphin, oder Halogensilane, wie Tetrachlorsilan.

Die N-Alkylierung wird beispielsweise in Ab- oder, üblicherweise, Anwesenheit eines geeigneten Lösungs- oder Verdünnungsmittels bzw. eines entsprechenden Gemisches, bei Raumtemperatur oder unter Erwärmen, z. B. in einem Temperaturbereich von 0 °C bis 200 °C, vorzugsweise zwischen Raumtemperatur und 150 °C, und erforderlichenfalls in einem geschlossenen Gefäss, gegebenenfalls unter Druck und/oder unter Inertgas durchgeführt.

Die in dieser Verfahrensvariante verwendeten Ausgangsstoffe sind z. T. bekannt oder können nach an sich bekannten Verfahren hergestellt werden.

So sind beispielsweise Ausgangsstoffe der Formel (IIIa), worin $X_2$ Wasserstoff ist und $X_3$ für eine Gruppe der Formel $-alk-X_5$ steht, zugänglich, indem man beispielsweise von einer Carbonsäure der Formel $R-Ar_1-COOH$ (IIId) oder einem reaktionsfähigen Säurederivat, insbesondere einem Carbonsäurehalogenid, davon ausgeht und mit einem Amin der Formel $X_5-alk-NH_2$ (IIIe) behandelt. Ausgangsstoffe der Formel (IIIa), worin $X_2$ und $X_3$ gemeinsam für alk' stehen, wobei alk' 2- bis 3-gliedriges Alkylen mit 2 bis und mit 7 C-Atomen darstellt, insbesondere Ethylen, können beispielsweise hergestellt werden, indem man ein entsprechendes Aziridin bzw. Azetidin mit einer Verbindung der Formel (IIId) oder einem reaktionsfähigen Säurederivat davon, z.B. in Gegenwart einer Base, z.B. Triethylamin, N-acyliert. Ausgangsstoffe der Formel (IIIa), worin $X_2$ und $X_3$ Wasserstoff sind, sind beispielsweise aus entsprechenden Verbindungen der Formel (IIId) oder deren reaktionsfähigen Derivaten durch Umsetzung mit Ammoniak zugänglich.

Zur Herstellung von Ausgangsstoffen der Formel (IIIb) oder ihrer Salze, worin $X_4$ für eine Gruppe der Formel $-alk-X_5$ steht, geht man beispielsweise von Verbindungen der Formel (IIIb) aus, worin $X_4$ Wasserstoff ist, und führt unter üblichen N-alkylierenden Bedingungen, z.B. in Gegenwart einer Base, wie Triethylamin, durch Behandeln mit einem Ethylen- oder einem Propylenoxid bzw. einer Verbindung der Formel $X_5-alk-OH$ (IIIf) zunächst die Gruppe $-alk-OH$ ein, in der die Hydroxygruppe anschliessend reaktionsfähig verestert wird.

Zu Variante c):

Ausgangsmaterial der Formel (IIIc) ist zugänglich, indem man beispielsweise Verbindungen der Formeln IIIg und IIIi

$R-Ar_1-CO-NH-alk-NH_2$ (IIIg)

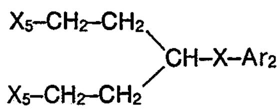 (IIIi)

in Gegenwart einer für die N-Alkylierung üblichen, z.B. einer der vorstehend genannten Basen, wie Pyridin, unter Abspaltung eines Äquivalents $H-X_5$ umsetzt. Je nach Wahl der Reaktionsbedingungen, z.B. der Konzentrationsverhältnisse, kann die N-Alkylierung unter Abspaltung eines weiteren Äquivalents $H-X_5$ in situ direkt zu entsprechenden Zielverbindungen der Formel (I) führen.

Verbindungen der Formel (IIIg) ihrerseits bzw. deren Salze können beispielsweise erhalten werden, indem man eine Verbindung der Formel (IIId) oder ein reaktionsfähiges Säurederivat davon mit einem Überschuss von einer Verbindung der Formel $H_2N-alk-NH_2$ (IIIh) unter üblichen Bedingungen amidiert. Verbindungen der Formel (IIIi) können beispielsweise durch Umsetzung einer Verbindung der Formel $X_6-CH_2-CH_2-CH_2-CO-Ar_2$ (IIIj), worin $X_6$ für z.B. Alkoxy steht, unter Basen-Katalyse, z.B. in Gegenwart von Natriumamid, mit Ethylenoxid und anschliessender Spaltung des Ethers, hergestellt werden. Die Etherspaltung kann beispielsweise mit Hilfe einer Lewissäure, wie Bortrifluorid, oder einer starken Halogenwasserstoffsäure, wie Brom- oder Iodwasserstoffsäure, erfolgen. Bei der Wahl einer Halogenwasserstoffsäure, die im Überschuss eingesetzt wird, können solche Verbindungen der Formel (IIIi) erhalten werden, worin X für die Carbonylgruppe steht und $X_5$ Halogen bedeutet. Gewünschtenfalls kann die Carbonylgruppe X in üblicher Weise reduktiv in die Hydroxymethylen- oder Methylengruppe übergeführt werden.

Zu Variante d):

Die verfahrensgemässe Umsetzung wird in an sich bekannter Weise durchgeführt, erforderlichenfalls in Gegenwart einer Base. Als Base kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide sowie carbocyclische Amine in Frage. Beispielhaft seien Natrium-hydroxid, -hydrid, -amid, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, oder Ethyl-diisopropylamin, N-Methyl-piperidin, Pyridin, Benzyltrimethyl-ammoniumhydroxid, 1,5-Diaza-bicyclo[4.3.0]non-5-en (DBN) sowie 1,8-Diaza-bicyclo[5.4.0]undec-7-en (DBU) genannt, ferner Phosphine, wie Triphenylphosphin, oder Halogensilane, wie Tetrachlorsilan.

Die Umsetzung wird in an sich bekannter Weise durchgeführt, beispielsweise in Ab- oder, üblicherweise, Anwesenheit eines geeigneten Lösungs- oder Verdünnungsmittels bzw. eines entsprechenden Gemisches, unter Kühlen, bei Raumtemperatur oder unter Erwärmen, z.B. in einem Temperaturbereich von $-20\,°C$ bis $200\,°C$, vorzugsweise zwischen Raumtemperatur und $150\,°C$, und erforderlichenfalls in einem geschlossenen Gefäss, gegebenenfalls unter Druck und/oder unter Inertgas.

Die in dieser Verfahrensvariante verwendeten Ausgangsstoffe sind z.T. bekannt bzw. können nach an sich bekannten Verfahren hergestellt werden.

So sind beispielsweise Ausgangsstoffe der Formel (IVa) zugänglich, indem man beispielsweise von einer Carbonsäure der Formel $R-Ar_1-COOH$ (IIId) oder einem reaktionsfähigen Säurederivat, insbesondere einem Säurehalogenid, davon ausgeht, und zunächst mit einem Amin der Formel $X_5-alk'-NH_2$ (IVc), worin $X_5$ reaktionsfähiges verestertes Hydroxy darstellt, und anschliessend das hieraus resultierende Carbonsäureamid der Formel IIIa

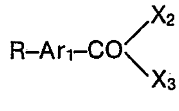 (IIIa)

worin $X_2$ Wasserstoff und $X_3$ eine Gruppe der Formel $-alk'-X_5$ bedeuten, mit einer Base, z.B. Triethylamin oder Ethyl-diisopropylamin, behandelt.

Zu Variante e):

Ein in R überführbarer Rest $X_7$ steht beispielsweise für die Diazonium-Gruppierung der Formel $N_2^{\oplus}A^{\ominus}$, in der $A^{\ominus}$ das Anion einer starken Protonsäure, z.B. ein Anion einer Mineralsäure oder komplexen Metallsäure, wie Chlorid, Perchlorat, Sulfat, Tetrafluorborat oder Hexachlorantimonat, darstellt.

Die Substitution der Diazoniumgruppe $X_7$ in Verbindungen der Formel (V) durch Hydroxy kann analog der «Phenolverkochung» in wässrigem Medium erfolgen, während die Behandlung des Diazoniumsalzes der Formel (V) mit einem aliphatischen Alkohol, insbesondere mit einem Niederalkanol, zu Verbindungen der Formel (I) führen kann, worin R mit einem aliphatischen Alkohol verethertes Hydroxy bedeutet.

Der Ersatz von $X_7$ in Verbindungen der Formel (V) durch Chlor, Brom oder Iod kann beispielsweise durch Umsetzung mit entsprechenden Halogeniden, wie Alkalimetallhalogeniden, erfolgen. Dabei liegt als Anion $A^{\ominus}$ das dem jeweiligen Halogenatom entsprechende Halogenid vor.

Die Reaktion kann beispielsweise analog der Sandmeyer-Reaktion durch Cu(I)-Ionen, gemäss der Gattermann-Reaktion durch Kupferpulver oder in Anlehnung an die Körner-Contardi-Reaktion durch Cu(II)-Ionen katalysiert werden. Zur Einführung von Fluor in entsprechende Verbindungen der Formel (V) kann man beispielsweise gemäss der Schiemann-Reaktion von Diazoniumsalzen der Formel (V) ausgehen und diese mit Borfluorwasserstoffsäure oder einem Salz, insbesondere Alkalimetallsalz davon, oder in einer Abwandlung dieser Reaktion, mit entsprechenden Pentafluorsilikaten bzw. Hexafluorphosphaten umsetzen. Dabei wird zunächst das gebildete, den jeweiligen Diazoniumfluor-Komplex aufweisende Salze, insbesondere in trockenem Zustand, thermisch zu einer Verbindung der Formel (I) zersetzt, worin R Fluor bedeutet.

Die Substitution der Gruppierung $X_7$ kann in an sich bekannter Weise durchgeführt werden, erforderlichenfalls in Gegenwart eines Lösungs- oder Verdünnungsmittels, bei erniedrigter oder erhöhter Temperatur, z.B. in einem Bereich von etwa 40° bis etwa 120 °C, und/oder in einem geschlossenen Gefäss.

Zur Herstellung von Ausgangsmaterial der Formel (V), worin $X_7$ für die ionische Gruppierung $-N_2^{\ominus}A^{\ominus}$ steht, kann man beispielsweise von Verbindungen der Formeln Va und IIa

$X_8-Ar_1-X_1$ (Va) 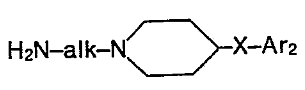 (IIa)

ausgehen, worin $X_1$ die vorstehend angegebene Bedeutung hat und $X_8$ für eine gegebenenfalls, z.B. vorteilhaft durch eine leicht abspaltbare Acylgruppe, geschützte Aminogruppe steht, und diese Verbindungen z.B. in der unter Variante a) beschriebenen Weise kondensiert und die gegebenenfalls vorhandene Aminoschutzgruppe wieder abspaltet. In so erhältlichen Verbindungen der Formel

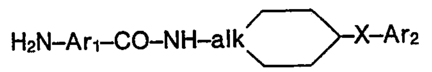 (Vb)

kann die Aminogruppe in an sich bekannter Weise durch übliche Diazotierung, z.B. mit salpetriger Säure oder einem Nitrit in Gegenwart einer Säure, in die entsprechende ionische Gruppierung $X_7$ übergeführt werden.

Vorteilhaft führt man die Diazotierung der aromatischen Aminoverbindung (Vb) und die sich anschliessende Überführung von so erhältlichen Verbindungen der Formel (V) in Verbindungen der Formel (I) ohne Isolierung entsprechenden Ausgangsmaterials der Formel (V) in situ durch. Jedoch ist dessen Isolierung für die Verfahrensvariante analog der Schiemann-Reaktion angezeigt.

Ein in R überführbarer Rest $X_7$ kann weiterhin für einen von einer organischen Carbonsäure abgeleiteten Acyloxyrest stehen, wobei dieser beispielsweise gegebenenfalls, z.B. durch Halogen oder Aryl, substituiertes Niederalkanoyloxy oder Aroyl-, wie gegebenenfalls substituiertes Benzoyloxy, bedeuten kann.

Die Überführung von entsprechenden Verbindungen der Formel (V) in Verbindungen der Formel (I), worin R Hydroxy bedeutet, erfolgt beispielsweise durch Hydrolyse oder Umesterung, wobei jeweils in Gegenwart einer Säure oder Base gearbeitet wird. Dabei können mit einem aliphatischen Alkohol veresterte Hydroxygruppen, sofern sie Substituenten der aromatischen Systeme $Ar_1$ bzw. $Ar_2$ sind, gleichzeitig entsprechend hydrolysiert bzw. umgeestert werden.

Ausgehend von entsprechenden Verbindungen der Formel (V) kann man beispielsweise durch Umsetzung mit einem reaktionsfähigen Ester eines aliphatischen Alkohols, z.B. mit einem Niederalkylhalogenid oder -sulfonat, zu Verbindungen der Formel (I) gelangen, worin R mit einem aliphatischen Alkohol verethertes Hydroxy bedeutet. Dabei arbeitet man vorteilhaft in Gegenwart einer Base. Im Verlauf dieser Umsetzung können mit einer organischen Carbonsäure veresterte Hydroxygruppen als Substituenten der aromatischen Reste $Ar_1$ bzw. $Ar_2$ gleichzeitig in mit einem aliphatischen Alkohol veretherte Hydroxygruppen übergeführt werden.

Ein in R überführbarer Rest $X_7$ kann weiterhin, z.B. durch eine in der einschlägigen Literatur üblicherweise verwendete Hydroxyschutzgruppe, geschütztes Hydroxy bedeuten, wobei dieses beispielsweise 1-Arylniederalkoxycarbonyloxy, wie gegebenenfalls im Phenylring, z.B. durch Halogen, substituiertes Benzyloxycarbonyloxy, 1-Arylniederalkoxy, wie gegebenenfalls im Phenylteil, z.B. durch Halogen oder Niederalkoxy, substituiertes Benzyloxy, oder Silyloxy, wie gegebenenfalls substituiertes Triniederalkylsilyloxy, darstellt. Entsprechende bevorzugte Reste $X_7$ sind z.B. Benzyl-, 2- oder 4-Brombenzyloxycarbonyloxy, Benzyloxy, 3-Brom-, 2,6-Dichlor- oder 4-Methoxybenzyloxy oder Trimethylsilyloxy.

Derartige Reste $X_7$ können in an sich bekannter Weise, z.B. durch Hydrolyse, Acidolyse oder Reduktion im Hydroxy R übergeführt werden.

Die hydrolytische Abspaltung der Hydroxyschutzgruppe kann gegebenenfalls in Gegenwart einer die Hydrolyse unterstützenden Base oder Säure erfolgen, wobei als Basen beispielsweise Alkalimetall- oder Erdalkalimetallhydroxide bzw. -carbonate und als Säuren beispielsweise anorganische oder organische Protonsäuren geeignet sind.

Bevorzugt werden durch Hydrolyse 1-Arylniederalkoxycarbonyloxy- oder Silyloxyreste in Hydroxy übergeführt.

Bei der Acidolyse werden in der Regel starke Säuren, wie Mineralsäuren, z.B. Halogenwasserstoffsäuren, Perchlorsäure, gegebenenfalls geeignet substituierte Niederalkancarbonsäuren oder Sulfonsäuren, wie gegebenenfalls substituierte Benzolsulfonsäuren, oder Gemische derselben verwendet.

Bevorzugte Säuren sind z.B. Fluorwasserstoffsäuren, Bromwasserstoffsäure/Eisessig oder Trifluoressigsäure.

Entsprechend in Hydroxy R überführbare Gruppen sind beispielsweise 1-Arylniederalkoxycarbonyloxy oder 1-Arylniederalkoxy.

Diese Reste können ebenfalls reduktiv, z.B. durch Hydrogenolyse mit Wasserstoff in Gegenwart eines Hydrierungskatalysators, bzw. 1-Arylniederalkoxycarbonyloxy zusätzlich reduktiv durch metallische Systeme aus Metallkomponente und einer Wasserstoff-liefernden Komponente, wie Natrium/Ammoniak, in Hydroxy übergeführt werden.

Diese Umsetzungen können in an sich bekannter Weise, erforderlichenfalls in Gegenwart eines Lösungs- oder Verdünnungsmittels, bei erniedrigter oder insbesondere bei erhöhter Temperatur, in einem Temperaturbereich von 50° bis 150 °C, in einem geschlossenen Gefäss und/oder unter Inertgas, durchgeführt werden.

Das Ausgangsmaterial der Formel (V), worin $X_7$ für einen Acyloxyrest oder für geschütztes Hydroxy steht, kann erhalten werden, indem man beispielsweise eine Verbindung der Formel Vd

$$X_7\text{–}Ar_1\text{–}X_1 \qquad \text{(Vd)}$$

mit einer Verbindung der Formel (IIa) in Analogie zu der unter Variante a) angegebenen Weise kondensiert.

Eine verfahrensgemäss oder auf andere Weise erhältliche erfindungsgemässe Verbindung kann in an sich bekannter Weise in eine andere erfindungsgemässe Verbindung übergeführt werden.

So kann man beispielsweise in den erfindungsgemässen Verbindungen, die eine freie Hydroxy- und/oder eine Hydroxymethylengruppe aufweisen, die Hydroxylgruppe mit einer organischen Carbonsäure, wie Niederalkancarbonsäure, verestern. Diese Veresterung erfolgt in an sich bekannter Weise, z.B. durch Behandeln mit der gewünschten Carbonsäure oder einem reaktionsfähigen Derivat, z.B. Säureanhydrid oder -halogenid, erforderlichenfalls in Gegenwart einer Säure, wie einer Protonsäure, z.B. Mineralsäure oder Sulfonsäure, oder einer Lewissäure, z.B. entsprechenden Halogeniden von geeigneten Elementen der 3. Hauptgruppe oder der entsprechenden Nebengruppen.

Die Veresterung kann ebenso in Gegenwart einer Base, wie eines Alkalimetallhydroxids, -carbonats oder eines Amins bzw. einer cyclischen Stickstoffbase, bzw. eines wasserbindenden Mittels, wie eines üblichen Carbodiimids, ablaufen. Umgekehrt kann in verestertes Hydroxy aufweisenden Gruppen, die Hydroxygruppe durch Solvolyse, z.B. mit Hilfe einer Basen-Katalyse, in Freiheit gesetzt werden.

Die entsprechenden Umsetzungen können, falls erforderlich, unter Kühlen oder Erwärmen, z.B. in einem Temperaturbereich von 0° bis 100 °C, in An- oder Abwesenheit eines Lösungs- oder Verdünnungsmittels, unter Inertgas und/oder unter Druck und gegebenenfalls in einem geschlossenen Gefäss durchgeführt werden.

Eine Hydroxy aufweisende erfindungsgemässe Verbindung kann nach an sich bekannten Methoden verethert werden. Die Veretherung kann z.B. mit einem Alkohol, wie gegebenenfalls substituiertem Niederalkanol, oder einem reaktionsfähigen Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide, Methan-, Benzol- oder p-Toluol-sulfonate, in Betracht. Die Veretherung kann z.B. in Gegenwart einer Base, eines Alkalimetallhydrids, -hydroxids, -carbonats oder eines basischen Amins, erfolgen. Umgekehrt können entsprechende Ether, wie Niederalkoxyverbindungen, z.B. mittels starker Säuren, wie Mineralsäuren, z.B. den Halogenwasserstoffsäuren Brom- oder Iodwasserstoffsäure, die vorteilhaft in Form von Pyridiniumhalogeniden vorliegen können, oder mittels Lewissäuren, z.B. Halogeniden von Elementen der 3. Hauptgruppe oder der entsprechenden Nebengruppen, gespalten werden. Diese Umsetzungen können, falls erforderlich, unter Kühlen oder Erwärmen, z.B. einem Temperaturbereich von −20° bis 100 °C, in An- oder Abwesenheit eines Lösungs- oder Verdünnungsmittels, unter Inertgas und/oder unter Druck und gegebenenfalls in einem geschlossenen Gefäss, durchgeführt werden.

In einer erfindungsgemässen Verbindung, welche einen Hydroxyniederalkylrest aufweist, kann die Hydroxygruppe, z.B. durch Behandeln mit einem geeigneten Halogenierungsmittel, z.B. Thionylchlorid, in Halogen, z.B. Chlor, übergeführt werden.

Weist der Arylrest $Ar_1$ bzw. $Ar_2$ z.B. eine Cyanogruppe als Substituenten auf, kann diese z.B. hydrolytisch, vorzugsweise unter sauren oder basischen Bedingungen, z.B. in Gegenwart eines Alkalimetallhydroxids, und, wenn erwünscht, in Gegenwart von Wasserstoffperoxid in einem wässrig-alkoholischen Lösungsmittel, in die Carbamoylgruppe übergeführt werden. Die entsprechenden Umsetzungen können, falls erforderlich, unter Kühlen oder Erwärmen, z.B. in einem Temperaturbereich von 0° bis 150°C, gelegentlich auch bei höheren Temperaturen, in An- oder Abwesenheit eines Lösungs- oder Verdünnungsmittels, unter Inertgas und/oder unter Druck und gegebenenfalls in einem geschlossenen Gefäss durchgeführt werden.

Weisen in erfindungsgemässen Verbindungen die Reste $Ar_1$ bzw. $Ar_2$ als Substituenten Cyano auf, so kann dieser, beispielsweise durch Behandeln mit einem Alkohol, z.B. Niederalkanol, in Gegenwart einer Säure, z.B. Salzsäure, z.B. in Alkoxycarbonyl übergeführt werden.

In Verbindungen der Formel (I), worin $Ar_1$ bzw. $Ar_2$ als Substituenten eine veresterte oder amidierte Carboxygruppe aufweisen, kann man eine solche Gruppe z.B. mittels Hydrolyse, z.B. in Gegenwart eines basischen Mittels, wie einer anorganischen Base, z.B. eines Alkalimetall- oder Erdalkalimetall-, z.B. Natrium-, Kalium- oder Calciumhydroxids, oder eines sauren Mittels, wie einer Mineralsäure, in eine freie Carboxygruppe überführen.

Ferner kann man in Verbindungen der Formel (I), worin $Ar_1$ bzw. $Ar_2$ als Substituenten eine Carboxygruppe aufweisen, z.B. durch Behandeln mit einem Alkohol, wie einem Niederalkanol, in Gegenwart eines geeigneten Veresterungsmittels, wie eines sauren Reagens, z.B. einer anorganischen oder organischen Säure, wie Chlorwasserstoff-, Trifluoressig- oder p-Toluolsulfonsäure, oder einer Lewissäure, z.B. Zinkchlorid, oder eines wasserbindenden Kondensationsmittels, z.B. eines Carbodiimids, wie N,N'-Dicyclohexyl-carbodiimid, oder durch Behandeln mit einem Diazoreagens, wie einem Diazoniederalkan, z.B. Diazomethan, die Carboxygruppe in eine veresterte Carboxygruppe überführen. Diese kann man auch erhalten, wenn man Verbindungen der Formel I, worin in $Ar_1$ bzw. $Ar_2$ als Substituenten eine Carboxygruppe in freier Form oder in Salz-, wie Ammonium- oder Metall-, z.B. Alkalimetall-, wie Natrium- oder Kaliumsalzform vorliegt, mit einem reaktionsfähigen Ester eines Alkohols, wie Niederalkylhalogenid, z.B. Methyl- oder Ethylchlorid, -bromid oder -iodid, oder einem organischen Sulfonsäureester, wie einem organischen Sulfonsäureester, wie einem entsprechenden Niederalkylester, z.B. Methansulfonsäure- oder p-Toluolsulfonsäuremethylester oder -ethylester, behandelt.

Verbindungen der Formel (I), worin $Ar_1$ bzw. $Ar_2$ als Substituenten eine veresterte Carboxygruppe aufweisen, kann man durch Umesterung, z.B. durch Behandeln mit einem Alkohol, üblicherweise einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetallniederalkanoats, -niederalkanolats oder -cyanids, wie Natriumacetat, -methanolat, -ethylat, -tert-butanolat oder -cyanid, oder eines geeigneten sauren Mittels, gegebenenfalls unter Entfernung des entstehenden Alkohols, z.B. durch Destillation, in andere Esterverbindungen der Formel (I) umwandeln. Man kann auch von entsprechenden, sogenannten aktivierten Estern der Formel (I) ausgehen, worin $Ar_1$ bzw. $Ar_2$ als Substituenten eine aktivierte veresterte Carboxygruppe aufweisen (siehe unten), und diese durch Behandeln mit einem Alkohol, wie einem Niederalkanol, in einen anderen Ester umwandeln.

Verbindungen der Formel (I), worin $Ar_1$ bzw. $Ar_2$ als Substituenten eine amidierte Carboxygruppe aufweisen, können vorteilhaft auch aus den entsprechenden Säure- und Esterverbindungen der Formel (I), worin $Ar_1$ bzw. $Ar_2$ als Substituenten eine gegebenenfalls veresterte Carboxygruppe aufweisen, erhalten werden. So kann man z.B. Verbindungen der Formel (I), die eine freie Carboxylgruppe aufweisen, mit Harnstoff bei erhöhten Temperaturen, z.B. bei 200–240°C, mit einem Formamid, z.B. Dimethylformamid, in Gegenwart eines geeigneten Kondensationsmittels, wie Phosphorpentoxid, bei erhöhten Temperaturen, oder mit einem Amin in Gegenwart eines geeigneten Kondensationsmittels, wie eines Carbodiimids, z.B. N,N'-Diethyl-carbodiimid, ferner eines Phosphins, wie Triphenylphosphin (z.B. zusammen mit Bis-2-pyridyl-disulfid), oder eines Silans, wie Trichlorsilan (z.B. zusammen mit Pyridin) umsetzen, und die entsprechenden Amidverbindungen der Formel (I), worin $Ar_1$ bzw. $Ar_2$ als Substituenten eine amidierte Carboxygruppe enthalten, erhalten. Sie können auch aus Verbindungen der Formel I, worin $Ar_1$ bzw. $Ar_2$ als Substituenten eine in Salzform vorliegende Carboxygruppe aufweisen, erhalten werden, z.B. indem man ein entsprechendes Ammoniumsalz, z.B. durch Behandeln mit einem Dehydratisierungsmittel, wie Phosphorpentoxid, dehydratisiert, oder ein entsprechendes Alkalimetall-, z.B. Natriumsalz, mit einem Amin, vorzugsweise in Gegenwart eines geeigneten Kondensationsmittels, wie Phenylphosphonsäuredichlorid, umsetzt.

Man kann in Verbindungen der Formel (I), worin $Ar_1$ bzw. $Ar_2$ als Substituenten eine Carboxylgruppe enthalten, diese auch zuerst in ein reaktionsfähiges Derivat, wie ein Anhydrid, inkl. ein gemischtes Anhydrid, wie ein Säurehalogenid, z.B. -chlorid (z.B. durch Behandeln mit einem Thionylhalogenid, z.B. -chlorid), oder ein Anhydrid mit einem Ameisensäureester, z.B. -niederalkylester (z.B. durch Behandeln eines Salzes, wie eines Ammonium- oder Alkalimetallsalzes, mit einem Halogen-, wie Chlorameisensäureester, wie niederalkylester), oder in einen aktivierten Ester, wie einen Cyanmethyl-, Nitrophenyl-, z.B. 4-Nitrophenyl-, oder Polyhalogenphenyl-, z.B. Pentachlorphenylester (z.B. durch Behandeln mit einer entsprechenden Hydroxyverbindung in Gegenwart eines geeigneten Kondensationsmittels, wie N,N'-Dicyclohexyl-carbodiimid) überführen, und ein solches reaktionsfähiges Derivat dann mit Ammoniak (gegebenenfalls in derivatisierter Form) oder einem Amin umsetzen und so zu Amidverbindungen der Formel (I) gelangen, worin $Ar_1$ bzw. $Ar_2$ als Substituenten eine amidierte Carboxygruppe aufweisen. Dabei kann man diese direkt oder über Zwischenverbindungen erhalten; so kann man z. B. einen aktivierten Ester, wie einen 4-Nitrophenylester, einer Verbindung der Formel I mit einer Carboxygruppe zuerst mit einem 1-unsubstituierten Imidazol umsetzen und die so entstandene 1-Imidazolylcarbonylverbindung mit dem Ammoniak oder dem Amin in Reaktion bringen. Man kann aber auch andere, nicht-aktivierte Ester, wie Niederalkylester von Verbindungen der Formel (I), worin $Ar_1$ bzw. $Ar_2$ als Substituenten z. B. Niederalkoxycarbonyl aufweisen, mit Ammoniak oder Aminen zur Reaktion bringen.

In erfindungsgemässen Verbindungen, in welchen der Rest $Ar_1$ bzw. $Ar_2$ eine freie Amino-, Carbamoyl- oder Sulfamoylgruppe enthält, kann die jeweilige Aminogruppe in der vorstehend unter Variante b) angegebenen Weise mono- oder disubstituiert werden. Ebenso können primäre oder sekundäre Aminogruppen analog der Leuckart-Wallach- (bzw. Eschweiler-Clarke)-Reaktion aus Carbonylverbindungen, z. B. unter Verwendung von Ameisensäure als Reduktionsmittel, reduktiv alkyliert werden.

In erfindungsgemässen Verbindungen, worin X für die Carbonylgruppe steht, kann diese z. B. durch Reduktion, beispielsweise durch Behandeln mit einem geeigneten, gegebenenfalls komplexen Hydrid, wie einem Hydrid, gebildet aus einem Element der 1. und 3. Hauptgruppe des PSE, z. B. Natriumborhydrid oder Natriumcyanoborhydrid, in die Hydroxymethylengruppe X übergeführt werden. Die Hydroxymethylengruppe X ihrerseits kann z. B. durch Reduktion, beispielsweise mit Wasserstoff unter Verwendung eines Hydrierungskatalysators zur Methylengruppe reduziert werden.

In erfindungsgemässen Verbindungen, worin X für die Carbonylgruppe steht, kann diese beispielsweise in Gegenwart einer Säure, wie Mineralsäure, z. B. Schwefel- oder Chlorwasserstoffsäure, oder Sulfonsäure, z. B. p-Toluolsulfonsäure, mit einem Alkohol, wie Niederalkanol oder Niederalkandiol, z. B. Ethanol oder Glykol, acetalisiert werden. Acetalisiertes Carbonyl X kann z. B. umgekehrt durch Säuren, z. B. der vorstehend genannten Art, hydrolysiert werden.

Falls der Ring $Ar_1$ durch Niederalkylthio substituiert ist, kann man dieses auf übliche Weise zu entsprechendem Niederalkansulfinyl bzw. -sulfonyl oxidieren. Als geeignetes Oxidationsmittel für die Oxidation zur Sulfoxidstufe kommen beispielsweise anorganische Persäuren, wie Persäuren von Mineralsäuren, z. B. Periodsäure oder Perschwefelsäure, organische Persäuren, wie entsprechende Percarbon- oder Persulfonsäuren, z. B. Perameisen-, Peressig-, Trifluorperessig- bzw. Perbenzoesäure oder p-Toluolpersulfonsäure, oder Gemische aus Wasserstoffperoxid und Säuren, z. B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z. B. Essigsäure oder Trifluoressigsäure, oder Übergangsmetalloxide, wie Oxide von Elementen der VII. Nebengruppe, z. B. Vanadium-, Molybdän- oder Wolframoxid, zu nennen sind. Die Oxidation wird unter milden Bedingungen, z. B. bei Temperaturen von etwa −50° bis etwa +100 °C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des Niederalkylthio zum Niederalkansulfonyl. Jedoch setzt man hierbei üblicherweise das Oxidationsmittel im Überschuss ein.

Weist ein aromatischer Ring ($Ar_1$ bzw. $Ar_2$) als Substituenten ein Wasserstoffatom auf, so kann dieses mit Hilfe eines Halogenierungsmittels in üblicher Weise durch ein Halogenatom ersetzt, z. B. mit Brom, Hypobromsäure, Acylhyprobromite oder andere organische Bromverbindungen, z. B. N-Bromsuccinimid, N-Bromacetamid, N-Bromphthalimid, Pyridiniumperbromid, Dioxandibromid, 1,3-Dibrom-5,5-dimethylhydantoin, 2,4,4,6-Tetrabrom-2,5-cyclohexandien-1-on, bromiert bzw. mit elementarem Chlor, z. B. in einem halogenierten Kohlenwasserstoff, wie Chloroform, und unter Kühlen, z. B. bis auf etwa −10° bis etwa +10 °C, chloriert werden.

Ferner kann die Cyanogruppe in den Ring $Ar_1$ eingeführt werden, indem man beispielsweise ein Halogenatom, insbesondere Iod oder Brom, durch Umsetzung mit einer Cyanoverbindung, wie einem Alkalimetallcyanid, z. B. Kaliumcyanid, oder insbesondere mit Cu(I)-cyanid, vorzugsweise unter Erwärmen, z. B. bis auf etwa 60° bis etwa 250 °C, in An- oder Abwesenheit eines Lösungs- oder Verdünnungsmittels, oder vorteilhaft unter Inertgas, durch die Cyanogruppe substituiert.

Enthält der Arylrest $Ar_1$ bzw. $Ar_2$ in den erfindungsgemässen Verbindungen eine Aminogruppe, so kann diese in üblicher Weise diazotiert werden, z. B. durch Behandeln mit einem Nitrit, z. B. Natriumnitrit, in Gegenwart einer geeigneten Protonsäure, z. B. Mineralsäure, wobei die Reaktionstemperatur vorteilhaft unter etwa 5 °C gehalten wird. Die so erhältliche, in Salzform vorliegende Diazoniumgruppe kann man nach analogen Verfahren beispielsweise wie folgt substituieren: durch die Hydroxygruppe analog der Phenolverkochung in Gegenwart von Wasser; durch eine Alkoxygruppe durch Behandeln mit einem entsprechenden Alkohol, wobei Energie zugeführt werden muss; durch das Fluoratom analog der Schiemann-Reaktion bei der Thermolyse von entsprechenden Diazoniumtetrafluorboraten; durch die Halogenatome Chlor, Brom oder Iod sowie die Cyanogruppe analog der Sandmeyer-Reaktion bei der Umsetzung mit entsprechenden Cu(I)-Salzen, zunächst unter Kühlen, z. B. auf etwa unter 5 °C, und anschliessendem Erhitzen, z. B. auf etwa 60° bis etwa 150 °C.

Weisen die erfindungsgemässen Verbindungen im Arylrest $Ar_1$ bzw. $Ar_2$ eine Nitrogruppe auf, so kann diese in an sich bekannter Weise, beispielsweise durch katalytische Hydrierung, zur Aminogruppe reduziert werden.

Ferner kann ein aromatischer Ring $Ar_1$ bzw. $Ar_2$ beispielsweise mit einer Niederalkanol bzw. einem Niederalkylhalogenid oder einem Phosphorsäureniederalkylester in Gegenwart von Lewis-Säuren alkyliert werden (Friedel-Crafts-Alkylierung). In einer Verbindung der Formel (I), worin ein aromatischer Ring Brom enthält, kann beispielsweise das Brom durch Umsetzung mit einem Niederalkylbromid in Gegenwart eines Alkalimetalls durch Niederalkyl ersetzt werden.

Enthält ein aromatischer Ring als Substituenten ein Wasserstoffatom, so kann dieses in an sich bekannter Weise durch eine Acylgruppe ausgetauscht werden. So kann beispielsweise die Einfügung der Acylgruppe analog der Friedel-Crafts-Acylierung (cf. G. A. Olah, Friedel-Crafts and Related Reactions, Vol. I, Interscience, New York, 1963–1965) durchgeführt werden, z. B. durch Umsetzung eines reaktiven funktionellen Acylderivates, wie eines Halogenids oder Anhydrids, einer organischen Carbonsäure in Gegenwart einer Lewis-Säure, wie Aluminium-, Antimon(III)- oder -(V)-, Eisen(III)-, Zink(II)-chlorid oder Bortrifluorid.

Enthalten die Verbindungen der Formel (I) ungesättigte Reste, wie Niederalkenyl oder Niederalkadienylgruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z. B. Edelmetalle bzw. deren Derivate, z. B. Oxide, geeignet sind, wie Nickel, Raney-Nickel, Palladium, Platinoxid, die gegebenenfalls auf Trägermaterialien, z. B. auf Kohle oder Calciumcarbonat, aufgezogen sein können. Die Hydrierung kann vorzugsweise bei Drucken zwischen 1 und 100 at und bei einer Temperatur zwischen −80° bis 200 °C, vor allem zwischen Raumtemperatur und 100 °C, durchgeführt werden. Die Reaktion erfolgt zweckmässig in einem Lösungsmittel, wie Wasser, einem Niederalkanol, z. B. Ethanol, Isopropanol oder n-Butanol, einem Ether, z. B. Dioxan, oder einer Niederalkancarbonsäure, z. B. Essigsäure.

Die Erfindung betrifft insbesondere die in den Beispielen beschriebenen Verfahren.

Weisen die genannten Ausgangsstoffe basische Zentren auf, können z. B. auch Säureadditionssalze gebildet werden, während Ausgangsstoffe mit aciden Gruppen z. B. Salze mit Basen bilden.

Je nach Wahl der Reaktionsbedingungen können die Ausgangsstoffe in freier Form oder als Salze eingesetzt werden bzw. können die erfindungsgemässen Verbindungen mit salzbildenden Eigenschaften in freier Form oder in Form von Salzen erhalten werden.

So können erhaltene Säureadditionssalze in an sich bekannter Weise, z. B. durch Behandeln mit einer Base, wie einem Alkalimetallhydroxid, in die freien Verbindungen oder z. B. durch Behandeln mit geeigneten Säuren oder Derivaten davon in andere Salze umgewandelt werden. Erhaltene freie Verbindungen mit salzbildenden basischen Eigenschaften können, z. B. durch Behandeln mit Säuren oder entsprechenden Anionenaustauschern, in ihre Salze umgewandelt werden.

Infolge der engen Beziehung zwischen den salzbildenden Verbindungen in freier Form und in Form von Salzen, sind im Vorausgegangenen und nachfolgend unter den freien Verbindungen oder ihren Salzen sinn- und zweckmässig gegebenenfalls auch die entsprechenden Salze bzw. freien Verbindungen zu verstehen. Umfasst sind ferner für pharmazeutische Verwendungen ungeeignete Salze, da diese beispielsweise für die Isolierung bzw. Reinigung freier erfindungsgemässer Verbindungen sowie deren pharmazeutisch verwendbarer Salze verwendet werden können.

Die erfindungsgemässen Verbindungen, einschliesslich Salze von entsprechenden salzbildenden Verbindungen, können auch in Form ihrer Hydrate erhalten werden, oder ihre Kristalle können z. B. das zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z. B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Diastereomerengemische und Racematgemische können auf Grund der physikalischchemischen Unterschiede der Bestandteile in bekannter Weise in die reinen Isomeren, Diastereomeren oder Racemate aufgetrennt werden, beispielsweise durch Chromatographie an chiralen Adsorbentien und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, durch Spaltung mit spezifischen, immobiliserten Enzymen, über die Bildung von Einschlussverbindungen, z. B. unter Verwendung chiraler Kronenether, wobei ein Enantiomeres komplexiert wird, oder durch Überführung in diastereomere Salze oder Ester, z. B. durch Umsetzung eines basischen Endstoffracemats mit einer mit der racemischen Base Salze bildenden optisch aktiven Säure oder einer optisch aktiven Carbonsäure oder einem reaktiven Derivat davon, und Trennung des auf diese Weise erhaltenen Diastereomerengemisches, z. B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden

verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, ihre Verwendung, z.B. als Zwischenprodukte, ferner gegebenenfalls als Arzneimittelwirkstoffe, und Verfahren zu ihrer Herstellung, bilden ebenfalls einen Gegenstand der Erfindung, wobei die Variablen R, $Ar_1$, alk, X und $Ar_2$ die für die jeweils bevorzugten Verbindungsgruppen der Formel (I) bevorzugten Bedeutungen haben.

Die Dosierung des Wirkstoffes, der allein oder zusammen mit dem üblichen Träger- und Hilfsmaterial verabreicht wird, hängt von den zu behandelnden Spezies, deren Alter und individuellen Zustand sowie der Verabreichungsweise ab. Die Einzeldosen liegen z.B. für Mammalien mit einem Körpergewicht von ca. 70 kg, je nach Art der Erkrankung, individuellem Zustand und Alter, vorzugsweise zwischen 0,5 und 100 mg, z.B. zwischen 0,7 und 70 mg, z.B. bei oraler Verabreichung.

Die Erfindung betrifft im weiteren Verfahren zur Herstellung pharmazeutischer Präparate, die Verbindungen der Formel (I) oder pharmazeutisch verwendbare Salze von solchen Verbindungen mit salzbildenden Eigenschaften als Wirkstoffe enthalten.

Bei den erfindungsgemässen pharmazeutischen Präparaten handelt es sich um solche zur enteralen, wie peroralen oder rektalen, weiter zur parenteralen Verabreichung an Warmblüter. Entsprechende Dosiseinheitsformen, insbesondere zur peroralen Verabreichung, z.B. Dragées, Tabletten oder Kapseln, enthalten vorzugsweise von 1 mg bis 100 mg, insbesondere von 1 mg bis 25 mg, einer Verbindung der Formel (I) oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaftresistenten, Überzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, oder Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können. Bevorzugt sind u.a. Kapseln, die sowohl leicht zerbissen als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung. Temperaturen werden in Celsiusgraden angegeben.

Beispiel 1:

41,4 g (0,2 Mol) 4-(p-Fluorbenzoyl)-piperidin werden in 120 ml Dioxan gelöst und mit 28,3 g (0,1 Mol) N-(2-Bromethyl)-5-cyano-2-methoxy-benzamid versetzt und dann während 60 Stunden bei Raumtemperatur gerührt. Anschliessend versetzt man das Reaktionsgemisch mit einer Lösung von Kaliumcarbonat und extrahiert dann mit Methylenchlorid. Die Methylenchloridlösung wird mit Wasser neutral gewaschen und im Wasserstrahlvakuum eingeengt. Es verbleibt ein hellgelbes kristallines Produkt, das in 200 ml Ethanol aufgeschlämmt und während 2 Stunden gerührt wird. Dann wird das feste Produkt abgenutscht und mit 20 ml eiskaltem Ethanol nachgewaschen. Im Nutschgut erhält man

5-Cyano-N-[2-[4-(p-fluorbenzoyl)-piperidinyl]-ethyl]-2-methoxy-benzamid

als weisses Pulver vom Smp. 167–169°.

Zur Überführung ins Hydrochlorid löst man die freie Base in Methylenchlorid und versetzt diese mit einer etherischen Chlorwasserstofflösung bis zur kongosauren Reaktion. Anschliessend versetzt man bis zur beginnenden Kristallisation mit Ether. Man erhält so das

5-Cyano-N-[2-[4-(p-fluorbenzoyl)-piperidinyl]-ethyl]-2-methoxy-benzamid-hydrochlorid

vom Smp. 193–194°.

Das Ausgangsmaterial wird wie folgt hergestellt:

39,1 g (0,2 Mol) rohes 5-Cyano-2-methoxy-benzoesäurechlorid, hergestellt nach FR-Pat. Nr. 1 525 M/72 CAM und 45 g (0,22 Mol) 2-Bromethylamin-hydrobromid in 300 ml Methylenchlorid werden unter Rühren bei Raumtemperatur tropfenweise mit 64,6 g (0,5 Mol) Ethyldiisopropylamin versetzt. Die erhaltene klare Lösung wird dann noch 2 Stunden bei Raumtemperatur gerührt. Hierauf wird die Methylenchloridlösung zweimal mit 2-n. Salzsäure und einmal mit Wasser ausgeschüttelt, über Magnesiumsulfat getrocknet und eingedampft. Das erhaltene N-(2-Bromethyl)-5-cyano-2-methoxy-benzamid wird zur Reinigung einmal aus Methylenchlorid-Ether umkristallisiert. Smp. 119–120°.

Beispiel 2:

In analoger Weise wie in Beispiel 1 beschrieben erhält man:

4-Chlor-5-cyano-N-[2-[4-(p-fluorbenzoyl)-piperidinyl]-ethyl]-2-methoxy-benzamid-methansulfonat,

Smp. 178–180°; ausgehend von 41,4 g (0,2 Mol) 4-(p-Fluorbenzoyl)-piperidin und 31,8 g (0,1 Mol) N-(2-Bromethyl)-4-chlor-5-cyano-2-methoxy-benzamid in 240 ml Dioxan.

Das Ausgangsmaterial wird wie folgt hergestellt:

Zu einer Lösung von 37,3 g (0,2 Mol) 4-Chlor-2-methoxybenzoesäure in 500 ml Eisessig und 500 ml Wasser werden unter Rühren bei Raumtemperatur 10,7 ml (33,6 g; 0,21 Mol) Brom zugetropft. Anschliessend lässt man noch eine Stunde rühren, versetzt dann mit 500 ml Wasser und nutscht die ausgefallene 5-Brom-4-chlor-2-methoxy-benzoesäure ab und wäscht das Nutschgut mit Wasser nach.

Eine Lösung von 26,5 g (0,1 Mol) 5-Brom-4-chlor-2-methoxy-benzoesäure in 150 ml Ethanol wird mit Chlorwasserstoffgas gesättigt und dann während 15 Stunden stehen gelassen. Anschliessend wird im Wasserstrahlvakuum eingedampft, der Rückstand in Methylenchlorid aufgenommen und mit Natriumbicarbonat ausgeschüttelt. Die Methylenchloridlösung wird über Magnesiumsulfat getrocknet und dann im Wasserstrahlvakuum eingedampft. Als Rückstand verbleibt 5-Brom-4-chlor-2-methoxy-benzoesäure-ethylester vom Smp. 79–81°.

14,7 g (0,05 Mol) 5-Brom-4-chlor-2-methoxy-benzoesäure-ethylester, 5,4 g (0,06 Mol) Kupfer-I-cyanid und 8 ml Dimethylformamid werden unter Rühren und Stickstoffatmosphäre während 3 Stunden auf 190° erhitzt. Nach dem Abkühlen wird das Reaktionsgemisch mit 250 ml Methylenchlorid und 250 ml 2-n. Salzsäure gut verrührt. Man nutscht von den unlöslichen Anteilen ab und trennt die Schichten im Scheidetrichter. Die Methylenchloridlösung wird mit Wasser neutral gewaschen und dann eingedampft. Als Rückstand verbleibt 4-Chlor-5-cyano-2-methoxy-benzoesäure-ethylester, der nach Umkristallisation aus Methylenchlorid-Hexan bei 102–103° schmilzt.

Zur Verseifung werden 24 g (0,1 Mol) 4-Chlor-5-cyano-2-methoxy-benzoesäure-ethylester in 500 ml Methanol, 100 ml Wasser und 110 ml 1-n. Natronlauge während 15 Stunden gerührt. Hierauf wird das Methanol im Wasserstrahlvakuum abgezogen und aus der Restlösung durch Zugabe von verdünnter Salzsäure die 4-Chlor-5-cyano-2-methoxy-benzoesäure ausgefällt. Diese wird abgenutscht und anschliessend aus Dioxan umkristallisiert. Man erhält 4-Chlor-5-cyano-2-methoxy-benzoesäure vom Smp. 191–192°.

In analoger Weise wie in Beispiel 2 beschrieben erhält man:

4-Chlor-5-cyano-2-methoxy-benzoesäurechlorid, ausgehend von 8,5 g (0,04 Mol) 4-Chlor-5-cyano-2-methoxy-benzoesäure und 12 ml (19 g; 0,16 Mol) Thionylchlorid in 120 ml Chloroform.

In analoger Weise wie in Beispiel 1 beschrieben erhält man:

N-(2-Bromethyl)-4-chlor-5-cyano-2-methoxy-benzamid

vom Smp. 127–129°; ausgehend von 23 g (0,1 Mol) 4-Chlor-5-cyano-2-methoxy-benzoesäurechlorid, 20,5 g (0,1 Mol) 2-Bromethylamin-hydrobromid und 27,1 g (0,21 Mol) Ethyl-diisopropylamin.

Beispiel 3:

In analoger Weise wie in Beispiel 1 beschrieben erhält man:

5-Brom-4-chlor-N-[2-[4-(p-fluorbenzoyl)-piperidinyl]-ethyl]-2-methoxy-benzamid

vom Smp. 166–168°; ausgehend von 43,5 g (0,21 Mol) p-Fluorbenzoyl-piperidin und 37,1 g (0,1 Mol)

N-(2-Bromethyl)-5-brom-4-chlor-2-methoxy-benzamid in 200 ml Dioxan.

Das Ausgangsmaterial wird wie folgt hergestellt:

13,3 g (0,05 Mol) 5-Brom-4-chlor-2-methoxy-benzoesäure in 10 ml Chloroform und 6,6 ml (10,7 g; 0,09 Mol) Thionylchlorid werden während 2 Stunden zum Sieden erhitzt. Dann dampft man im Rotationsverdampfer ein, wobei das 5-Brom-4-chlor-2-methoxy-benzoesäurechlorid als weisses Pulver zurückbleibt.

In analoger Weise wie in Beispiel 1 beschrieben erhält man:
N-(2-Bromethyl)-5-brom-4-chlor-2-methoxy-benzamid
vom Smp. 99–100°; ausgehend von 5,7 g (0,02 Mol) 5-Brom-4-chlor-2-methoxy-benzoesäurechlorid, 4,1 g (0,02 Mol) 2-Bromethylamin-hydrobromid und 5,2 g (0,04 Mol) Ethyl-diisopropylamin in 30 ml Methylenchlorid.

Beispiel 4:

In analoger Weise wie in Beispiel 1 beschrieben erhält man:
N-[2-[4-(p-Fluorbenzoyl)-piperidinyl]-ethyl]-4-fluor-2-methoxy-benzamid-hydrochlorid,
Smp. 211° unter Zersetzung; ausgehend von 41,4 g (0,2 Mol) 4-(p-Fluorbenzoyl)-piperidin und 27,6 g (0,1 Mol) N-(2-Bromethyl)-4-fluor-2-methoxy-benzamid in 120 ml Dioxan.

Das Ausgangsmaterial wird analog Beispiel 1 wie folgt hergestellt:
N-(2-Bromethyl)-4-fluor-2-methoxy-benzamid
vom Smp. 92–94°; ausgehend von 18,9 g (0,1 Mol) 4-Fluor-2-methoxy-benzoesäurechlorid (hergestellt nach US Pat. 3 177 252), 20,5 g (0,1 Mol) 2-Bromethylamin-hydrobromid und 27,1 g (0,21 Mol) Ethyl-diisopropylamin.

Beispiel 5:

In analoger Weise wie in Beispiel 1 beschrieben erhält man:
5-Brom-N-[2-[4-(p-fluorbenzoyl)-piperidinyl]-ethyl]-4-fluor-2-methoxy-benzamid
vom Smp. 152–153°; ausgehend von 45,5 g (0,22 Mol) 4-(p-Fluorbenzoyl)-piperidin und 35,5 g (0,1 Mol) N-(2-Bromethyl)-5-brom-4-fluor-2-methoxy-benzamid in 200 ml Dioxan.

Das Ausgangsmaterial wird wie folgt hergestellt:

In analoger Weise wie in Beispiel 2 beschrieben erhält man:
5-Brom-4-fluor-2-methoxy-benzoesäure vom Smp. 176–178°; ausgehend von 3,4 g (0,02 Mol) 4-Fluor-2-methoxy-benzoesäure, 1,07 ml (3,35 g; 0,21 Mol) Brom in 90 ml Eisessig und 140 ml Wasser.

In analoger Weise wie in Beispiel 3 beschrieben erhält man:
5-Brom-4-fluor-2-methoxy-benzoesäurechlorid als weisses Pulver; ausgehend von 5,0 g (0,02 Mol) 5-Brom-4-fluor-2-methoxy-benzoesäure, 4,3 ml (7,1 g; 0,06 Mol) Thionylchlorid und 15 ml Chloroform.

In analoger Weise wie in Beispiel 1 beschrieben erhält man:
N-(2-Bromethyl)-5-brom-4-fluor-2-methoxy-benzamid
vom Smp. 91–95°; ausgehend von 5,4 g (0,02 Mol) 5-Brom-4-fluor-2-methoxy-benzoesäurechlorid, 4,1 g (0,02 Mol) 2-Bromethylamin-hydrobromid und 5,2 g (0,04 Mol) Ethyl-diisopropylamin in 30 ml Methylenchlorid.

Beispiel 6:

N-[2-[4-(p-Fluorbenzoyl)-piperidinyl]-ethyl]-2-methoxy-5-trifluormethyl-benzamid-hydrochlorid,
Smp. 212° unter Zersetzung; ausgehend von 41,4 g (0,2 Mol) 4-(p-Fluorbenzoyl)-piperidin und 32,6 g (0,1 Mol) N-(2-Bromethyl)-2-methoxy-5-trifluormethyl-benzamid in 180 ml Dioxan.

Das Ausgangsmaterial wird analog Beispiel 1 wie folgt hergestellt:
N-(2-Bromethyl)-2-methoxy-5-trifluormethyl-benzamid
vom Smp. 85–86°; ausgehend von 23,9 g (0,1 Mol) 2-Methoxy-5-trifluormethyl-benzoesäurechlorid, (hergestellt nach FR.-Pat. 1 472 025), 20,5 g (0,1 Mol) 2-Bromethylamin-hydrobromid und 27,1 g (0,21 Mol) Ethyl-diisopropylamin.

Beispiel 7:

In analoger Weise wie in Beispiel 1 beschrieben erhält man:
2-Methoxy-5-sulfamoyl-N-[2-[4-(p-fluorbenzoyl)-piperidinyl]-ethyl]-benzamid
vom Smp. 183–185°; ausgehend von 45,5 g (0,22 Mol) 4-(p-Fluorbenzoyl)-piperidin und 33,7 g (0,1 Mol) N-(2-Bromethyl)-2-methoxy-5-sulfamoyl-benzamid in 250 ml Dimethylformamid.

Das Ausgangsmaterial wird analog Beispiel 1 wie folgt hergestellt:
N-(2-Bromethyl)-2-methoxy-5-sulfamoyl-benzamid
vom Smp. 179° unter Zersetzung; ausgehend von 26,5 g (0,1 Mol) 2-Methoxy-5-sulfamoyl-benzoesäurechlorid (hergestellt nach FR.-Pat. 1 472 025), 20,5 g 2-Bromethylamin-hydrobromid und 21,2 g (0,21 Mol) Triethylamin.

Beispiel 8:

24,1 g (0,1 Mol) 2-(5-Chlor-2-methoxy-4-methylaminophenyl)-2-oxazolin, 22,8 g (0,11 Mol) 4-(p-Fluorbenzoyl)-piperidin und 120 ml Dioxan werden in einer Stickstoffatmosphäre während 18 Stunden zum Sieden erhitzt. Die erhaltene dunkelbraune Lösung wird im Wasserstrahlvakuum eingedampft. Man erhält dickflüssiges Öl, das in 300 ml Aceton gelöst wird und unter Rühren mit einer etherischen Chlorwasserstofflösung bis zur kongosauren Reaktion versetzt wird, wobei das Produkt auszukristallisieren beginnt. Das ausgefallene 5-Chlor-N-[2-[4-(p-fluorbenzoyl)-piperidinyl]-ethyl]-2-methoxy-4-methylamino-benzamid-hydrochlorid wird abgenutscht. Es schmilzt bei 230° unter Zersetzung.

Das Ausgangsmaterial wird wie folgt hergestellt:

Zu einer Lösung von 21,6 g (0,1 Mol) 5-Chlor-2-methoxy-4-methylamino-benzoesäure (hergestellt nach S. Iwanami et al., J. Med. Chem. 1981, (24), 1224) in 250 ml Methylenchlorid werden unter Rühren und Stickstoffatmosphäre 11,2 g (0,11 Mol) Triethylamin zugetropft. Die erhaltene Lösung wird bei −10° mit 10,9 g (0,1 Mol) Chlorameisensäureethylester versetzt. Man rührt weitere 30 Minuten bei dieser Temperatur mit 20,5 g (0,1 Mol) 2-Bromethylamin-hydrobromid und 10,1 g (0,1 Mol) Triethylamin. Dann wird das Kühlbad entfernt und das Reaktionsgemisch während 4 Stunden bei Raumtemperatur gerührt. Zur Aufarbeitung versetzt man mit 1 Liter Methylenchlorid und 1 Liter Wasser, stellt die wässrige Phase mit 2-n. Salzsäure auf pH 5 ein und trennt die Schichten im Scheidetrichter. Die wässrige Phase wird mit Natriumbicarbonat versetzt und dann mit Methylenchlorid extrahiert. Nach dem Eindampfen des Methylenchlorids erhält man 2-(5-Chlor-2-methoxy-4-methylamino-phenyl)-2-oxazolin vom Smp. 159–161°.

Beispiel 9:

In analoger Weise wie in Beispiel 8 beschrieben erhält man:

N-[2-[4-(p-Fluorbenzoyl)-piperidinyl]-ethyl]-2-methoxy-5-dimethylsulfamoyl-benzamid-hydrochlorid

vom Smp. 172° unter Zersetzung, ausgehend von 28,4 g (0,1 Mol) 2-(5-Dimethylsulfamoyl-2-methoxy-phenyl)-2-oxazolin und 20,7 g (0,1 Mol) 4-(p-Fluorbenzoyl)-piperidin und 140 ml Dioxan.

Das Ausgangsmaterial wird analog Beispiel 8 wie folgt hergestellt:

2-(5-Dimethylsulfamoyl-2-methoxy-phenyl)-2-oxazolin

vom Smp. 135–138°; ausgehend von 24,3 g (0,1 Mol) 5-Dimethylsulfamoyl-2-methoxy-benzoesäure, 21,3 g (0,21 Mol) Triethylamin, 10,9 g (0,1 Mol) Chlorameisensäure-ethylester und 20,5 g (0,1 Mol) 2-Bromethylamin-hydrobromid.

Beispiel 10:

20,2 g (0,1 Mol) N-(5-Cyano-2-methoxy-benzoyl)-aziridin, 22,8 g (0,11 Mol) 4-(p-Fluorbenzoyl)-piperidin und 250 ml Toluol werden unter Rühren 5 Stunden auf 80° erwärmt. Nach dem Abkühlen wird das ausgefallene 5-Cyano-N-[2-[4-(p-fluorbenzoyl)-piperidinyl]-ethyl]-2-methoxy-benzamid abgenutscht und mit wenig Toluol nachgewaschen, Smp. 167–169°.

Das Ausgangsmaterial wird wie folgt hergestellt:

Zu einem Gemisch von 100 ml 0,5n-Natronlauge und 2,3 g (0,53 Mol) Aziridin in 30 ml Toluol wird unter gutem Rühren bei einer Reaktionstemperatur von 3–5° eine Lösung von 9,8 g (0,05 Mol) 5-Cyano-2-methoxy-benzoylchlorid in 50 ml Methylenchlorid zugetropft und das Reaktionsgemisch anschliessend noch eine Stunde bei 5° gerührt. Hierauf werden die Schichten im Scheidetrichter getrennt, die organische Phase mit 50 ml Wasser ausgeschüttelt, über Magnesiumsulfat getrocknet und im Wasserstrahlvakuum eingedampft. Als Rückstand verbleibt N-(5-Cyano-2-methoxy-benzoyl)-aziridin vom Smp. 99–101°.

Beispiel 11:

In analoger Weise wie in Beispiel 8 beschrieben erhält man:

3-Brom-N-[2-[4-(p-fluorbenzoyl)-piperidinyl]-ethyl]-4-methoxy-benzamid-methansulfonat,

Smp. 125°, ausgehend von 25,6 g (0,1 Mol) 2-(3-Brom-4-methoxy-phenyl)-2-oxazolin und 20,7 g (0,1 Mol) 4-(p-Fluorbenzoyl)-piperidin in 75 ml Toluol.

Das Ausgangsmaterial wird analog Beispiel 8 wie folgt hergestellt:

2-(3-Brom-4-methoxy-phenyl)-2-oxazolin vom Smp. 95°; ausgehend von 15,2 g (0,1 Mol) 3-Brom-4-methoxy-benzoesäure, 21,3 g (0,21 Mol) Triethylamin, 10,9 g (0,1 Mol) Chlorameisensäureethylester und 20,5 g (0,1 Mol) 2-Bromethylamin-hydrobromid.

Beispiel 12:

In analoger Weise wie in Beispiel 8 beschrieben erhält man:

N-[2-[4-(p-Fluorbenzoyl-piperidinyl]-ethyl]-2-methoxy-benzamid-hydrochlorid

vom Smp. 205° unter Zersetzung; ausgehend von 17,7 g (0,1 Mol) 2-(2-Methoxy-phenyl)-2-oxazolin und 10,7 g (0,1 Mol) 4-(p-Fluorbenzoyl)-piperidin und 40 ml Dioxan.

Das Ausgangsmaterial wird analog Beispiel 8 wie folgt hergestellt:

2-(2-Methoxy-phenyl)-2-oxazolin vom Smp. 35–38°; ausgehend von 15,2 g (0,1 Mol) 2-Methoxybenzoesäure, 21,3 g (0,21 Mol) Triethylamin, 10,9 g (0,1 Mol) Chlorameisensäure-ethylester und 20,5 g (0,1 Mol) 2-Bromethylamin-hydrobromid.

Beispiel 13:

Zu einer Suspension von 4,1 g (0,01 Mol)

5-Cyano-N-[2-[4-(p-fluorbenzoyl)-piperidin]-ethyl]-2-methoxy-benzamid

in 50 ml Ethanol werden unter Rühren 0,38 g (0,01 Mol) Natrium-borhydrid zugegeben und während 1 Stunde bei Raumtemperatur gerührt. Die erhaltene klare Lösung wird mit 1 ml Aceton versetzt und dann im Wasserstrahlvakuum eingedampft. Der Rückstand wird mit Methylenchlorid und Wasser aufgenommen, die Schichten im Scheidetrichter getrennt und die organische Phase nach dem Trocknen über Magnesiumsulfat eingedampft. Als Rückstand erhält man

5-Cyano-N-[2-[4-[(4-fluorphenyl)-hydroxymethylen]-piperidinyl]-ethyl]-2-methoxy-benzamid

vom Smp. 135–137°.

Beispiel 14:

Zu einem Gemisch von 32,3 g (0,1 Mol) N-(2-Aminoethyl)-4-(p-fluorbenzoyl)-piperidinhydrochlorid und 24 g 3-Brom-4-fluorbenzoylchlorid in 250 ml Methylenchlorid lässt man unter Rühren und Stickstoffatmosphäre bei −10° bis 0° 45 g (0,35 Mol) Diisopropylethylamin zutropfen. Anschliessend lässt man auf Raumtemperatur erwärmen und hält das Reaktionsgemisch während 4 Stunden bei dieser Temperatur. Dann versetzt man mit 200 ml 1N Natronlauge, trennt die Schichten und wäscht die organische Phase mit Wasser neutral, trocknet sie über Magnesiumsulfat und engt sie im Wasserstrahlvakuum ein. Als Rückstand verbleiben 3-Brom-4-fluor-N-[2-[4-(p-fluorbenzoyl)-piperidinyl]-ethyl]-benzamid vom Smp. 135°.

Zur Überführung ins Hydrochlorid wird diese Base in Methylenchlorid gelöst und mit etherischer Chlorwasserstofflösung versetzt. Das Hydrochlorid schmilzt bei 189–190°.

Das als Ausgangsmaterial verwendete N-(2-Aminoethyl)-4-(p-fluorbenzoyl)-piperidin-hydrochlorid wird wie folgt hergestellt:

Zu einer Suspension von 24,4 g (0,1 Mol) 4-(p-Fluorbenzoyl)-piperidin-hydrochlorid und 8,3 g (0,11 Mol) Chloracetonitril in 50 ml Methylenchlorid lässt man unter Rühren 29,5 g (0,23 Mol) Ethyldiisopropylamin zutropfen. Das Reaktionsgemisch wird während 15 Stunden bei Raumtemperatur gerührt. Hierauf versetzt man mit 150 ml Ether und schüttelt mit 2N Salzsäure aus. Dieser Säureauszug wird mit 2N Natronlauge alkalisch gestellt und die sich abscheidende Base mit einem 3:1 Ether-Methylenchlorid-Gemisch ausgeschüttelt. Dieses wird neutral gewaschen, über Magnesiumsulfat getrocknet und eingedampft. Als Rückstand verbleibt das N-(Cyano-methyl)-4-(p-fluorbenzoyl)-piperidin vom Smp. 133–134°.

14,1 g (0,05 Mol) N-(Cyanomethyl)-4-(p-fluorbenzoyl)-piperidin werden in 1,5 l Eisessig und 30 ml konz. Salzsäure gelöst und zusammen mit 2 g Platinoxid in einer Wasserstoffatmosphäre hydriert. Nach 6 Stunden wird die theoretische Menge von 2,25 l Wasserstoff aufgenommen. Hierauf wird vom Katalysator abgenutscht und das Filtrat im Wasserstrahlvakuum auf ca. 30 ml eingeengt. Der entstandene Kristallbrei wird mit 5 ml Isopropanol und 15 ml Ether versetzt und abgenutscht. Nach dem Abnutschen und Trocknen erhält man das N-(2-Aminoethyl)-4-(p-fluorbenzoyl)-piperidin-hydrochlorid, das nach einer Umkristallisation aus Methanol bei 265° unter Zersetzung schmilzt.

Das als Ausgangsmaterial verwendete 3-Brom-4-fluor-benzoylchlorid wird analog Beispiel 3 aus 21,9 g (0,1 Mol) 3-Brom-4-fluorbenzoesäure (hergestellt nach J. Indian Chem. Soc. 21, 115 (1944)) und 10 ml Thionylchlorid hergestellt.

Beispiel 15:

In analoger Weise wie in Beispiel 14 beschrieben erhält man:

2-Brom-4-fluor-N-[2-[4-(p-fluorbenzoyl)-piperidinyl]-ethyl]-benzamid

vom Smp. 123–125°; ausgehend von 3,23 g (0,01 Mol) N-(2-Aminoethyl)-4-(p-fluorbenzoyl)-piperidin-hydrochlorid, 2,6 g (0,01 Mol) 2-Brom-4-fluorbenzoylchlorid und 4,5 g (0,035 Mol) Ethyl-diisopropylamin in 25 ml Methylenchlorid.

N-[2-[4-(p-Fluorbenzoyl)-piperidinyl]-ethyl]-2,6-dimethoxy-benzamid-hydrochlorid

vom Smp. 232–234° unter Zersetzung; ausgehend von 32,3 g (0,1 Mol) N-(2-Aminoethyl)-4-(p-fluorbenzoyl)-piperidin-hydrochlorid, 20 g 2,6-Dimethoxy-benzoylchlorid und 45 g (0,35 Mol) Ethyl-diisopropylamin in 200 ml Methylenchlorid.

Beispiel 16:

In analoger Weise wie in Beispiel 1 beschrieben erhält man:

4-Fluor-N-[2-[4-(p-fluorbenzoyl)-piperidinyl]-ethyl]-benzamid-hydrochlorid,

Smp. 240–242°; ausgehend von 6,2 g (0,03 Mol) 4-(p-Fluorbenzoyl)-piperidin und 7,4 g (0,03 Mol) N-(2-Bromethyl)-4-fluorbenzamid in 25 ml Dimethylformamid.

Beispiel 17:

In analoger Weise wie in Beispiel 10 beschrieben erhält man:

5-Brom-N-[2-[4-(p-fluorbenzoyl)-piperidinyl]-ethyl]-2-methoxy-benzamid-hydrochlorid

vom Smp. 213° unter Zersetzung; ausgehend von 5,2 g (0,02 Mol) N-(5-Brom-2-methoxybenzoyl)-aziridin und 4,4 g (0,021 Mol) 4-(p-Fluorbenzoyl)-piperidin in 16 ml Toluol.

Das Ausgangsmaterial wird analog Beispiel 10 wie folgt hergestellt:

N-(5-Brom-2-methoxy-benzoyl)-aziridin; ausgehend von 9 g (0,21 Mol) Aziridin, 27,1 g (0,21 Mol) Ethyl-diisopropylamin und 50 g (0,02 Mol) 5-Brom-2-methoxy-benzoylchlorid in 300 Methylenchlorid.

In analoger Weise wie in Beispiel 10 beschrieben erhält man:

3-Cyano-N-[2-[4-(p-fluorbenzoyl)-piperidinyl]-ethyl]-4-methoxy-benzamid-hydrochlorid

vom Smp. 230° unter Zersetzung; ausgehend von 20,2 g (0,1 Mol) N-(3-Cyano-4-methoxybenzoyl)-aziridin und 22,8 g (0,11 Mol) 4-(p-Fluorbenzoyl)-piperidin in 120 ml Toluol.

N-(3-Cyano-4-methoxy-benzoyl)-aziridin vom Smp. 176–177°; ausgehend von 4,52 g (0,105 Mol) Aziridin, 12,9 g (0,1 Mol) Ethyldiisopropylamin und 19,6 g (0,1 Mol) 3-Cyano-4-methoxy-benzoylchlorid in 150 ml Methylenchlorid.

Beispiel 18:

In analoger Weise wie in Beispiel 14 beschrieben erhält man:

3,5-Dichlor-N-[2-[4-(p-fluorbenzoyl)-piperidinyl]-ethyl]-2-methoxy-4-methyl-benzamid-methansulfonat

vom Smp. 112–114°; ausgehend von 3,23 g (0,01 Mol) N-(2-Aminoethyl)-4-(p-fluorbenzoyl)-piperidin-hydrochlorid, 2,54 g (0,01 Mol) 3,5-Dichlor-2-methoxy-4-methyl-benzoylchlorid und 4,5 g (0,035 Mol) Ethyl-diisopropylamin in 25 ml Methylenchlorid.

N-[2-[4-(p-Fluorbenzoyl)-piperidinyl]-ethyl]-2-methoxy-5-methylmercapto-benzamid hydrochlorid

vom Smp. 209° unter Zersetzung; ausgehend von 3,23 g (0,01 Mol) N-(2-Aminoethyl)-4-(p-fluorbenzoyl)-piperidin-hydrochlorid, 2,17 g (0,01 Mol) 2-Methoxy-5-methylmercapto-benzoylchlorid und 4,5 g (0,035 Mol) Ethyl-diisopropylamin in 30 ml Methylenchlorid.

N-[2-[4-(p-Fluorbenzoyl)-piperidinyl]-ethyl]-
2-methoxy-5-methansulfonyl-benzamid-
hydrochlorid
vom Smp. 163–164°; ausgehend von 6,5 g (0,02 Mol) N-(2-Aminoethyl)-4-(p-fluorbenzoyl)-piperidin-hydrochlorid, 4,99 g (0,02 Mol) 2-Methoxy-5-methansulfonyl-benzoylchlorid und 9 g (0,07 Mol) Ethyldiisopropylamin in 50 ml Methylenchlorid.

Beispiel 19:

In analoger Weise wie in Beispiel 14 beschrieben erhält man:
5-Cyano-N-[2-[4-(p-fluorbenzyl)-piperidinyl]-
ethyl]-2-methoxy-benzamid-hydrochlorid
vom Smp. 147–148°; ausgehend von 4,6 g (0,015 Mol) N-(2-Aminoethyl)-4-(p-fluorbenzyl)-piperidin-dihydrochlorid, 2,93 g (0,015 Mol) 5-Cyano-2-methoxy-benzoylchlorid und 6,4 g (0,05 Mol) Ethyldiisopropylamin in 35 ml Methylenchlorid.

Das als Ausgangsmaterial verwendete N-(2-Aminoethyl)-4-(p-fluorbenzyl)-piperidin-dihydrochlorid wird wie folgt hergestellt:

24,4 g (0,10 Mol) 4-(p-Fluorbenzoyl)-piperidin-hydrochlorid werden in 120 ml Trifluoressigsäure mit 5 g Palladium-Kohle (5%ig) bei 70° bis zur Aufnahme der theoretischen Wasserstoffmenge hydriert. Hierauf wird vom Katalysator abgenutscht und das Filtrat im Wasserstrahlvakuum eingedampft. Der Rückstand wird in 150 ml Wasser gelöst, mit 50 ml konz. Natronlauge versetzt und dann mit 250 ml Ether extrahiert. Die so erhaltene etherische Lösung wird mit Chlorwasserstoffgas auf schwach kongosauer gestellt, wobei das 4-(p-Fluorbenzyl)-piperidin-hydrochlorid ausfällt, Smp. 166–166,5°.

Zu einer Suspension von 23 g (0,10 Mol) 4-(p-Fluorbenzyl)-piperidin-hydrochlorid und 8,3 g (0,11 Mol) Chloracetonitril in 75 ml Methylenchlorid lässt man unter Rühren 28,4 g (0,22 Mol) Ethyldiisopropylamin zutropfen. Das Reaktionsgemisch wird während 15 Stunden bei Raumtemperatur gerührt. Hierauf versetzt man mit 150 ml Ether und schüttelt mit 2N Salzsäure aus. Dieser Säureauszug wird mit 2N Natronlauge alkalisch gestellt und die sich abscheidende Base mit Methylenchlorid ausgeschüttelt. Dieses wird mit Wasser neutral gewaschen, über Natriumsulfat getrocknet und eingedampft. Der Rückstand wird in wenig Isopropanol gelöst und mit etherischer Chlorwasserstofflösung auf schwach kongosauer gestellt, wobei das N-(Cyanomethyl)-4-(p-fluorbenzyl)-piperidin-hydrochlorid vom Smp. 158–160° ausfällt.

13,44 g (0,05 Mol) N-(Cyanomethyl)-4-(p-fluorbenzyl)-piperidin-hydrochlorid werden in 300 ml Eisessig und 7 ml konz. Salzsäure gelöst und zusammen mit 1 g Platinoxid in einer Wasserstoffatmosphäre hydriert. Nach 4 Stunden wird die theoretische Menge von 2,25 l Wasserstoff aufgenommen. Hierauf wird vom Katalysator abgenutscht und das Filtrat im Wasserstrahlvakuum auf ca. 30 ml eingeengt. Der entstandene Kristallbrei wird mit 5 ml Isopropanol und 10 ml Ether versetzt und abgenutscht. Man erhält so das N-(2-Aminoethyl)-4-(p-fluorbenzyl)-piperidin-hydrochlorid, das nach Umkristallisation aus Ethanol-Ether bei 171–173° schmilzt.

Beispiel 20:

6,69 g (0,015 Mol)
5-Cyano-N-[2-[4-(p-fluorbenzoyl)-piperidinyl]-
ethyl]-2-methoxy-benzamid-hydrochlorid
werden in 50 ml Methanol und 50 ml Toluol suspendiert. Unter Rühren und Kühlen wird bei 0° Chlorwasserstoffgas eingeleitet bis eine klare Lösung entsteht. Dann lässt man langsam 100 ml Eiswasser zulaufen und erwärmt dann noch 1 Stunde auf 70°. Nach dem Abkühlen wird mit Pottasche vorsichtig auf schwach alkalisch gestellt und das so erhaltene Gemisch mit Methylenchlorid extrahiert. Diese Methylenchloridlösung wird mit Wasser neutral gewaschen, über Magnesiumsulfat getrocknet und im Wasserstrahlvakuum eingedampft. Das als Rückstand verbleibende N-[2-[4-(p-Fluorbenzoyl)-piperidinyl]-ethyl]-2-methoxy-5-methoxycarbonyl-benzamid wird in Methylenchlorid-Ether 1:4 gelöst und mit etherischer Chlorwasserstofflösung ins Hydrochlorid überführt. Dieses wird abgenutscht und einmal aus Methylenchlorid-Aceton umkristallisiert, Smp. 221° unter Zersetzung.

Beispiel 21:

6,69 g (0,015 Mol)
5-Cyano-N-[2-[4-(p-fluorbenzoyl)-piperidinyl]-
ethyl]-2-methoxy-benzamid-hydrochlorid
werden unter Stickstoffatmosphäre in 60 ml 85% Schwefelsäure gelöst und während 7 Tagen bei Raumtemperatur stehen gelassen. Dann tropft man diese Lösung unter Rühren zu einem Gemisch von 130 ml konz. Ammoniak und 200 g Eis, wobei sich ein weisser Niederschlag bildet. Dieser wird abgenutscht und aus Methylenchlorid-Methanol umkristallisiert. Man erhält so das 5-Carbamoyl-N-[2-[4-(p-fluorbenzoyl)-piperidinyl]-ethyl-2-methoxy-benzamid vom Smp. 236–238°.

Zur Überführung ins Hydrochlorid suspendiert man die freie Base in Methylenchlorid und versetzt diese unter Rühren mit einer etherischen Chlorwasserstofflösung bis zur kongosauren Reaktion, wobei eine klare Lösung resultiert. Anschliessend versetzt man bis zur beginnenden Kristallisation mit Ether. Man erhält so das 5-Carbamoyl-N-[2-[4-(p-fluorbenzoyl)-piperidinyl]-ethyl]-2-methoxy-benzamid-hydrochlorid vom Smp. 240° unter Zersetzung.

Beispiel 22:

2,14 g (0,01 Mol) 2-Methoxy-5-methansulfinyl-benzoesäure und 3,23 g (0,01 Mol) N-(2-Aminoethyl)-4-(p-fluorbenzoyl)-piperidin-dihydrochlorid werden in 10 ml Dimethylformamid gelöst und mit 2,13 g (0,021 Mol) Triethylamin versetzt. Unter Rühren und Stickstoffatmosphäre versetzt man mit 3,26 g (0,0105 Mol) Triphenylphosphit und erhitzt das erhaltene Reaktionsgemisch 2,5 Stunden auf 85–90°. Nach dem Abkühlen versetzt man mit etherischer Chlorwasserstofflösung bis zur kongosauren Reaktion und dekantiert dann die abgeschiedene etherische Lösung ab. Der Rückstand

wird mit Methylenchlorid und Kaliumcarbonatlösung versetzt, die Schichten getrennt und die Methylenchloridlösung über Pottasche getrocknet und eingeengt. Das als Rückstand zurückbleibende

N-[2-[4-(p-Fluorbenzoyl)-piperidinyl]-ethyl]-2-methoxy-5-methansulfinyl-benzamid

wird in Methylenchlorid-Methanol mit etherischer Chlorwasserstofflösung ins Hydrochlorid überführt. Dieses wird durch Zugabe von Ether ausgefällt. Man erhält so das

N-[2-[4-(p-Fluorbenzoyl)-piperidinyl]-ethyl]-2-methoxy-5-methansulfinyl-benzamid-hydrochlorid,

das nach einmaliger Umkristallisation aus Methylenchlorid-Methanol bei 194° unter Zersetzung schmilzt.

Beispiel 23:

In analoger Weise wie in Beispiel 10 beschrieben erhält man:

5-Cyano-2-methoxy-N-[2-[4-(2-thenoyl)-piperidinyl]-ethyl]-benzamid-hydrochlorid

vom Smp. 168° unter Zersetzung; ausgehend von 4,04 g (0,02 Mol) N-(5-Cyano-2-methoxybenzoyl)-aziridin und 3,9 g (0,02 Mol) 4-(2-Thenoyl)-piperidin in 22 ml Toluol.

p-Fluor-N-[2-[4-(2-thenoyl)-piperidinyl]-ethyl]-benzamid-hydrochlorid

vom Smp. 204–205°; ausgehend von 4,95 g (0,03 Mol) p-Fluorbenzoyl-aziridin und 5,85 g (0,03 Mol) 4-[2-Thenoyl]-piperidin in 25 ml Toluol.

N-[2-[4-(p-Chlorbenzoyl)-piperidinyl]-ethyl]-5-cyano-2-methoxy-benzamid-methansulfonat

vom Smp. 177–178°; ausgehend von 4,04 g (0,02 Mol) N-(5-Cyano-2-methoxy-benzoyl)-aziridin und 4,46 g (0,02 Mol) 4-(p-Chlorbenzoyl)-piperidin in 20 ml Toluol.

N-[2-[4-(p-Chlorbenzoyl)-piperidinyl]-ethyl]-p-fluor-benzamid-methansulfonat

vom Smp. 149–151°; ausgehend von 3,3 g (0,02 Mol) p-Fluorbenzoyl-aziridin und 4,46 g (0,02 Mol) 4-(p-Chlorbenzoyl)-piperidin in 20 ml Toluol.

Beispiel 24:

In analoger Weise wie in Beispiel 10 beschrieben erhält man:

p-Fluor-N-[3-[4-(p-fluorbenzoyl)-piperidinyl]-2-methylpropyl]-benzamid-hydrochlorid

vom Smp. 238–240°; ausgehend von 1,93 g (0,01 Mol) N-(p-Fluorbenzoyl)-2,2-dimethyl-aziridin und 2,07 g (0,01 Mol) 4-(p-Fluorbenzoyl)-piperidin in 10 ml Toluol.

Das als Ausgangsmaterial verwendete N-(p-Fluorbenzoyl)-2,2-dimethyl-aziridin wird analog Beispiel 10 wie folgt hergestellt:

N-(p-Fluorbenzoyl)-2,2-dimethyl-aziridin, dickflüssiges Öl; ausgehend von 2,13 g (0,03 Mol) 2,2-Dimethyl-aziridin und 4,76 g (0,03 Mol) p-Fluorbenzoylchlorid in 30,6 ml 1N Natronlauge.

Beispiel 25:

26,8 g (0,11 Mol) 4-(p-Fluorbenzoyl)-piperidin-hydrochlorid, 34,5 g (0,25 Mol) Pottasche und 25,3 g (0,1 Mol) N-(3-Chlorpropyl)-5-cyano-2-methoxy-benzamid werden zusammen mit 0,5 g Kaliumiodid in 250 ml Ethanol unter Rühren während 15 Stunden zum Sieden erhitzt. Dann wird das Reaktionsgemisch im Wasserstrahlvakuum eingedampft und der Rückstand mit Ether, wenig Methylenchlorid und Wasser aufgenommen. Nach dem Abtrennen der wässrigen Phase werden die basischen Anteile aus der organischen Phase durch Ausschütteln mit 2N Methansulfonsäure abgetrennt. Die sauren Auszüge werden mit Natronlauge auf pH 9 gestellt und dann mit Methylenchlorid extrahiert. Die Methylenchloridlösung wird mit Wasser neutral gewaschen, über Magnesiumsulfat getrocknet und im Wasserstrahlvakuum eingedampft. Das so erhaltene Rohprodukt wird einer Flash-Chromatographie unterworfen, wobei die Chloroform-Methanol 49:1-Fraktionen das gewünschte 5-Cyano-N-[3-[4-(p-fluorbenzoyl)-piperidinyl]-propyl]-2-methoxy-benzamid vom Smp. 133–135° eluieren.

Zur Überführung ins Hydrochlorid löst man die freie Base in Methylenchlorid und versetzt diese mit einer etherischen Chlorwasserstofflösung bis zur kongosauren Reaktion. Anschliessend versetzt man bis zur beginnenden Kristallisation mit Ether. Man erhält so das 5-Cyano-N-[3-[4-(p-fluorbenzoyl)-piperidinyl]-propyl]-2-methoxy-benzamid vom Smp. 171° unter Zersetzung.

Beispiel 26:

20,8 g (0,1 Mol) 2-(2,6-Dimethoxy-pyridinyl)-2-oxazolin, 22,8 g (0,11 Mol) 4-(p-Fluorbenzoyl)-piperidin, 12,9 g (0,1 Mol) Diisopropyl-ethylamin und 120 ml Dioxan werden unter Rühren während 16 Stunden auf 80° erwärmt. Hierauf versetzt man mit Methylenchlorid und Wasser und stellt mit gesättigter Kaliumcarbonatlösung auf pH 9 ein. Die Schichten werden im Scheidetrichter getrennt und die organische Phase im Wasserstrahlvakuum eingedampft. Der erhaltene Rückstand wird in 150 ml Methanol gelöst und unter Rühren mit Wasser versetzt, wobei das N-[2-[4-(p-Fluorbenzoyl)-piperidinyl]-ethyl]-2,6-dimethoxy-nicotinsäureamid ausfällt. Smp. 116–119°.

Zur Überführung ins Methansulfonat löst man die freie Base in wenig Methylenchlorid und versetzt dann unter Rühren mit einer etherischen Methansulfonsäurelösung bis zur schwach kongosauren Reaktion, wobei das N-[2-[4-(1-Fluorbenzoyl)-piperidinyl]-ethyl]-2,6-nicotinsäureamid-methansulfonat ausfällt. Das Rohprodukt wird 1 mal aus Isopropanol umkristallisiert. Smp. 170–172°.

Das Ausgangsmaterial wird wie folgt hergestellt:

110,6 g (0,8 Mol) Kaliumcarbonat werden in 120 ml Wasser gelöst und auf 10° abgekühlt. Man versetzt mit einem Gemisch von 150 ml Ether und 100 ml Methylenchlorid, gibt dann 49,2 g (0,24 Mol) 2-Bromethylamin-hydrobromid hinzu und tropft dann eine Lösung von 40,3 g (0,2 Mol) 2,6-Dimethoxy-nicotinsäurechlorid in 100 ml Methylenchlorid bei einer Reaktionstemperatur von 15–20° rasch zu. Dann lässt man auf Raumtemperatur erwärmen und rührt noch 1 Stunde bei dieser Temperatur. Hierauf versetzt man mit 50 ml Wasser und trennt die Schichten im Scheidetrichter. Die organische Phase wird im Wasserstrahlvakuum eingedampft, wobei das 2-(2,6-Dimethoxy-pyrimidinyl)-2-oxazolin als leicht gelbliche Kristalle zurückbleibt.

Beispiel 27:

Tabletten enthaltend 25 mg Wirkstoff, z.B. 5-Cyano-N-[2-[4-(p-fluorbenzoyl)-piperidinyl]-ethyl]-2-methoxy-benzamid, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung:

Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig, unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Maschenweite verpresst.

Beispiel 28:

Tabletten, enthaltend 0,02 g des Methansulfonsäuresalzes von 5-Cyano-N-[2-[4-(p-fluorbenzoyl)-piperidinyl]-ethyl]-2-methoxy-benzamid werden wie folgt hergestellt:

| Zusammensetzung (für 10 000 Tabletten) | |
|---|---|
| Wirkstoff | 200,00 g |
| Lactose | 290,80 g |
| Kartoffelstärke | 274,70 g |
| Stearinsäure | 10,00 g |
| Talk | 200,00 g |
| Magnesiumstearat | 2,50 g |
| Kolloidales Siliciumdioxid | 32,00 g |
| Ethanol | q.s. |

Ein Gemisch des Wirkstoffs, der Lactose und 194,70 g Kartoffelstärke wird mit einer ethanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewüschtenfalls mit Teilkerben zur feineren Anpassung der Dosierung versehen sein können.

Beispiel 29:

Kapseln, enthaltend 0,025 g des Wirkstoffs, z.B. 5-Cyano-[2-[4-(p-fluorbenzoyl)-piperidinyl]-ethyl]-2-methoxy-benzamid, können wie folgt hergestellt werden:

| Zusammensetzung (für 1000 Kapseln) | |
|---|---|
| Wirkstoff | 25,00 g |
| Lactose | 249,00 g |
| Gelatine | 2,00 g |
| Maisstärke | 10,00 g |
| Talk | 15,00 g |
| Wasser | q.s. |

Man mischt den Wirkstoff mit der Lactose, befeuchtet die Mischung gleichmässig mit einer wässrigen Lösung der Gelatine und granuliert sie durch ein Sieb mit einer Maschenweite von 1,2–1,5 mm. Das Granulat mischt man mit der getrockneten Maisstärke und dem Talk und füllt Portionen von 300 mg in Hartgelatinekapseln (Grösse 1) ab.

**Patentansprüche für die Vertragsstaaten: BE, CH, DE, FR, GB, IT, LU, LI, NL, SE**

1. N-(Piperidinyl-alkyl)-carboxamide der allgemeinen Formel I

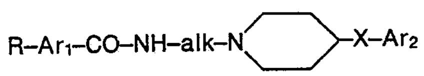 (I)

worin

R Hydroxy, Niederalkoxy, Niederalkenyloxy oder Halogen bedeutet,

$Ar_1$ für jeweils unsubstituiertes oder ein- oder mehrfach zusätzlich durch Niederalkyl, Niederalkenyl, Niederalkadienyl, Halogenniederalkyl, Hydroxy, Niederalkanoyloxy, Halogen, Niederalkoxy, Niederalkenyloxy, Halogenniederalkoxy, Niederalkanoyl, Nitro, Cyano, Carbamoyl, N-Niederalkyl-carbamoyl, N,N-Diniederalkyl-carbamoyl, Carboxy, Niederalkoxycarbonyl, Amino, N-Niederalkyl-amino, N,N-Diniederalkylamino, Sulfamoyl, N-Niederalkylsulfamoyl, N,N-Diniederalkyl-sulfamoyl, Niederalkylthio, Halogenniederalkylthio, Niederalkansulfinyl, Halogenniederalkansulfinyl, Niederalkansulfonyl, und/oder Halogenniederalkansulfonyl substituiertes Phenylen bzw. über ein C-Atom gebundenes, monocyclisches Azaarylen mit bis und mit 3 Stickstoffatomen steht,

alk die beiden N-Atome durch 2 bis 3 C-Atome trennendes Alkylen mit 2 bis und mit 7 C-Atomen darstellt,

X für Carbonyl, Diniederalkoxymethylen, Niederalkylendioxymethylen, Hydroxymethylen, Niederalkanoyloxymethylen oder Methylen, steht,

und $Ar_2$ einen jeweils unsubstituierten oder ein- oder mehrfach durch Niederalkyl, Niederalkenyl, Niederalkadienyl, Halogenniederalkyl, Hydroxy, Niederalkanoyloxy, Halogen, Niederalkoxy, Niederalkenyloxy,

Halogenniederalkoxy, Niederalkanoyl, Nitro, Cyano, Carbamoyl, N-Niederalkyl-carbamoyl, N,N-Diniederalkyl-carbamoyl, Carboxy, Niederalkoxycarbonyl, Amino, N-Niederalkyl-amino, N,N-Diniederalkyl-amino, Sulfamoyl, N-Niederalkyl-sulfamoyl, N,N-Diniederalkyl-sulfamoyl, Niederalkylthio, Halogenniederalkylthio, Niederalkansulfinyl, Halogenniederalkansulfinyl, Niederalkansulfonyl und/oder Halogenniederalkansulfonyl substituierten Phenylrest bzw. jeweils monocyclischen Monooxa-, Monoaza- oder Monothiaarylrest darstellt, und ihre Salze,
mit der Massgabe, dass die Gruppierung R–$Ar_1$– von dem Rest der Formel Ia

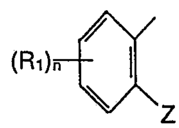

(Ia)

verschieden ist, worin $R_1$ ausgewählt ist aus der Gruppe bestehend aus Niederalkyl, Trifluormethyl, Halogen, Niederalkoxy, Nitro und Cyano und mindestens einer der Reste $R_1$ Halogen oder Niederalkoxy bedeutet, n für eine ganze Zahl von 1 bis 3 steht und Z Nitro, Amino oder Niederalkylamino darstellt, wenn alk und X die angegebenen Bedeutungen haben und $Ar_2$ unsubstituiertes Phenyl, Thienyl bzw. Pyridyl oder substituiertes Phenyl mit einem, zwei oder drei Substituenten ausgewählt aus der Gruppe bestehend aus Halogen, Niederalkyl, Niederalkoxy, Trifluormethyl und Amino bedeutet, wobei unter mit «Nieder» bezeichneten Resten solche zu verstehen sind, die bis und mit 7 C-Atomen aufweisen.

2. Verbindungen gemäss Anspruch 1 der Formel I, worin R Niederalkoxy,

$Ar_1$ für unsubstituiertes oder jeweils ein- oder mehrfach zusätzlich durch Niederalkyl, Halogenniederalkyl, Halogen, Niederalkoxy, Cyano, Carbamoyl, Amino, N-Niederalkylamino, N,N-Diniederalkylamino, Sulfamoyl, N,N-Diniederalkyl-sulfamoyl, Halogenniederalkylthio, Niederalkansulfonyl und/oder Halogenniederalkansulfonyl substituiertes Phenylen bzw. Pyridylen steht,

alk die beiden N-Atome durch 2 bis 3 C-Atome trennendes Alkylen mit 2 oder 3 C-Atomen darstellt, X für Carbonyl, Hydroxymethylen oder Methylen steht, und $Ar_2$ durch Halogen substituiertes Phenyl oder unsubstituiertes Thienyl darstellt, und ihre Salze.

3. Verbindungen gemäss Anspruch 1 der Formel I, worin einerseits R Niederalkoxy oder Halogen bedeutet und $Ar_1$ für unsubstituiertes oder zusätzlich ein- oder mehrfach durch Niederalkyl, Halogenniederalkyl, Hydroxy, Halogen, Niederalkoxy, Cyano, Carbamoyl, Niederalkoxycarbonyl, Amino, Niederalkylamino, Diniederalkylamino, Sulfamoyl, N,N-Diniederalkyl-sulfamoyl, Niederalkylthio, Niederalkansulfinyl und/oder Niederalkansulfonyl substituiertes Phenylen steht,

oder worin andererseits R Niederalkoxy bedeutet, und $Ar_1$ für unsubstituiertes oder durch Niederalkoxy substituiertes Pyridylen steht, und jeweils alk die beiden N-Atome durch 2 bis 3 C-Atome trennendes Alkylen mit 2 bis und mit 7 C-Atomen darstellt, X für Carbonyl, Hydroxymethylen oder Methylen steht und $Ar_2$ durch Halogen substituiertes Phenyl oder unsubstituiertes Thienyl darstellt, und ihre Salze.

4. Verbindungen gemäss Anspruch 1 der Formel I, worin

R jeweils Niederalkoxy bedeutet,

$Ar_1$ einerseits für einen unsubstituierten oder ein- oder mehrfach zusätzlich durch Halogenniederalkyl, Halogen, Niederalkoxy, Cyano, Carbamoyl, N-Niederalkylamino, Sulfamoyl, N,N-Diniederalkyl-sulfamoyl, Halogenniederalkylthio und/ oder Halogenniederalkan-sulfonyl substituierten Phenylrest, andererseits für einen ein- oder mehrfach zusätzlich durch Niederalkoxy und/oder Halogen substituierten Pyridylenrest steht,

alk Ethylen oder 1,3-Propylen darstellt,

X für Carbonyl, Hydroxymethylen oder Methylen steht, und

$Ar_2$ einen durch Halogen, substituierten Phenylrest darstellt, und ihre Salze.

5. Verbindungen gemäss Anspruch 1 der Formel I, worin die Gruppierung R–$Ar_1$ für das Strukturelement der Formel Ib

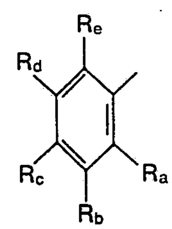

(Ib)

steht, in dem einer der Reste $R_a$ und $R_c$ den Rest R und dieser Niederalkoxy oder Halogen bedeutet und der andere Wasserstoff, Niederalkyl, Halogen oder Niederalkylamino darstellt, und die Reste $R_b$, $R_d$ und $R_e$ unabhängig voneinander für Wasserstoff, Niederalkyl, Halogenniederalkyl, Hydroxy, Halogen, Niederalkoxy, Cyano, Carbamoyl, Niederalkoxycarbonyl, Amino, Sulfamoyl, N,N-Diniederalkyl-sulfamoyl, Niederalkylthio, Niederalkansulfinyl und/oder Niederalkansulfonyl bedeuten, und worin alk für die beiden N-Atome durch 2 bis 3 C-Atome trennendes Alkylen mit 2 bis und mit 4 C-Atomen steht, X für Carbonyl, Hydroxymethylen oder Methylen steht und $Ar_2$ durch Halogen substituiertes Phenyl oder unsubstituiertes Thienyl darstellt, und ihre Salze.

6. Verbindungen gemäss Anspruch 1 der Formel I, worin die Gruppierung R–$Ar_1$ für das Strukturelement der Formel Ib gemäss Anspruch 5 steht, in dem einerseits der Rest $R_a$ den Rest R und dieser Niederalkoxy mit bis und mit 4 C-Atomen bedeutet und $R_c$ Wasserstoff, Halogen mit Atomnummer bis und mit 35 oder Niederalkylamino mit bis und mit 4 C-Atomen darstellt, bzw. $R_a$ Wasserstoff ist und $R_c$ den Rest R und dieser Niederalkoxy mit bis und mit 4 C-Atomen bedeutet, oder in dem andererseits der Rest $R_c$ den Rest R und dieser Halogen mit Atomnummer bis und mit 35 bedeutet und $R_a$ Wasserstoff oder Halogen mit Atomnummer bis und mit 35 darstellt und einer

der Reste $R_b$ und $R_d$ für Wasserstoff, Niederalkyl mit bis und mit 4 C-Atomen, Halogenniederalkyl mit Atomnummer bis und mit 35 und mit bis und mit 4 C-Atomen, Halogen mit Atomnummer bis und mit 35, Niederalkoxy mit bis und mit 4 C-Atomen, Cyano, Carbamoyl, Niederalkoxycarbonyl mit 2 bis und mit 5 C-Atomen, Niederalkylthio mit bis und mit 4 C-Atomen, Niederalkansulfinyl mit bis und mit 4 C-Atomen oder Niederalkansulfonyl mit bis und mit 4 C-Atomen steht, und der andere sowie $R_e$ Wasserstoff sind, und worin jeweils alk wie in Anspruch 5 angegeben definiert ist, X für Carbonyl oder Hydroxymethylen steht und $Ar_2$ in p-Stellung durch Halogen mit Atomnummer bis und mit 35 substituiertes Phenyl darstellt, und ihre Salze.

7. Verbindungen gemäss Anspruch 1 der Formel I, worin $Ar_1$ einerseits ein- oder mehrfach zusätzlich durch Halogen mit Atomnummer bis und mit 35, Cyano und/oder N-Niederalkylamino mit bis und mit 4 C-Atomen im Niederalkylteil substituiertes Phenylen bedeutet oder andererseits Pyridylen darstellt, und jeweils R Niederalkoxy bis und mit 4 C-Atomen bedeutet, alk Ethylen ist, X Carbonyl darstellt, und $Ar_2$ für durch Halogen mit Atomnummer bis und mit 35 substituiertes Phenyl steht, und ihre Salze.

8. Verbindungen gemäss Anspruch 1 der Formel I, worin die Gruppierung $R-Ar_1-$ für das Strukturelement der Formel Ib gemäss Anspruch 5 steht, in dem einerseits $R_a$ den Rest R und dieser Niederalkoxy mit bis und mit 4 C-Atomen bedeutet, $R_b$ und $R_e$ Wasserstoff sind, $R_c$ Niederalkylamino mit bis und mit 4 C-Atomen darstellt und $R_d$ Halogen mit Atomnummer bis und mit 35 bedeutet oder $R_c$ Wasserstoff oder Halogen mit Atomnummer bis und mit 35 darstellt und $R_d$ Cyano ist, oder in dem andererseits $R_a$, $R_d$ und $R_e$ Wasserstoff sind, $R_b$ Halogen mit Atomnummer bis und mit 35 und $R_c$ den Rest R und dieser Niederalkoxy mit bis und mit 4 C-Atomen darstellt, und jeweils alk Ethylen bedeutet, X für Carbonyl oder Hydroxymethylen steht, und $Ar_2$ 4-Fluorphenyl darstellt, und ihre Salze.

9. Verbindungen gemäss Anspruch 1 der Formel I, worin die Gruppierung $R-Ar_1-$ für das Strukturelement der Formel Ib gemäss Anspruch 5 steht, in dem einer der Reste $R_a$ und $R_b$ Wasserstoff oder Halogen mit Atomnummer bis und mit 35 darstellt, und der andere Wasserstoff bedeutet, $R_c$ den Rest R und dieser Halogen mit Atomnummer bis und mit 35 darstellt und $R_d$ und $R_e$ jeweils Wasserstoff sind, und alk Ethylen ist, X für Carbonyl steht und $Ar_2$ p-Fluorphenyl bedeutet, und ihre Salze.

10. Verbindungen gemäss Anspruch 1 der Formel I, worin die Gruppierung $R-Ar_1-$ für das Strukturelement der Formel Ib gemäss Anspruch 5 steht, in dem $R_a$, $R_d$ und $R_e$ Wasserstoff sind, $R_b$ Wasserstoff oder Halogen mit Atomnummer bis und mit 35 bedeutet, und $R_c$ den Rest R und dieser Halogen mit Atomnummer bis und mit 35 darstellt, alk Ethylen bedeutet, X für Carbonyl steht und $Ar_2$ 4-Fluorphenyl darstellt, und ihre Salze.

11. Verbindungen gemäss Anspruch 1 der Formel I, worin die Gruppierung $R-Ar_1-$ für das Strukturelement der Formel Ib gemäss Anspruch 5 steht, in dem $R_a$ den Rest R und dieser Niederalkoxy mit bis und mit 4 C-Atomen bedeutet, $R_b$, $R_c$ und $R_e$ Wasserstoff darstellen und $R_d$ Cyano ist, alk Ethylen bedeutet, X für Carbonyl steht und $Ar_2$ 4-Fluorphenyl darstellt, und ihre Salze.

12. 5-Cyano-N-[2-[4-(p-fluorbenzoyl)-piperidinyl]-ethyl]-2-methoxy-benzamid
oder ein Salz davon.

13. 4-Chlor-5-cyano-N-[2-[4-(p-fluorbenzoyl)-piperidinyl]-ethyl]-2-methoxy-benzamid
oder ein Salz davon.

14. 5-Brom-4-chlor-N-[2-[4-(p-fluorbenzoyl)-piperidinyl]-ethyl]-2-methoxy-benzamid
oder ein Salz davon,
N-[2-[4-(p-Fluorbenzoyl)-piperidinyl]-ethyl]-4-fluor-2-methoxy-benzamid
oder ein Salz davon,
5-Brom-N-[2-[4-(p-fluorbenzoyl)-piperidinyl]-ethyl]-4-fluor-2-methoxy-benzamid
oder ein Salz davon,
N-[2-[4-(p-Fluorbenzoyl)-piperidinyl]-ethyl]-2-methoxy-5-trifluormethyl-benzamid
oder ein Salz davon,
2-Methoxy-5-sulfamoyl-N-[2-[4-(p-fluorbenzoyl)-piperidinyl]-ethyl]-benzamid
oder ein Salz davon,
5-Chlor-N-[2-[4-(p-fluorbenzoyl)-piperidinyl]-ethyl]-2-methoxy-4-methylaminosulfamoyl-benzamid
oder ein Salz davon,
N-[2-[4-(p-Fluorbenzoyl)-piperidinyl]-ethyl]-2-methoxy-5-dimethylsulfamoyl-benzamid
oder ein Salz davon,
3-Brom-N-[2-[4-(p-fluorbenzoyl)-piperidinyl]-ethyl]-4-methoxy-benzamid
oder ein Salz davon,
N-[2-[4-(p-Fluorbenzoyl)-piperidinyl]-ethyl]-2-methoxy-benzamid
oder ein Salz davon,
5-Cyano-N-[2-[4-(4-fluorphenyl)-hydroxymethylen]-piperidinyl]-ethyl]-2-methoxy-benzamid
oder ein Salz davon.

15. 3-Brom-4-fluor-N-[2-[4-(p-fluorbenzoyl)-piperidinyl]-ethyl]-benzamid
oder ein Salz davon,
2-Brom-4-fluor-N-[2-[4-(p-fluorbenzoyl)-piperidinyl]-ethyl]-benzamid
oder ein Salz davon,
N-[2-[4-(p-Fluorbenzoyl)-piperidinyl]-ethyl]-2,6-dimethoxy-benzamid
oder ein Salz davon,
4-Fluor-N-[2-[4-(p-fluorbenzoyl)-piperidinyl]-ethyl]-benzamid
oder ein Salz davon,
5-Brom-N-[2-[4-(p-fluorbenzoyl)-piperidinyl]-ethyl]-2-methoxy-benzamid
oder ein Salz davon,
3-Cyano-N-[2-[4-(p-fluorbenzoyl)-piperidinyl]-ethyl]-4-methoxy-benzamid

oder ein Salz davon.

16. 3,5-Dichlor-N-[2-[4-(p-fluorbenzoyl-piperidinyl]-ethyl]-2-methoxy-4-methyl-benzamid
oder ein Salz davon,
N-[2-[4-(p-Fluorbenzoyl)-piperidinyl]-ethyl]-2-methoxy-5-methylmercapto-benzamid
oder ein Salz davon,
N-[2-[4-(p-Fluorbenzoyl)-piperidinyl]-ethyl]-2-methoxy-5-methansulfonyl-benzamid
oder ein Salz davon,
5-Cyano-N-[2-[4-(p-fluorbenzyl)-piperidinyl]-ethyl]-2-methoxy-benzamid
oder ein Salz davon,
N-[2-[4-(p-Fluorbenzoyl)-piperidinyl]-ethyl]-2-methoxy-5-methoxycarbonyl-benzamid
oder ein Salz davon,
5-Carbamoyl-N-[2-[4-(p-fluorbenzoyl)-piperidinyl]-ethyl-2-methoxy-benzamid
oder ein Salz davon,
N-[2-[4-(p-Fluorbenzoyl)-piperidinyl]-ethyl]-2-methoxy-5-methansulfinyl-benzamid
oder ein Salz davon,
5-Cyano-2-methoxy-N-[2-[4-(2-thenoyl)-piperidinyl]-ethyl]-benzamid
oder ein Salz davon,
4-Fluor-N-[2-[4-(2-thenoyl)-piperidinyl]-ethyl]-benzamid
oder ein Salz davon,
N-[2-[4-(p-Chlorbenzoyl)-piperidinyl]-ethyl]-5-cyano-2-methoxy-benzamid
oder ein Salz davon,
N-[2-[4-(p-Chlorbenzoyl)-piperidinyl]-ethyl]-4-fluor-benzamid
oder ein Salz davon,
4-Fluor-N-[3-[4-(p-fluorbenzoyl)-piperidinyl]-2-methylpropyl]-benzamid
oder ein Salz davon,
5-Cyano-N-[3-[4-(p-fluorbenzoyl)-piperidinyl]-propyl]-2-methoxy-benzamid
oder ein Salz davon,
N-[2-[4-(p-Fluorbenzoyl)-piperidinyl]-ethyl]-2,6-dimethoxynicotinsäureamid
oder ein Salz davon.

17. Verbindungen zur Anwendung bei der prophylaktischen oder therapeutischen Behandlung des menschlichen oder tierischen Körpers der allgemeinen Formel I

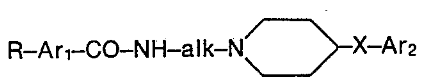 (I)

worin R, $Ar_1$, alk, X und $Ar_2$ die jeweils in einem der Ansprüche 1 bis 11 angegebenen Bedeutungen haben, unter Einschluss von Verbindungen der Formel I, worin die Gruppierung $R-Ar_1-$ für den Rest der Formel

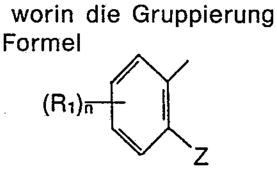 (Ia)

steht, in dem $R_1$ ausgewählt ist aus der Gruppe bestehend aus Niederalkyl, Trifluormethyl, Halogen, Niederalkoxy, Nitro und Cyano, mindestens einer der Reste $R_1$ Halogen oder Niederalkoxy bedeutet, n für eine ganze Zahl von 1 bis 3 steht und Z Nitro, Amino oder Niederalkylamino darstellt, alk und X die angegebenen Bedeutungen haben und $Ar_2$ unsubstituiertes Phenyl, Thienyl bzw. Pyridyl oder substituiertes Phenyl mit einem, zwei oder drei Substituenten ausgewählt aus der Gruppe bestehend aus Halogen, Niederalkyl, Niederalkoxy, Trifluormethyl und Amino bedeutet, wobei unter mit «Nieder» bezeichneten Resten solche zu verstehen sind, die bis und mit 7 C-Atome aufweisen, oder ein pharmazeutisch verwendbares Salz davon.

18. Verbindung gemäss einem der Ansprüche 12 bis 16 oder ein pharmazeutisch verwendbares Salz davon zur Anwendung bei der prophylaktischen oder therapeutischen Behandlung des menschlichen oder tierischen Körpers.

19. Verbindungen gemäss einem der Ansprüche 17 bis 18 der Formel I zur Anwendung als Antipsychotika.

20. Pharmazeutische Präparate, enthaltend eine Verbindung der Formel I gemäss einem der Ansprüche 17 bis 18.

21. Verwendung von Verbindungen der Formel I gemäss einem der Ansprüche 17 bis 18 zur Herstellung von Antipsychotika.

22. Verfahren zur Herstellung von N-(Piperidinyl-alkyl)-carboxamiden gemäss Anspruch 1 und ihrer Salze, dadurch gekennzeichnet, dass man

a) eine Verbindung der Formel IIa

$$R-Ar_1-X_1 \quad (IIa)$$

worin $X_1$ Carboxy oder reaktionsfähiges funktionell abgewandeltes Carboxy bedeutet, mit einer Verbindung der Formel IIb

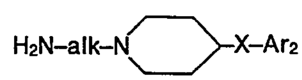 (IIb)

oder einem Salz davon kondensiert, oder

b) Verbindungen der Formeln IIIa und IIIb

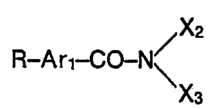 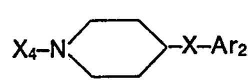

(IIIa) (IIIb)

worin $X_2$ für Wasserstoff steht, einer der Reste $X_3$ und $X_4$ Wasserstoff bedeutet und der andere Rest für eine Gruppe der Formel $-alk-X_5$ steht und $X_5$ reaktionsfähiges verestertes Hydroxy darstellt, oder Verbindungen der Formeln (IIIa) und (IIIb), worin $X_2$ und $X_3$ gemeinsam für alk' stehen, wobei alk' 2- bis 3-gliedriges Alkylen mit 2 bis und mit 7 C-Atomen darstellt, und $X_4$ Wasserstoff ist, oder deren Salze intermolekular kondensiert,

c) oder eine Verbindung der Formel IIIc

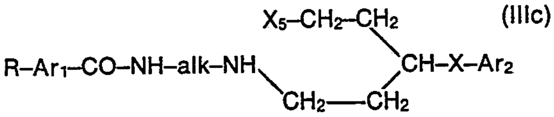

(IIIc)

worin $X_5$ reaktionsfähiges verestertes Hydroxy bedeutet, intramolekular kondensiert, oder

d) eine Verbindung der Formel IVa

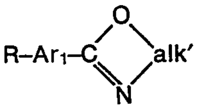

(IVa)

worin alk' eine das C-Atom und das N-Atom durch 2 bis 3 C-Atome trennende Alkylengruppe mit 2 bis und mit 7 C-Atomen darstellt, mit einer Verbindung der Formel IVb

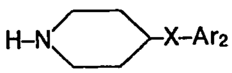

(IVb)

umsetzt, oder

e) in einer Verbindung der Formel V

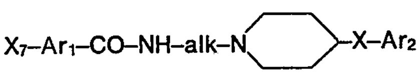

(V)

oder einem Salz davon, worin $X_7$ einen in R überführbaren Rest bedeutet, $X_7$ in R überführt,

und gewünschtenfalls eine verfahrensgemäss oder auf andere Weise erhältliche Verbindung in eine andere erfindungsgemässe Verbindung überführt, und/oder ein verfahrensgemäss erhältliches Salz in die freie Verbindung oder in ein anderes Salz überführt, und/oder eine verfahrensgemäss erhältliche freie Verbindung mit salzbildenden Eigenschaften in ein Salz überführt und/oder ein verfahrensgemäss erhältliches Isomerengemisch in die einzelnen Isomeren auftrennt.

23. Verfahren zur Herstellung pharmazeutischer Präparate, dadurch gekennzeichnet, dass man eine Verbindung gemäss einem der Ansprüche 17–19, gegebenenfalls unter Beimischung von üblichen Hilfs- und Trägerstoffen, zu pharmazeutischen Präparaten verarbeitet.

**Patentansprüche für den Vertragsstaat AT**

1. Verfahren zur Herstellung von N-(Piperidinyl-alkyl)-carboxamiden der allgemeinen Formel I

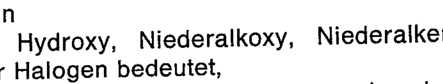

(I)

worin

R Hydroxy, Niederalkoxy, Niederalkenyloxy oder Halogen bedeutet,

$Ar_1$ für jeweils unsubstituiertes oder ein- oder mehrfach zusätzlich durch Niederalkyl, Niederalkenyl, Niederalkadienyl, Halogenniederalkyl, Hydroxy, Niederalkanoyloxy, Halogen, Niederalkoxy, Niederalkenyloxy, Halogenniederalkoxy, Niederalkanoyl, Nitro, Cyano, Carbamoyl, N-Niederalkyl-carbamoyl, N,N-Diniederalkyl-carbamoyl, Carboxy, Niederalkoxycarbonyl, Amino, N-Niederalkyl-amino, N,N-Diniederalkyl-amino, Sulfamoyl, N-Niederalkylsulfamoyl, N,N-Diniederalkyl-sulfamoyl, Niederalkylthio, Halogenniederalkylthio, Niederalkansulfinyl, Halogenniederalkansulfinyl, Niederalkansulfonyl, und/oder Halogenniederalkansulfonyl substituiertes Phenylen bzw. über ein C-Atom gebundenes, monocyclisches Azaarylen mit bis und mit 3 Stickstoffatomen steht,

alk die beiden N-Atome durch 2 bis 3 C-Atome trennendes Alkylen mit 2 bis und mit 7 C-Atomen darstellt,

X für Carbonyl, Diniederalkoxymethylen, Niederalkylendioxymethylen, Hydroxymethylen, Niederalkanoyloxymethylen oder Methylen steht, und

$Ar_2$ einen jeweils unsubstituierten oder ein- oder mehrfach durch Niederalkyl, Niederalkenyl, Niederalkadienyl, Halogenniederalkyl, Hydroxy, Niederalkanoyloxy, Halogen, Niederalkoxy, Niederalkenyloxy, Halogenniederalkoxy, Niederalkanoyl, Nitro, Cyano, Carbamoyl, N-Niederalkyl-carbamoyl, N,N-Diniederalkyl-carbamoyl, Carboxy, Niederalkoxycarbonyl, Amino, N-Niederalkyl-amino, N,N-Diniederalkyl-amino, Sulfamoyl, N-Niederalkylsulfamoyl, N,N-Diniederalkyl-sulfamoyl, Niederalkylthio, Halogenniederalkylthio, Niederalkansulfinyl, Halogenniederalkansulfinyl, Niederalkansulfonyl und/oder Halogenniederalkansulfonyl substituierten Phenylrest bzw. jeweils monocyclischen Monooxa-, Monoaza- oder Monothiaarylrest darstellt, und ihrer Salze, mit der Massgabe, dass die Gruppierung $R-Ar_1-$ von dem Rest der Formel Ia

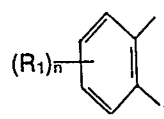

(Ia)

verschieden ist, worin $R_1$ ausgewählt ist aus der Gruppe bestehend aus Niederalkyl, Trifluormethyl, Halogen, Niederalkoxy, Nitro und Cyano und mindestens einer der Reste $R_1$ Halogen oder Niederalkoxy bedeutet, n für eine ganze Zahl von 1 bis 3 steht und Z Nitro, Amino oder Niederalkylamino darstellt, wenn alk und X die angegebenen Bedeutungen haben und $Ar_2$ unsubstituiertes Phenyl, Thienyl bzw. Pyridyl oder substituiertes Phenyl mit einem, zwei oder drei Substituenten ausgewählt aus der Gruppe bestehend aus Halogen, Niederalkyl, Niederalkoxy, Trifluormethyl und Amino bedeutet, wobei unter mit «Nieder» bezeichneten Resten solche zu verstehen sind, die bis und mit 7 C-Atome aufweisen, dadurch gekennzeichnet, dass man a) eine Verbindung der Formel IIa

$$R-Ar_1-X_1 \quad \text{(IIa)}$$

worin $X_1$ Carboxy oder reaktionsfähiges funktionell abgewandeltes Carboxy bedeutet, mit einer Verbindung der Formel IIb

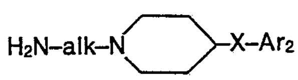
(IIb)

oder einem Salz davon kondensiert, oder

b) Verbindungen der Formeln IIIa und IIIb

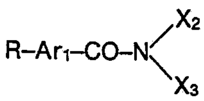
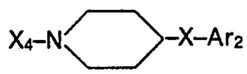

(IIIa) (IIIb)

worin $X_2$ für Wasserstoff steht, einer der Reste $X_3$ und $X_4$ Wasserstoff bedeutet und der andere Rest für eine Gruppe der Formel –alk–$X_5$ steht und $X_5$ reaktionsfähiges verestertes Hydroxy darstellt, oder Verbindungen der Formeln (IIIa) und (IIIb), worin $X_2$ und $X_3$ gemeinsam für alk' stehen, wobei alk' 2- bis 3-gliedriges Alkylen mit 2 bis und mit 7 C-Atomen darstellt, und $X_4$ Wasserstoff ist, oder deren Salze intermolekular kondensiert,

c) oder eine Verbindung der Formel IIIc

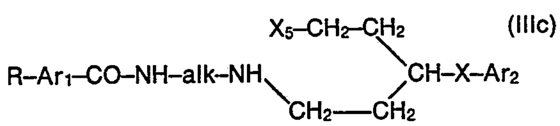
(IIIc)

worin $X_5$ reaktionsfähiges verestertes Hydroxy bedeutet, intramolekular kondensiert, oder

d) eine Verbindung der Formel IVa

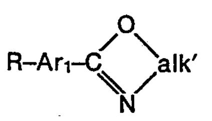
(IVa)

worin alk' eine das C-Atom und das N-Atom durch 2 bis 3 C-Atome trennende Alkylengruppe mit 2 bis und mit 7 C-Atomen darstellt, mit einer Verbindung der Formel IVb

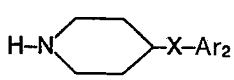
(IVb)

umsetzt, oder

e) in einer Verbindung der Formel V

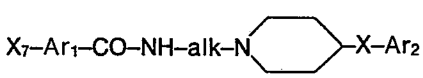
(V)

oder einem Salz davon, worin $X_7$ einen in R überführbaren Rest bedeutet, $X_7$ in R überführt,

und gewünschtenfalls eine verfahrensgemäss oder auf andere Weise erhältliche Verbindung in eine andere erfindungsgemässe Verbindung überführt, und/oder ein verfahrensgemäss erhältliches Salz in die freie Verbindung oder in ein anderes Salz überführt, und/oder eine verfahrensgemäss erhältliche freie Verbindung mit salzbildenden Eigenschaften in ein Salz überführt und/oder ein verfahrensgemäss erhältliches Isomerengemisch in die einzelnen Isomeren auftrennt.

2. Verfahren gemäss Anspruch 1, dadurch gekennzeichnet, dass man Verbindungen der Formel I, worin R Niederalkoxy,

$Ar_1$ für unsubstituiertes oder jeweils ein- oder mehrfach zusätzlich durch Niederalkyl, Halogenniederalkyl, Halogen, Niederalkoxy, Cyano, Carbamoyl, Amino, N-Niederalkylamino, N,N-Diniederalkylamino, Sulfamoyl, N,N-Diniederalkyl-sulfamoyl, Halogenniederalkylthio, Niederalkansulfonyl und/oder Halogenniederalkansulfonyl substituiertes Phenylen bzw. Pyridylen steht,

alk die beiden N-Atome durch 2 bis 3 C-Atome trennendes Alkylen mit 2 oder 3 C-Atomen darstellt, X für Carbonyl, Hydroxymethylen oder Methylen steht, und $Ar_2$ durch Halogen substituiertes Phenyl oder unsubstituiertes Thienyl darstellt, und ihre Salze herstellt.

3. Verfahren gemäss Anspruch 1, dadurch gekennzeichnet, dass man Verbindungen der Formel I, worin einerseits R Niederalkoxy oder Halogen bedeutet und $Ar_1$ für unsubstituiertes oder zusätzlich ein- oder mehrfach durch Niederalkyl, Halogenniederalkyl, Hydroxy, Halogen, Niederalkoxy, Cyano, Carbamoyl, Niederalkoxycarbonyl, Amino, Niederalkylamino, Diniederalkylamino, Sulfamoyl, N,N-Diniederalkyl-sulfamoyl, Niederalkylthio, Niederalkansulfinyl und/oder Niederalkansulfonyl substituiertes Phenylen steht,

oder worin andererseits R Niederalkoxy bedeutet, und $Ar_1$ für unsubstituiertes oder durch Niederalkoxy substituiertes Pyridylen steht, und jeweils alk die beiden N-Atome durch 2 bis 3 C-Atome trennendes Alkylen mit 2 bis und mit 7 C-Atomen darstellt, X für Carbonyl, Hydroxymethylen oder Methylen steht und $Ar_2$ durch Halogen substituiertes Phenyl oder unsubstituiertes Thienyl darstellt, und ihre Salze herstellt.

4. Verfahren gemäss Anspruch 1, dadurch gekennzeichnet, dass man Verbindungen der Formel I, worin

R jeweils Niederalkoxy bedeutet,

$Ar_1$ einerseits für einen unsubstituierten oder ein- oder mehrfach zusätzlich durch Halogenniederalkyl, Halogen, Niederalkoxy, Cyano, Carbamoyl, N-Niederalkylamino, Sulfamoyl, N,N-Diniederalkylsulfamoyl, Halogenniederalkylthio und/oder Halogenniederalkansulfonyl substituierten Phenylrest, andererseits für einen ein- oder mehrfach zusätzlich durch Niederalkoxy und/oder Halogen substituierten Pyridylenrest steht,

alk Ethylen oder 1,3-Propylen darstellt,

X für Carbonyl, Hydroxymethylen oder Methylen steht, und

$Ar_2$ einen durch Halogen, substituierten Phenylrest darstellt, und ihre Salze herstellt.

5. Verfahren gemäss Anspruch 1, dadurch gekennzeichnet, dass man Verbindungen der Formel I, worin die Gruppierung R–$Ar_1$ für das Strukturelement der Formel Ib

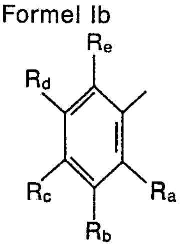
(Ib)

steht, in dem einer der Reste $R_a$ und $R_c$ den Rest R und dieser Niederalkoxy oder Halogen bedeutet und der andere Wasserstoff, Niederalkyl, Halogen oder Niederalkylamino darstellt, und die Reste $R_b$, $R_d$ und $R_e$ unabhängig voneinander für Wasserstoff, Niederalkyl, Halogenniederalkyl, Hydroxy, Halogen, Niederalkoxy, Cyano, Carbamoyl, Niederalkoxycarbonyl, Amino, Sulfamoyl, N,N-Diniederalkyl-sulfamoyl, Niederalkylthio, Niederalkansulfinyl und/oder Niederalkansulfonyl bedeuten, und worin alk für die beiden N-Atome durch 2 bis 3 C-Atome trennendes Alkylen mit 2 bis und mit 4 C-Atomen steht, X für Carbonyl, Hydroxymethylen oder Methylen steht und $Ar_2$ durch Halogen substituiertes Phenyl oder unsubstituiertes Thienyl darstellt, und ihre Salze herstellt.

6. Verfahren gemäss Anspruch 1, dadurch gekennzeichnet, dass man Verbindungen der Formel I, worin die Gruppierung R–$Ar_1$ für das Strukturelement der Formel Ib gemäss Anspruch 5 steht, in dem einerseits der Rest $R_a$ den Rest R und dieser Niederalkoxy mit bis und mit 4 C-Atomen bedeutet und $R_c$ Wasserstoff, Halogen mit Atomnummer bis und mit 35 oder Niederalkylamino mit bis und mit 4 C-Atomen darstellt, bzw. $R_a$ Wasserstoff ist und $R_c$ den Rest R und dieser Niederalkoxy mit bis und mit 4 C-Atomen bedeutet, oder in dem andererseits der Rest $R_c$ den Rest R und dieser Halogen mit Atomnummer bis und mit 35 bedeutet und $R_a$ Wasserstoff oder Halogen mit Atomnummer bis und mit 35 darstellt und einer der Reste $R_b$ und $R_d$ für Wasserstoff, Niederalkyl mit bis und mit 4 C-Atomen, Halogenniederalkyl mit Atomnummer bis und mit 35 und mit bis und mit 4 C-Atomen, Halogen mit Atomnummer bis und mit 35, Niederalkoxy mit bis und mit 4 C-Atomen, Cyano, Carbamoyl, Niederalkoxycarbonyl mit 2 bis und mit 5 C-Atomen, Niederalkylthio mit bis und mit 4 C-Atomen, Niederalkansulfinyl mit bis und mit 4 C-Atomen oder Niederalkansulfonyl mit bis und mit 4 C-Atomen steht, und der andere sowie $R_e$ Wasserstoff sind, und worin jeweils alk gemäss Anspruch 5 definiert ist, X für Carbonyl oder Hydroxymethylen steht und $Ar_2$ in p-Stellung durch Halogen mit Atomnummer bis und mit 35 substituiertes Phenyl darstellt, und ihre Salze herstellt.

7. Verfahren gemäss Anspruch 1, dadurch gekennzeichnet, dass man Verbindungen der Formel I, worin $Ar_1$ einerseits ein- oder mehrfach zusätzlich durch Halogen mit Atomnummer bis und mit 35, Cyano und/oder N-Niederalkylamino mit bis und mit 4 C-Atomen im Niederalkylteil substituiertes Phenylen bedeutet oder andererseits Pyridylen darstellt, und jeweils R Niederalkoxy bis und mit 4 C-Atomen bedeutet, alk Ethylen ist, X Carbonyl darstellt, und $Ar_2$ für durch Halogen mit Atomnummer bis und mit 35 substituiertes Phenyl steht, und ihre Salze herstellt.

8. Verfahren gemäss Anspruch 1, dadurch gekennzeichnet, dass man Verbindungen der Formel I, worin die Gruppierung R–$Ar_1$– für das Strukturelement der Formel Ib gemäss Anspruch 5 steht, in dem einerseits $R_a$ den Rest R und dieser Niederalkoxy mit bis und mit 4 C-Atomen bedeutet, $R_b$ und $R_e$ Wasserstoff sind, $R_c$ Niederalkylamino mit bis und mit 4 C-Atomen darstellt und $R_d$ Halogen mit Atomnummer bis und mit 35 bedeutet oder $R_c$ Wasserstoff oder Halogen mit Atomnummer bis und mit 35 darstellt und $R_d$ Cyano ist, oder in dem andererseits $R_a$, $R_d$ und $R_e$ Wasserstoff sind, $R_b$ Halogen mit Atomnummer bis und mit 35 und $R_c$ den Rest R und dieser Niederalkoxy mit bis und mit 4 C-Atomen darstellt, und jeweils alk Ethylen bedeutet, X für Carbonyl oder Hydroxymethylen steht, und $Ar_2$ 4-Fluorphenyl darstellt, und ihre Salze herstellt.

9. Verfahren gemäss Anspruch 1, dadurch gekennzeichnet, dass man Verbindungen der Formel I, worin die Gruppierung R–$Ar_1$– für das Strukturelement der Formel Ib gemäss Anspruch 5 steht, in dem einer der Reste $R_a$ und $R_b$ Wasserstoff oder Halogen mit Atomnummer bis und mit 35 darstellt, und der andere Wasserstoff bedeutet, $R_c$ den Rest R und dieser Halogen mit Atomnummer bis und mit 35 darstellt und $R_d$ und $R_e$ jeweils Wasserstoff sind, und alk Ethylen ist, X für Carbonyl steht und $Ar_2$ p-Fluorphenyl bedeutet, und ihre Salze herstellt.

10. Verfahren gemäss Anspruch 1, dadurch gekennzeichnet, dass man Verbindungen der Formel I, worin die Gruppierung R–$Ar_1$– für das Strukturelement der Formel Ib gemäss Anspruch 5 steht, in dem $R_a$, $R_d$ und $R_e$ Wasserstoff sind, $R_b$ Wasserstoff oder Halogen mit Atomnummer bis und mit 35 bedeutet, und $R_c$ den Rest R und dieser Halogen mit Atomnummer bis und mit 35 darstellt, alk Ethylen bedeutet, X für Carbonyl steht und $Ar_2$ 4-Fluorphenyl darstellt, und ihre Salze herstellt.

11. Verfahren gemäss Anspruch 1, dadurch gekennzeichnet, dass man Verbindungen der Formel I, worin die Gruppierung R–$Ar_1$– für das Strukturelement der Formel Ib gemäss Anspruch 5 steht, in dem $R_a$ den Rest R und dieser Niederalkoxy mit bis und mit 4 C-Atomen bedeutet, $R_b$, $R_c$ und $R_e$ Wasserstoff darstellen und $R_d$ Cyano ist, alk Ethylen bedeutet, X für Carbonyl steht und $Ar_2$ 4-Fluorphenyl darstellt, und ihre Salze herstellt.

12. Verfahren nach Anspruch 1, dadurch gekennzeichnet, dass man
5-Cyano-N-[2-[4-(p-fluorbenzoyl)-piperidinyl]-ethyl]-2-methoxy-benzamid
oder ein Salz davon herstellt.

13. Verfahren nach Anspruch 1, dadurch gekennzeichnet, dass man
4-Chlor-5-cyano-N-[2-[4-(p-fluorbenzoyl)-piperidinyl]-ethyl]-2-methoxy-benzamid
oder ein Salz davon herstellt.

14. Verfahren nach Anspruch 1, dadurch gekennzeichnet, dass man
5-Brom-4-chlor-N-[2-[4-(p-fluorbenzoyl]-piperidinyl]-ethyl]-2-methoxy-benzamid,
N-[2-[4-(p-Fluorbenzoyl)-piperidinyl]-ethyl]-4-fluor-2-methoxy-benzamid,
5-Brom-N-[2-[4-(p-fluorbenzoyl)-piperidinyl]-ethyl]-4-fluor-2-methoxy-benzamid,
N-[2-[4-(p-Fluorbenzoyl)-piperidinyl]-ethyl]-2-methoxy-5-trifluormethyl-benzamid,

2-Methoxy-5-sulfamoyl-N-[2-[4-(p-fluorbenzoyl)-piperidinyl]-ethyl]-benzamid,
5-Chlor-N-[2-[4-(p-fluorbenzoyl)-piperidinyl]-ethyl]-2-methoxy-4-methylaminosulfamoyl-benzamid,
N-[2-[4-(p-Fluorbenzoyl)-piperidinyl]-ethyl]-2-methoxy-5-dimethylsulfamoyl-benzamid,
3-Brom-N-[2-[4-(p-fluorbenzoyl)-piperidinyl]-ethyl]-4-methoxy-benzamid,
N-[2-[4-(p-Fluorbenzoyl)-piperidinyl]-ethyl]-2-methoxy-benzamid,
5-Cyano-N-[2-[4-(4-fluorphenyl)-hydroxymethylen]-piperidinyl]-ethyl]-2-methoxy-benzamid,
oder ein Salz davon herstellt.

15. Verfahren nach Anspruch 1, dadurch gekennzeichnet, dass man
3-Brom-4-fluor-[2-[4-(p-fluorbenzoyl)-piperidinyl]-ethyl]-benzamid,
2-Brom-4-fluor-N-[2-[4-(p-fluorbenzoyl)-piperidinyl]-ethyl]-benzamid,
N-[2-[4-(p-Fluorbenzoyl)-piperidinyl]-ethyl]-2,6-dimethoxy-benzamid,
4-Fluor-N-[2-[4-(p-fluorbenzoyl)-piperidinyl]-ethyl]-benzamid,
5-Brom-N-[2-[4-(p-fluorbenzoyl)-piperidinyl]-ethyl]-2-methoxy-benzamid,
3-Cyano-N-[2-[4-(p-fluorbenzoyl)-piperidinyl]-ethyl]-4-methoxy-benzamid
oder ein Salz davon herstellt.

16. Verfahren nach Anspruch 1, dadurch gekennzeichnet, dass man
3,5-Dichlor-N-[2-[4-(p-fluorbenzoyl)-piperidinyl]-ethyl]-2-methoxy-4-methyl-benzamid,
N-[2-[4-(p-Fluorbenzoyl)-piperidinyl]-ethyl]-2-methoxy-5-methylmercapto-benzamid,
N-[2-[4-(p-Fluorbenzoyl)-piperidinyl]-ethyl]-2-methoxy-5-methansulfonyl-benzamid,
5-Cyano-N-[2-[4-(p-fluorbenzyl)-piperidinyl]-ethyl]-2-methoxy-benzamid,
N-[2-[4-(p-Fluorbenzoyl)-piperidinyl]-ethyl]-2-methoxy-5-methoxycarbonyl-benzamid,
5-Carbamoyl-N-[2-[4-(p-fluorbenzoyl)-piperidinyl]-ethyl-2-methoxy-benzamid,
N-[2-[4-(p-Fluorbenzoyl)-piperidinyl]-ethyl]-2-methoxy-5-methansulfinyl-benzamid,
5-Cyano-2-methoxy-N-[2-[4-(2-thenoyl)-piperidinyl]-ethyl]-benzamid,
4-Fluor-N-[2-[4-(2-thenoyl)-piperidinyl]-ethyl]-benzamid,
N-[2-[4-(p-Chlorbenzoyl)-piperidinyl]-ethyl]-5-cyano-2-methoxy-benzamid,
N-[2-[4-(p-Chlorbenzoyl)-piperidinyl]-ethyl]-4-fluor-benzamid,
4-Fluor-N-[3-[4-(p-fluorbenzoyl)-piperidinyl]-2-methylpropyl]-benzamid,
5-Cyano-N-[3-[4-(p-fluorbenzoyl)-piperidinyl]-propyl]-2-methoxy-benzamid,
N-[2-[4-(p-Fluorbenzoyl)-piperidinyl]-ethyl]-2,6-dimethoxynicotinsäureamid
oder ein Salz davon herstellt.

17. Verfahren zur Herstellung pharmazeutischer Präparate, dadurch gekennzeichnet, dass man eine Verbindung gemäss einem der Ansprüche 1–16, gegebenenfalls unter Beimischung von üblichen Hilfs- und Trägerstoffen, zu pharmazeutischen Präparaten verarbeitet.

**Claims for the Contracting States: BE, CH, DE, FR, GB, IT, LI, LU, NL, SE**

1. N-(piperidinyl-alkyl)-carboxamides of the general formula I

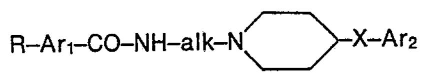

(I)

in which R represents hydroxy, lower alkoxy, lower alkenyloxy or halogen,

$Ar_1$ represents a phenylene radical or a monocyclic azaarylene radical having up to and including 3 nitrogen atoms and bonded by a carbon atom, each of which radicals is unsubstituted or mono- or polysubstituted by lower alkyl, lower alkenyl, lower alkadienyl, halo-lower alkyl, hydroxy, lower alkanoyloxy, halogen, lower alkoxy, lower alkenyloxy, halo-lower alkoxy, lower alkanoyl, nitro, cyano, carbamoyl, N-lower alkylcarbamoyl, N,N-di-lower alkylcarbamoyl, carboxy, lower alkoxycarbonyl, amino, N-lower alkylamino, N,N-di-lower alkylamino, sulphamoyl, N-lower alkylsulphamoyl, N,N-di-lower alkylsulphamoyl, lower alkylthio, halo-lower alkylthio, lower alkanesulphinyl, halo-lower alkanesulphinyl, lower alkanesulphonyl and/or by halo-lower alkanesulphonyl,

alk represents alkylene that has from 2 up to and including 7 carbon atoms and separates the two N atoms by 2 or 3 carbon atoms,

X represents carbonyl, di-lower alkoxymethylene, lower alkylenedioxymethylene, hydroxymethylene, lower alkanoyloxymethylene or methylene, and

$Ar_2$ represents a phenyl radical or a monocyclic monooxa-, monoaza- or monothia-aryl radical, each of which radicals is unsubstituted or mono- or poly-substituted by lower alkyl, lower alkenyl, lower alkadienyl, halo-lower alkyl, hydroxy, lower alkanoyloxy, halogen, lower alkoxy, lower alkenyloxy, halo-lower alkoxy, lower alkanoyl, nitro, cyano, carbamoyl, N-lower alkylcarbamoyl, N,N-di-lower alkycarbamoyl, carboxy, lower alkoxycarbonyl, amino, N-lower alkylamino, N,N-di-lower alkylamino, sulphamoyl, N-lower alkylsulphamoyl, N,N-di-lower alkylsulphamoyl, lower alkylthio, halo-lower alkylthio, lower alkanesulphinyl, halo-lower alkanesulphinyl, lower alkanesulphonyl and/or by halo-lower alkanesuphonyl, and salts thereof, with the proviso that the grouping $R–Ar_1–$ is other than a radical of the formula Ia

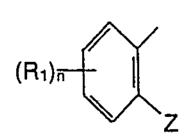

(Ia),

in which $R_1$ is selected from the group consisting of lower alkyl, trifluoromethyl, halogen, lower alkoxy, nitro and cyano and lat least one of the radi-

cals $R_1$ represents halogen or lower alkoxy, n represents an integer from 1 to 3, and Z represents nitro, amino or lower alkylamino, if alk and X have the meanings given and $Ar_2$ represents unsubstituted phenyl, thienyl or pyridyl or substituted phenyl having one, two or three substituents selected from the group consisting of halogen, lower alkyl, lower alkoxy, trifluoromethyl and amino,
radicals designated "lower" being understood as those having up to and including 7 carbon atoms.

2. Compounds according to claim 1 of the formula I in which R represents lower alkoxy,

$Ar_1$ represents phenylene or pyridylene each of which is unsubstituted or mono- or poly-substituted by lower alkyl, halo-lower alkyl, halogen, lower alkoxy, cyano, carbamoyl, amino, N-lower alkylamino, N,N-di-lower alkylamino, sulphamoyl, N,N-di-lower alkylsulphamoyl, halo-lower alkylthio, lower alkanesulphonyl and/or by halo-lower alkanesulphonyl,

alk represents alkylene that has 2 or 3 carbon atoms and separates the two N atoms by 2 or 3 carbon atoms, X represents carbonyl, hydroxymethylene or methylene and $Ar_2$ represents phenyl substituted by halogen, or unsubstituted thienyl, and salts thereof.

3. Compounds according to claim 1 of the formula I in which, on the one hand, R represents lower alkoxy or halogen and $Ar_1$ represents phenylene which is unsubstituted or mono- or poly-substituted by lower alkyl, halo-lower alkyl, hydroxy, halogen, lower alkoxy, cyano, carbamoyl, lower alkoxycarbonyl, amino, lower alkylamino, di-lower alkylamino, sulphamoyl, N,N-di-lower alkylsulphamoyl, lower alkylthio, lower alkanesulphinyl and/or by lower alkanesulphonyl, or in which, on the other hand, R represents lower alkoxy and $Ar_1$ represents pyridylene which is unsubstituted or substituted by lower alkoxy, and in each case alk represents alkylene that has from 2 up to and including 7 carbon atoms and separates the two N atoms by 2 or 3 carbon atoms, X represents carbonyl, hydroxymethylene or methylene and $Ar_2$ represents phenyl substituted by halogen, or unsubstituted thienyl, and salts thereof.

4. Compounds according to claim 1 of the formula I in which

R in each case represents lower alkoxy,

$Ar_1$ represents, on the one hand, a phenyl radical which is unsubstituted or mono- or poly-substituted by halo-lower alkyl, halogen, lower alkoxy, cyano, carbamoyl, N-lower alkylamino, sulphamoyl, N,N-di-lower alkylsulphamoyl, halo-lower alkylthio and/or by halo-lower alkanesulphonyl, or, on the other hand, a pyridylene radical which is mono- or poly-substituted by lower alkoxy and/or by halogen,

alk represents ethylene or 1,3-propylene,

X represents carbonyl, hydroxymethylene or methylene and

$Ar_2$ represents a phenyl radical substituted by halogen, and salts thereof.

5. Compounds according to claim 1 of the formula I in which the grouping R–$Ar_1$ represents the structural element of the formula Ib

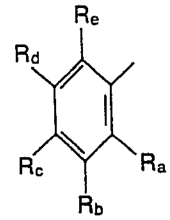

(Ib)

in which one of the radicals $R_a$ and $R_c$ represents the radical R which represents lower alkoxy or halogen, and the other represents hydrogen, lower alkyl, halogen or lower alkylamino, and each of the radicals $R_b$, $R_d$ and $R_e$, independently of the others, represents hydrogen, lower alkyl, halo-lower alkyl, hydroxy, halogen, lower alkoxy, cyano, carbamoyl, lower alkoxycarbonyl, amino, sulphamoyl, N,N-di-lower alkylsulphamoyl, lower alkylthio, lower alkanesulphinyl and/or lower alkanesulphonyl,
and in which alk represents alkylene that has from 2 up to and including 4 carbon atoms and separates the two N atoms by 2 or 3 carbon atoms, X represents carbonyl, hydroxymethylene or methylene and $Ar_2$ represents phenyl substituted by halogen, or unsubstituted thienyl, and salts thereof.

6. Compounds according to claim 1 of the formula I in which the grouping R–$Ar_1$ represents the structural element of the formula Ib according to claim 5 in which, on the one hand, the radical $R_a$ represents the radical R which represents lower alkoxy having up to and including 4 carbon atoms and $R_c$ represents hydrogen, halogen having an atomic number of up to and including 35 or lower alkylamino having up to and including 4 carbon atoms, or $R_a$ represents hydrogen and $R_c$ the radical R which represents lower alkoxy having up to and including 4 carbon atoms, or in which, on the other hand, the radical $R_c$ represents the radical R which represents halogen having an atomic number of up to and including 35 and $R_a$ represents hydrogen or halogen having an atomic number of up to and including 35 and one of the radicals $R_b$ and $R_d$ represents hydrogen, lower alkyl having up to and including 4 carbon atoms, halo-lower alkyl having an atomic number of up to and including 35 and having up to and including 4 carbon atoms, halogen having an atomic number of up to and including 35, lower alkoxy having up to and including 4 carbon atoms, cyano, carbamoyl, lower alkoxycarbonyl having from 2 up to and including 5 carbon atoms, lower alkylthio having up to and including 4 carbon atoms, lower alkanesulphinyl having up to and including 4 carbon atoms or lower alkanesulphonyl having up to and including 4 carbon atoms, and the other and $R_e$ represent hydrogen, and in which in each case alk has the definition given in claim 5, X represents carbonyl or hydroxymethylene and $Ar_2$ represents phenyl substituted in the p-position by halogen having an

atomic number of up to and including 35, and salts thereof.

7. Compounds according to claim 1 of the formula I in which $Ar_1$ represents, on the one hand, phenylene which is mono- or poly-substituted by halogen having an atomic number of up to and including 35, cyano and/or by N-lower alkylamino having up to and including 4 carbon atoms in the lower alkyl moiety, or, on the other hand, pyridylene, and in each case R represents lower alkoxy having up to and including 4 carbon atoms, alk represents ethylene, X represents carbonyl and $Ar_2$ represents phenyl substituted by halogen having an atomic number of up to and including 35, and salts thereof.

8. Compounds according to claim 1 of the formula I in which the grouping $R-Ar_1-$ represents the structural element of the formula Ib according to claim 5 in which, on the one hand, $R_a$ represents the radical R which represents lower alkoxy having up to and including 4 carbon atoms, $R_b$ and $R_e$ represent hydrogen, $R_c$ represents lower alkylamino having up to and including 4 carbon atoms and $R_d$ represents halogen having an atomic number of up to and including 35, or $R_c$ represents hydrogen or halogen having an atomic number of up to and including 35 and $R_d$ represents cyano, or in which, on the other hand, $R_a$, $R_d$ and $R_e$ represent hydrogen, $R_b$ represents halogen having an atomic number of up to and including 35 and $R_c$ represents the radical R which represents lower alkoxy having up to and including 4 carbon atoms, and in each case alk represents ethylene, X represents carbonyl or hydroxymethylene and $Ar_2$ represents 4-fluorophenyl, and salts thereof.

9. Compounds according to claim 1 of the formula I in which the grouping $R-Ar_1-$ represents the structural element of the formula Ib according to claim 5 in which one of the radicals $R_a$ and $R_b$ represents hydrogen or halogen having an atomic number of up to and including 35, and the other represents hydrogen, $R_c$ represents the radical R which represents halogen having an atomic number of up to and including 35 and each of $R_d$ and $R_e$ represents hydrogen, and alk represents ethylene, X represents carbonyl and $Ar_2$ represents p-fluorophenyl, and salts thereof.

10. Compounds according to claim 1 of the formula I in which the grouping $R-Ar_1-$ represents the structural element of the formula Ib according to claim 5 in which $R_a$, $R_d$ and $R_e$ represent hydrogen, $R_b$ represents hydrogen or halogen having an atomic number of up to and including 35, and $R_c$ represents the radical R which represents halogen having an atomic number of up to and including 35, alk represents ethylene, X represents carbonyl and $Ar_2$ represents 4-fluorophenyl, and salts thereof.

11. Compounds according to claim 1 of the formula I in which the grouping $R-Ar_1-$ represents the structural element of the formula Ib according to claim 5 in which $R_a$ represents the radical R which represents lower alkoxy having up to and including 4 carbon atoms, $R_b$, $R_c$ and $R_e$ represent hydrogen and $R_d$ represents cyano, alk represents ethylene, X represents carbonyl and $Ar_2$ represents 4-fluorophenyl, and salts thereof.

12. 5-cyano-N-[-2-[4-(p-fluorobenzoyl)-piperidinyl]-ethyl]-2-methoxy-benzamide or a salt thereof.

13. 4-chloro-5-cyano-N-[2-[4-(p-fluorobenzoyl)-piperidinyl]-ethyl]-2-methoxy-benzamide or a salt thereof.

14. 5-bromo-4-chloro-N-[2-[4-(p-fluorobenzoyl)-piperidinyl]-ethyl]-2-methoxy-benzamide
or a salt thereof,
N-[2-[4-(p-fluorobenzoyl)-piperidinyl]-ethyl]-4-fluoro-2-methoxy-benzamide
or a salt thereof,
5-bromo-N-[2-[4-(p-fluorobenzoyl)-piperidinyl]-ethyl]-4-fluoro-2-methoxy-benzamide
or a salt thereof,
N-[2-[4-(p-fluorobenzoyl)-piperidinyl]-ethyl]-2-methoxy-5-trifluoromethyl-benzamide
or a salt thereof,
2-methoxy-5-sulphamoyl-N-[2-[4-(p-fluorobenzoyl)-piperidinyl]-ethyl]-benzamide
or a salt thereof,
5-chloro-N-[2-[4-(p-fluorobenzoyl)-piperidinyl]-ethyl]-2-methoxy-4-methylamino-sulphamoyl-benzamide
or a salt thereof,
N-[2-[4-(p-fluorobenzoyl)-piperidinyl]-ethyl]-2-methoxy-5-dimethylsulphamoyl-benzamide
or a salt thereof,
3-bromo-N-[2-[4-(p-fluorobenzoyl)-piperidinyl]-ethyl]-4-methoxy-benzamide
or a salt thereof,
N-[2-[4-(p-fluorobenzoyl)-piperidinyl]-ethyl]-2-methoxy-benzamide
or a salt thereof,
5-cyano-N-[2-[4-[(4-fluorophenyl)-hydroxymethylene]-piperidinyl]-ethyl]-2-methoxy-benzamide
or a salt thereof.

15. 3-bromo-4-fluoro-N-[2-[4-(p-fluorobenzoyl)-piperidinyl]-ethyl]-benzamide
or a salt thereof,
2-bromo-4-fluoro-N-[2-[4-(p-fluorobenzoyl)-piperidinyl]-ethyl]-benzamide
or a salt thereof,
N-[2-[4-(p-fluorobenzoyl)-piperidinyl]-ethyl]-2,6-dimethoxy-benzamide
or a salt thereof,
4-fluoro-N-[2-[4-(p-fluorobenzoyl)-piperidinyl]-ethyl]-benzamide
or a salt thereof,
5-bromo-N-[2-[4-(p-fluorobenzoyl)-piperidinyl]-ethyl]-2-methoxy-benzamide
or a salt thereof,
3-cyano-N-[2-[4-(p-fluorobenzoyl)-piperidinyl]-ethyl]-4-methoxy-benzamide
or a salt thereof.

16. 3,5-dichloro-N-[2-[4-(p-fluorobenzoyl)-piperidinyl]-ethyl]-2-methoxy-4-methyl-benzamide
or a salt thereof,
N-[2-[4-(p-fluorobenzoyl)-piperidinyl]-ethyl]-2-methoxy-5-methylmercapto-benzamide
or a salt thereof,
N-[2-[4-(p-fluorobenzoyl)-piperidinyl]-ethyl]-2-methoxy-5-methanesulphonyl-benzamide
or a salt thereof,
5-cyano-N-[2-[4-(p-fluorobenzyl)-piperidinyl]-ethyl]-2-methoxy-benzamide
or a salt thereof,
N-[2-[4-(p-fluorobenzoyl)-piperidinyl]-ethyl]-2-methoxy-5-methoxycarbonyl-benzamide
or a salt thereof,
5-carbamoyl-N-[2-[4-(p-fluorobenzoyl)-piperidinyl]-ethyl]-2-methoxy-benzamide
or a salt thereof,
N-[2-[4-(p-fluorobenzoyl)-piperidinyl]-ethyl]-2-methoxy-5-methanesulphinyl-benzamide
or a salt thereof,
5-cyano-2-methoxy-N-[2-[4-(2-thenoyl)-piperidinyl]-ethyl]-benzamide
or a salt thereof,
4-fluoro-N-[2-[4-(2-thenoyl)-piperidinyl]-ethyl]-benzamide
or a salt thereof
N-[2-[4-(p-chlorobenzoyl)-piperidinyl]-ethyl]-5-cyano-2-methoxy-benzamide
or a salt thereof,
N-[2-[4-(p-chlorobenzoyl)-piperidinyl]-ethyl]-4-fluoro-benzamide
or a salt thereof,
4-fluoro-N-[3-[4-(p-fluorobenzoyl)-piperidinyl]-2-methylpropyl]-benzamide
or a salt thereof,
5-cyano-N-[3-[4-(p-fluorobenzoyl)-piperidinyl]-propyl]-2-methoxy-benzamide
or a salt thereof,
N-[2-[4-(p-fluorobenzoyl)-piperidinyl]-ethyl]-2,6-dimethoxynicotinic acid amide
or a salt thereof.

17. Compounds for use in the prophylactic or therapeutic treatment of the human or animal body of the general formula I

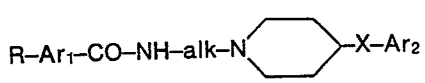 (I)

in which R, $Ar_1$ alk, X and $Ar_2$ each have the meanings given in any one of claims 1 to 11, with the inclusion of compounds of the formula I in which the grouping $R-Ar_1-$ represents the radical of the formula

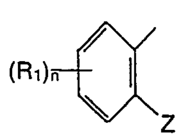 (Ia)

in which $R_1$ is selected from the group consisting of lower alkyl, trifluoromethyl, halogen, lower alkoxy, nitro and cyano and at least one of the radicals $R_1$ represents halogen or lower alkoxy, n represents an integer from 1 to 3, and Z represents nitro, amino or lower alkylamino, alk and X have the meanings given and $Ar_2$ represents unsubstituted phenyl, thienyl or pyridyl or substituted phenyl having one, two or three substituents selected from the group consisting of halogen, lower alkyl, lower alkoxy, trifluoromethyl and amino,
radicals designated "lower" being understood as those having up to and including 7 carbon atoms, or a pharmaceutically acceptable salt thereof.

18. A compound according to any one of claims 12 to 16 or a pharmaceutically acceptable salt thereof for use in the prophylactic or therapeutic treatment of the human or animal body.

19. Compounds according to any one of claims 17 and 18 of the formula I for use as antipsychotic agents.

20. Pharmaceutical preparations containing a compound of the formula I according to any one of claims 17 and 18.

21. Use of compounds of the formula I according to any one of claims 17 and 18 for the manufacture of antipsychotic agents.

22. Process for the manufacture of N-(piperidinyl-alkyl)-carboxamides according to claim 1 and salts thereof, characterised in that

a) a compound of the formula IIa

$$R-Ar_1-X_1 \qquad (IIa),$$

in which $X_1$ represents carboxy or reactive functionally modified carboxy, is condensed with a compound of the formula IIb

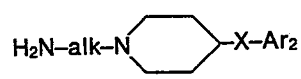 (IIb)

or with a salt thereof, or

b) compounds of the formulae IIIa and IIIb

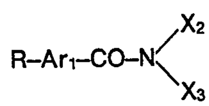 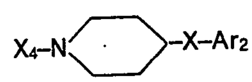

(IIIa) (IIIb)

in which $X_2$ represents hydrogen, one of the radicals $X_3$ and $X_4$ represents hydrogen and the other radical represents a group of the formula $-alk-X_5$ and $X_5$ represents reactive esterified hydroxy,
or compounds of the formulae (IIIa) and (IIIb) in which $X_2$ and $X_3$ together represent alk′ wherein alk′ represents 2- or 3-membered alkylene having from 2 up to and including 7 carbon atoms, and $X_4$ represents hydrogen, or the salts thereof, are intramolecularly condensed, or

c) a compound of the formula IIIc

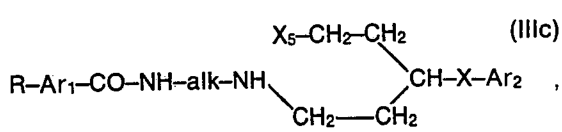 (IIIc) ,

in which $X_5$ represents reactive esterified hydroxy, is intramolecularly condensed, or

d) a compound of the formula IVa

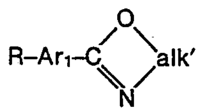 (IVa),

in which alk′ represents an alkylene group that has from 2 up to and including 7 carbon atoms and separates the C atom and the N atom by 2 or 3 carbon atoms, is reacted with a compound of the formula IVb

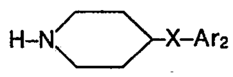 (IVb)

or

e) in a compound of the formula V

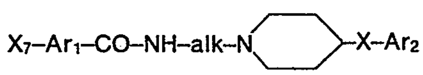 (V),

or in a salt thereof, in which $X_7$ represents a radical that can be converted into R, $X_7$ is converted into R,

and, if desired, a compound obtainable in accordance with the process or by another method is converted into a different compound according to the invention, and/or a salt obtainable in accordance with the process is converted into the free compound or into a different salt, and/or a free compound having salt-forming properties which is obtainable in accordance with the process is converted into a salt, and/or a mixture of isomers obtainable in accordance with the process is separated into the individual isomers.

23. Process for the manufacture of pharmaceutical preparations, characterised in that a compound according to any one of claims 17 to 19 is processed, optionally with the admixture of customary adjuncts and carriers, to form pharmaceutical preparations.

**Claims for the contracting state: AT**

1. Process for the manufacture of N-(piperidinyl-alkyl)-carboxamides of the general formula I

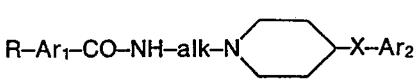 (I)

in which

R represents hydroxy, lower alkoxy, lower alkenyloxy or halogen,

$Ar_1$ represents a phenylene radical or a monocyclic azaarylene radical having up to and including 3 nitrogen atoms and bonded by a carbon atom, each of which radicals is unsubstituted or mono- or poly-substituted by lower alkyl, lower alkenyl, lower alkadienyl halo-lower alkyl, hydroxy, lower alkanoyloxy, halogen, lower alkoxy, lower alkenyloxy, halo-lower alkoxy, lower alkanoyl, nitro, cyano, carbamoyl, N-lower alkylcarbamoyl, N,N-di-lower alkylcarbamoyl, carboxy, lower alkoxycarbonyl, amino, N-lower alkylamino, N,N-di-lower alkylamino, sulphamoyl, N-lower alkylsulphamoyl, N,N-di-lower alkylsulphamoyl, lower alkylthio, halo-lower alkylthio, lower alkanesulphinyl, halo-lower alkanesulphinyl, lower alkanesulphonyl and/or by halo-lower alkanesulphonyl,

alk represents alkylene that has from 2 up to and including 7 carbon atoms and separates the two N atoms by 2 or 3 carbon atoms,

X represents carbonyl, di-lower alkoxymethylene, lower alkylenedioxymethylene, hydroxymethylene, lower alkanoyloxymethylene or methylene, and

$Ar_2$ represents a phenyl radical or a monocyclic monooxa-, monoaza- or monothia-aryl radical, each of which radicals is unsubstituted or mono- or poly-substituted by lower alkyl, lower alkenyl, lower alkadienyl, halo-lower alkyl, hydroxy, lower alkanoyloxy, halogen, lower alkoxy, lower alkenyloxy, halo-lower alkoxy, lower alkanoyl, nitro, cyano, carbamoyl, N-lower alkylcarbamoyl, N,N-di-lower alkylcarbamoyl, carboxy, lower alkoxycarbonyl, amino, N-lower alkylamino, N,N-di-lower alkylamino, sulphamoyl, N-lower alkylsulphamoyl, N,N-di-lower alkylsulphamoyl, lower alkylthio, halo-lower alkylthio, lower alkanesulphinyl, halo-lower alkanesulphinyl, lower alkanesulphonyl and/or by halo-lower alkanesulphonyl, and salts thereof, with the proviso that the grouping $R-Ar_1-$ is other than a radical of the formula Ia

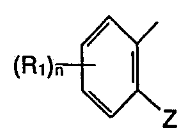 (Ia),

in which $R_1$ is selected from the group consisting of lower alkyl, trifluoromethyl, halogen, lower alkoxy, nitro and cyano and at least one of the radicals $R_1$ represents halogen or lower alkoxy, n represents an integer from 1 to 3, and Z represents nitro, amino or lower alkylamino, if alk and X have the meanings given and $Ar_2$ represents unsubstituted phenyl, thienyl or pyridyl or substituted phenyl having one, two or three substituents selected from the group consisting of halogen, lower alkyl, lower alkoxy, trifluoromethyl and amino, radicals designated "lower" being understood as those having up to and including 7 carbon atoms, characterised in that

a) a compound of the formula IIa

$$R-Ar_1-X_1 \qquad (IIa),$$

in which $X_1$ represents carboxy or reactive functionally modified carboxy, is condensed with a compound of the formula IIb

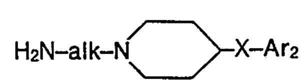 (IIb)

or with a salt thereof, or

b) compounds of the formulae IIIa and IIIb

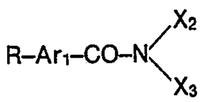 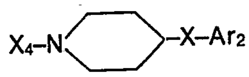

(IIIa) (IIIb)

in which $X_2$ represents hydrogen, one of the radicals $X_3$ and $X_4$ represents hydrogen and the other radical represents a group of the formula $-alk-X_5$ and $X_5$ represents reactive esterified hydroxy, or compounds of the formulae (IIIa) and (IIIb) in which $X_2$ and $X_3$ together represent alk′ wherein alk′ represents 2- or 3-membered alkylene having from 2 up to and including 7 carbon atoms, and $X_4$ represents hydrogen, or the salts thereof, are intramolecularly condensed, or

c) a compound of the formula IIIc

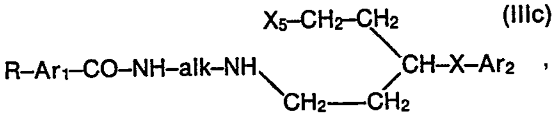 (IIIc) ,

in which $X_5$ represents reactive esterified hydroxy, is intramolecularly condensed, or

d) a compound of the formula IVa

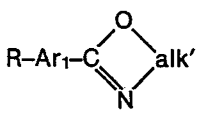 (IVa) ,

in which alk′ represents an alkylene group that has from 2 up to and including 7 carbon atoms and separates the C atom and the N atom by 2 or 3 carbon atoms, is reacted with a compound of the formula IVb

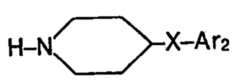 (IVb)

or

e) in a compound of the formula V

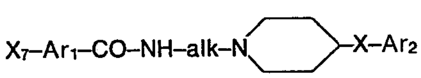 (V) ,

or in a salt thereof, in which $X_7$ represents a radical that can be converted into R, $X_7$ is converted into R,

and, if desired, a compound obtainable in accordance with the process or by another method is converted into a different compound according to the invention, and/or a salt obtainable in accordance with the process is converted into the free compound or into a different salt, and/or a free compound having salt-forming properties which is obtainable in accordance with the process is converted into a salt, and/or a mixture of isomers obtainable in accordance with the process is separated into the individual isomers.

2. Process according to claim 1, characterised in that compounds of the formula I in which R represents lower alkoxy,

$Ar_1$ represents phenylene or pyridylene each of which is unsubstituted or mono- or polysubstituted by lower alkyl, halo-lower alkyl, halogen, lower lakoxy, cyano, carbamoyl, amino, N-lower alkylamino, N,N-di-lower alkylamino, sulphamoyl, N,N-di-lower alkylsulphamoyl, halo-lower alkylthio, lower alkanesulphonyl and/or by halo-lower alkanesulphonyl,

alk represents alkylene that has 2 or 3 carbon atoms and separates the two N atoms by 2 or 3 carbon atoms, X represents carbonyl, hydroxymethylene or methylene and

$Ar_2$ represents phenyl substituted by halogen, or unsubstituted thienyl, and salts thereof, are manufactured.

3. Process according to claim 1, characterised in that compounds of the formula I in which, on the one hand, R represents lower alkoxy or halogen and $Ar_1$ represents phenylene which is unsubstituted or mono- or poly-substituted by lower alkyl, halo-lower alkyl, hydroxy, halogen, lower alkoxy, cyano, carbamoyl, lower alkoxycarbonyl, amino, lower alkylamino, di-lower alkylamino, sulphamoyl, N,N-di-lower alkylsulphamoyl, lower alkylthio, lower alkanesulphinyl and/or by lower alkanesulphonyl, or in which, on the other hand, R represents lower alkoxy and $Ar_1$ represents pyridylene which is unsubstituted or substituted by lower alkoxy, and in each case alk represents alkylene that has from 2 up to and including 7 carbon atoms and separates the two N-atoms by 2 or 3 carbon atoms, X represents carbonyl, hydroxymethylene or methylene and $Ar_2$ represents phenyl substituted by halogen, or unsubstituted thienyl, and salts thereof, are manufactured.

4. Process according to claim 1, characterised in that compounds of the formula I in which

R in each case represents lower alkoxy,

$Ar_1$ represents, on the one hand, a phenyl radical which is unsubstituted or mono- or poly-substituted by halo-lower alkyl, halogen, lower alkoxy, cyano, carbamoyl, N-lower alkylamino, sulphamoyl, N,N-di-lower alkylsulphamoyl, halo-lower alkylthio and/or by halo-lower alkanesulphonyl, or, on the other hand, a pyridylene radical which is mono- or poly-substituted by lower alkoxy and/or halogen,

alk represents ethylene or 1,3-propylene,

X represents carbonyl, hydroxymethylene or methylene, and

$Ar_2$ represents a phenyl radical substituted by halogen, and salts thereof, are manufactured.

5. Process according to claim 1, characterised in that compounds of the formula I in which the grouping $R-Ar_1$ represents the structural element of the formula Ib

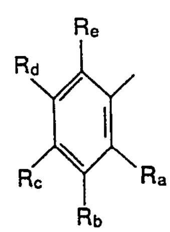 (Ib)

in which one of the radicals $R_a$ and $R_c$ represents the radical R which represents lower alkoxy or halogen, and the other represents hydrogen, lower alkyl, halogen or lower alkylamino, and each of the radicals $R_b$, $R_d$ and $R_e$, independently of the others, represents hydrogen, lower alkyl, halo-lower alkyl, hydroxy, halogen, lower alkoxy, cyano, carbamoyl, lower alkoxycarbonyl, amino, sulphamoyl, N,N-di-lower alkylsulphamoyl, lower alkylthio, lower alkanesulphinyl and/or lower alkanesulphonyl,
and in which alk represents alkylene that has from 2 up to and including 4 carbon atoms and separates the two N atoms by 2 or 3 carbon atoms, X represents carbonyl, hydroxymethylene or methylene and $Ar_2$ represents phenyl substituted by halogen, or unsubstituted thienyl, and salts thereof, are manufactured.

6. Process according to claim 1, characterised in that compounds of the formula I in which the grouping R–$Ar_1$ represents the structural element of the formula Ib according to claim 5 in which, on the one hand, the radical $R_a$ represents the radical R which represents lower alkoxy having up to and including 4 carbon atoms and $R_c$ represents hydrogen, halogen having an atomic number of up to and including 35 or lower alkylamino having up to and including 4 carbon atoms, or $R_a$ represents hydrogen and $R_c$ represents the radical R which represents lower alkoxy having up to and including 4 carbon atoms, or in which, on the other hand, the radical $R_c$ represents the radical R which represents halogen having an atomic number of up to and including 35 and $R_a$ represents hydrogen or halogen having an atomic number of up to and including 35 and one of the radicals $R_b$ and $R_d$ represents hydrogen, lower alkyl having up to and including 4 carbon atoms, halo-lower alkyl having an atomic number of up to and including 35 and having up to and including 4 carbon atoms, halogen having an atomic number of up to and including 35, lower alkoxy having up to and including 4 carbon atoms, cyano, carbamoyl, lower alkoxycarbonyl having from 2 up to and including 5 carbon atoms, lower alkylthio having up to and including 4 carbon atoms, lower alkanesulphinyl having up to and including 4 carbon atoms or lower alkanesulphonyl having up to and including 4 carbon atoms, and the other and $R_e$ represent hydrogen, and in which in each case alk has the definition given in claim 5, X represents carbonyl or hydroxymethylene and $Ar_2$ represents phenyl substituted in the p-position by halogen having an atomic number of up to and including 35, and salts thereof, are manufactured.

7. Process according to claim 1, characterised in that compounds of the formula I in which $Ar_1$ represents, on the one hand, phenylene which is mono- or poly-substituted by halogen having an atomic number of up to and including, cyano and/or by N-lower alkylamino having up to and including 4 carbon atoms in the lower alkyl moiety, or, on the other hand, pyridylene, and in each case R represents lower alkoxy having up to and including 4 carbon atoms, alk represents ethylene, X represents carbonyl and $Ar_2$ represents phenyl substituted by halogen having an atomic number of up to and including 35, and salts thereof, are manufactured.

8. Process according to claim 1, characterised in that compounds of the formula I in which the grouping R–$Ar_1$– represents the structural element of the formula Ib according to claim 5 in which, on the one hand, $R_a$ represents the radical R which represents lower alkoxy having up to and including 4 carbon atoms, $R_b$ and $R_e$ represent hydrogen, $R_c$ represents lower alkylamino having up to and including 4 carbon atoms and $R_d$ represents halogen having an atomic number of up to and including 35, or $R_c$ represents hydrogen or halogen having an atomic number of up to and including 35 and $R_d$ represents cyano, or in which, on the other hand, $R_a$, $R_d$ and $R_e$ represent hydrogen, $R_b$ represents halogen having an atomic number of up to and including 35 and $R_c$ represents the radical R which represents lower alkoxy having up to and including 4 carbon atoms, and in each case alk represents ethylene, X represents carbonyl or hydroxymethylene and $Ar_2$ represents 4-fluorophenyl, and salts thereof, are manufactured.

9. Process according to claim 1, characterised in that compounds of the formula I in which the grouping R–$Ar_1$– represents the structural element of the formula Ib according to claim 5 in which one of the radicals $R_a$ and $R_b$ represents hydrogen or halogen having an atomic number of up to and including 35, and the other represents hydrogen, $R_c$ represents the radical R which represents halogen having an atomic number of up to and including 35 and each of $R_d$ and $R_e$ represents hydrogen, and alk represents ethylene, X represents carbonyl and $Ar_2$ represents p-fluorophenyl, and salts thereof, are manufactured.

10. Process according to claim 1, characterised in that compounds of the formula I in which the grouping R–$Ar_1$– represents the structural element of the formula Ib according to claim 5 in which $R_a$, $R_d$ and $R_e$ represent hydrogen, $R_b$ represents hydrogen or halogen having an atomic number of up to and including 35, and $R_c$ represents the radical R which represents halogen having an atomic number of up to and including 35, alk represents ethylene, X represents carbonyl and $Ar_2$ represents 4-fluorophenyl, and salts thereof, are manufactured.

11. Process according to claim 1, characterised in that compounds of the formula I in which the grouping R–$Ar_1$– represents the structural element of the formula Ib according to claim 5 in which $R_a$ represents the radical R which represents lower alkoxy having up to and including 4 carbon atoms, $R_b$, $R_c$ and $R_e$ represent hydrogen and $R_d$ represents cyano, alk represents ethylene, X represents carbonyl and $Ar_2$ represents 4-fluorophenyl, and salts thereof, are manufactured.

12. Process according to claim 1, characterised in that
5-cyano-N-[2-[4-(p-fluorobenzoyl)-piperidinyl]-ethyl]-2-methoxy-benzamide

or a salt thereof is manufactured.

13. Process according to claim 1, characterised in that
4-chloro-5-cyano-N-[2-[4-(p-fluorobenzoyl)-piperidinyl]-ethyl]-2-methoxy-benzamide
or a salt thereof is manufactured.

14. Process according to claim 1, characterised in that
5-bromo-4-chloro-N-[2-[4-(p-fluorobenzoyl)-piperidinyl]-ethyl]-2-methoxy-benzamide,
N-[2-[4-(p-fluorobenzoyl)-piperidinyl]-ethyl]-4-fluoro-2-methoxy-benzamide,
5-bromo-N-[2-[4-(p-fluorobenzoyl)-piperidinyl]-ethyl]-4-fluoro-2-methoxy-benzamide,
N-[2-[4-(p-fluorobenzoyl)-piperidinyl]-ethyl]-2-methoxy-5-trifluoromethyl-benzamide,
2-methoxy-5-sulphamoyl-N-[2-[4-(p-flurobenzoyl)-piperidinyl]-ethyl]-benzamide,
5-chloro-N-[2-[4-(p-fluorobenzoyl)-piperidinyl]-ethyl]-2-methoxy-4-methylaminosulphamoyl-benzamide,
N-[2-[4-(p-fluorobenzoyl)-piperidinyl]-ethyl]-2-methoxy-5-dimethylsulphamoyl-benzamide,
3-bromo-N-[2-[4-(p-fluorobenzoyl)-piperidinyl]-ethyl]-4-methoxy-benzamide,
N-[2-[4-(p-fluorobenzoyl)-piperidinyl]-ethyl]-2-methoxy-benzamide,
5-cyano-N-[2-[4-[(4-fluorophenyl)-hydroxymethylene]-piperidinyl]-ethyl]-2-methoxy-benzamide,
or a salt thereof, is manufactured.

15. Process according to claim 1, characterised in that
3-bromo-4-fluoro-N-[2-[4-(p-fluorobenzoyl)-piperidinyl]-ethyl]-benzamide,
2-bromo-4-fluoro-N-[2-[4-(p-fluorobenzoyl)-piperidinyl]-ethyl]-benzamide,
N-[2-[4-(p-fluorobenzoyl)-piperidinyl]-ethyl]-2,6-dimethoxy-benzamide,
4-fluoro-N-[2-[4-(p-fluorobenzoyl)-piperidinyl]-ethyl]-benzamide,
5-bromo-N-[2-[4-(p-fluorobenzoyl)-piperidinyl]-ethyl]-2-methoxy-benzamide,
3-cyano-N-[2-[4-(p-fluorobenzoyl)-piperidinyl]-ethyl]-4-methoxy-benzamide,
or a salt thereof, is manufactured.

16. Process according to claim 1, characterised in that
3,5-dichloro-N-[2-[4-(p-fluorobenzoyl)-piperidinyl]-ethyl]-2-methoxy-4-methyl-benzamide,
N-[2-[4-(p-fluorobenzoyl)-piperidinyl]-ethyl]-2-methoxy-5-methylmercapto-benzamide,
N-[2-[4-(p-fluorobenzoyl)-piperidinyl]-ethyl]-2-methoxy-5-methanesulphonyl-benzamide,
5-cyano-N-[2-[4-(p-fluorobenzyl)-piperidinyl]-ethyl]-2-methoxy-benzamide,
N-[2-[4-(p-fluorobenzoyl)-piperidinyl]-ethyl]-2-methoxy-5-methoxycarbonyl-benzamide,
5-carbamoyl-N-[2-[4-(p-fluorobenzoyl)-piperidinyl]-ethyl]-2-methoxy-benzamide,
N-[2-[4-(p-fluorobenzoyl)-piperidinyl]-ethyl]-2-methoxy-5-methanesulphinyl-benzamide,
5-cyano-2-methoxy-N-[2-[4-(2-thenoyl)-piperidinyl]-ethyl]-benzamide,
4-fluoro-N-[2-[4-(2-thenoyl)-piperidinyl]-ethyl]-benzamide,
N-[2-[4-(p-chlorobenzoyl)-piperidinyl]-ethyl]-5-cyano-2-methoxy-benzamide,
N-[2-[4-(p-chlorobenzoyl)-piperidinyl]-ethyl]-4-fluoro-benzamide,
4-fluoro-N-[3-[4-(p-fluorobenzoyl)-piperidinyl]-2-methylpropyl]-benzamide,
5-cyano-N-[3-[4-(p-fluorobenzoyl)-piperidinyl]-propyl]-2-methoxy-benzamide,
N-[2-[4-(p-fluorobenzoyl)-piperidinyl]-ethyl]-2,6-dimethoxynicotinic acid amide,
or a salt thereof, is manufactured.

17. Process for the manufacture of pharmaceutical preparations, characterised in that a compound according to any one of claims 1 to 16 is processed, optionally with the admixture of customary adjuncts and carriers, to form pharmaceutical preparations.

**Revendications pour les Etats contractants: BE, CH, DE, FR, GB, IT, LI, LU, NL, SE.**

1. N-(pipéridinyl-alcoyl)-carboxamides de formule générale (I)

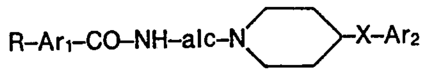
(I)

où

R signifie un hydroxy, alcoxy inférieur, alcényloxy inférieur ou halogène,

$Ar_1$ représente un phénylène non substitué ou mono- ou polysubstitué en outre par un alcoyle inférieur, alcényle inférieur, alcadiényle inférieur, halogènalcoyle inférieur, hydroxy, alcanoyloxy inférieur, halogène, alcoxy inférieur, alcényloxy inférieur, halogènalcoxy inférieur, alcanoyle inférieur, nitro, cyano-carbamoyle, N-alcoyle inférieur-carbamoyle, N,N-dialcoyle inférieur-carbamoyle, carboxy, alcoxy inférieur carbonyle, amino, N-alcoyle inférieur-amino, N,N-dialcoyle inférieur amino, sulfamoyle, N-alcoyle inférieur-sulfamoyle, N,N-dialcoyle inférieur-sulfamoyle, alcoyle inférieur-thio, halogènalcoyle inférieur-thio, alcane inférieur-sulfinyle, halogènalcane inférieur-sulfinyle, alcane inférieur-sulfonyle et/ou halogènalcane inférieur sulfonyle ou un azaarylène monocyclique lié par l'intermédiaire d'un atome de carbone et ayant jusqu'à et y compris 3 atomes d'azote, alc représente les alcoylènes séparant les 2 atomes de N par 2 à 3 atomes de C et ayant de 2 jusqu'à et y compris 7 atomes de carbone,

X représente un carbonyle, dialcoxy inférieur-méthylène, alcoylène inférieur-dioxyméthylène, hydroxyméthylène, alcanoyloxy inférieur-méthylène ou méthylène, et

$Ar_2$ représente un radical phényle non substitué ou mono- ou polysubstitué par un alcoyle inférieur, alcényle inférieur, alcadiényle inférieur, halogènalcoyle inférieur, hydroxy, alcanoyloxy inférieur, halogène, alcoxy inférieur, alcényloxy inférieur, halogènalcoxy inférieur, alcanoyle inférieur, nitro, cyano, carbamoyle, N-alcoyle inférieur-carbamoyle, N,N-dialcoyle inférieur-carbamoyle, carboxy, alcoxy inférieur carbonyle, amino, N-alcoyle inférieur-amino, N,N-dialcoyle inférieur-amino, sulfamoyle, N-alcoyle inférieur-sulfamoyle, N,N-dialcoyle inférieur sulfamoyle, alcoyle inférieur-thio, halogènalcoyle inférieur-thio, alcane inférieur-sufinyle, halogènalcane inférieur-sulfinyle, alcane inférieur-sulfonyle et/ou halogènalcane inférieur-sulfonyle, ou un radical monooxa-, monoaza- ou monothiaaryle monocyclique, et leurs sels, avec cette précision que le groupement R–$Ar_1$– est différent du radical de formule (Ia)

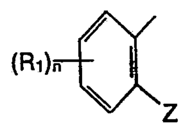

(Ia)

où $R_1$ est choisi dans le groupe constitué par alcoyle inférieur, trifluorométhyle, halogène, alcoxy inférieur, nitro et cyano et au moins l'un des radicaux $R_1$ représente un halogène ou un alcoxy inférieur, n représente un nombre entier allant de 1 à 3 et Z représente un nitro, amino ou alcoyle inférieur-amino lorsque alc et X ont les significations indiquées, et $Ar_2$ représente un phényle, thiényle ou pyridyle non substitué ou un phényle substitué par 1, 2 ou 3 substituants choisis dans le groupe constitué par halogène, alcoyle inférieur, alcoxy inférieur, trifluorométhyle et amino, où les radicaux décrits comme «inférieurs» doivent être compris comme ceux qui comportent jusqu'à 7 atomes de carbone compris.

2. Composés selon la revendication de formule (I) où R représente un alcoxy inférieur,

$Ar_1$ un phénylène ou pyridylène non substitué ou mono- ou polysubstitué en outre par un alcoyle inférieur, halogènalcoyle inférieur, halogène, alcoxy inférieur, cyano, carbamoyle, amino, N-alcoyle inférieur-amino, N,N-dialcoyle inférieur-amino, sulfamoyle, N,N-dialcoyle inférieur-sulfamoyle, halogènalcoyle inférieur-thio, alcane inférieur sulfonyle et/ou halogènalcane inférieur-sulfonyle, alc représente un alcoylène séparant les deux atomes de N par de 2 à 3 atomes de C et ayant 2 ou 3 atomes de carbone,

X représente un carbonyle, hydrométhylène ou méthylène, et $Ar_2$ représente un phényle substitué par un halogène, ou un thiényle non substitué et leurs sels.

3. Composés selon la revendication 1, de formule (I) où d'une part R représente un alcoxy inférieur ou un halogène, et $Ar_1$ un phénylène non substitué ou en outre mono- ou polysubstitué par un alcoyle inférieur, halogène alcoyle inférieur, hydroxy, halogène, alcoxy inférieur, cyano, carbamoyle, alcoxy inférieur carbonyle, amino, alcoyle inférieur amino, dialcoyle inférieur-amino, sulfamoyle, N,N-dialcoyle inférieur-sulfamoyle, alcoyle inférieur-thio, alcane inférieur-sulfinyle et/ou alcane inférieur-sulfonyle, ou où d'autre part,

R représente un alcoxy inférieur, et

Ar représente un pyridylène, non substitué ou substitué par un alcoxy inférieur, et

alc représente à chaque fois un alcoylène séparant les 2 atomes de N par de 2 à 3 atomes de C et ayant de 2 à 7 atomes de carbone compris,

X représente un carbonyle, hydroxyméthylène ou méthylène et $Ar_2$ représente un phényle substitué par un halogène ou un thiényle non substitué et leurs sels.

4. Composés selon la revendication 1 de formule (I), où R représente à chaque fois un alcoxy inférieur, $Ar_1$ d'une part un radical phényle non substitué ou mono- ou polysubstitué en outre par un halogènalcoyle inférieur, halogène, alcoxy inférieur, cyano, carbamoyle, N-alcoyle inférieur-amino, sulfamoyle, N,N-dialcoyle inférieur-sulfamoyle, halogènalcoyle inférieur-thio et/ou halogènalcane inférieur-sulfonyle, d'autre part, un radical pyridylène mono- ou polysubstitué en outre par un alcoxy inférieur et/ou un halogène,

alc représente un éthylène ou un 1,3-propylène,

X représente un carbonyle, hydroxyméthylène ou méthylène, et $Ar_2$ représente un radical phényle substitué par un halogène, et leurs sels.

5. Composés selon la revendication 1 de formule (I) où le groupement R–$Ar_1$ représente l'élément structural de formule (Ib)

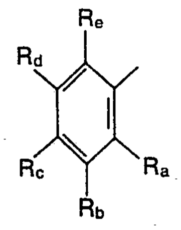

(Ib)

où l'un des radicaux $R_a$ et $R_c$ représente le radical R et celui-ci désigne un alcoxy inférieur ou un halogène et l'autre représente un hydrogène, alcoyle inférieur, halogène ou alcoyle inférieur-amino, et les radicaux $R_b$, $R_d$ et $R_e$ représentent indépendamment l'un de l'autre un hydrogène, alcoyle inférieur, halogènalcoyle inférieur, hydroxy-halogènalcoxy inférieur, cyano, carbamoyle, alcoxy inférieur-carbonyle, amino, sulfamoyle, N,N-dialcoyle inférieur-sulfamoyle, alcoyle inférieur-thio, alcane inférieur sulfinyle et/ou alcane inférieur sulfonyle et/ou où alc représente un alcoylène séparant les deux atomes de N par de 2 à 3 atomes de C et ayant jusqu'à 4 atomes de carbone compris,

X représente un carbonyle, hydroxyméthylène ou méthylène, et

$Ar_2$ représente un phényle substitué par un halogène ou un thiényle non substitué et leurs sels.

6. Composés selon la revendication 1 de formule (I) où le groupement R–$Ar_1$ représente l'élément de structure de formule (Ib) selon la revendication 5, où d'une par le radical $R_a$ représente le radical R et celui-ci un alcoxy inférieur ayant jusqu'à 4 atomes de carbone compris et $R_c$ représente un hydrogène, un halogène de numéro atomique allant jusqu'à 35 compris, ou un alcoyle inférieur-amino ayant jsuqu'à 4 atomes de carbone compris, ou bien où $R_a$ est un hydrogène et $R_c$ représente le radical R et celui-ci représente un alcoxy inférieur ayant jusqu'à 4 atomes de carbone compris, ou bien où dans lequel d'autre part le radical $R_c$ représente le radical R et celui-ci représente un halogène de numéro atomique allant jusqu'à 35

compris et $R_a$ représente un hydrogène ou un halogène de numéro atomique allant jusqu'à 35 compris, et l'un des radicaux $R_b$ et $R_d$ représente un hydrogène, alcoyle inférieur ayant jusqu'à 4 atomes de carbone compris, halogènalcoyle inférieur de numéro atomique allant jusqu'à 35 compris et ayant jusqu'à 4 atomes de carbone, un halogène de numéro atomique allant jusqu'à 35 compris, un alcoxy inférieur ayant jusqu'à 4 atomes de carbone compris, cyano, carbamoyle, alcoxy inférieur-carbonyle ayant de 2 à 5 atomes de carbone compris, alcoyle inférieur-thio ayant jusqu'à 4 atomes de carbone compris, alcane inférieur sulfinyle ayant jusqu'à 4 atomes de carbone compris, ou alcane inférieur sulfonyle ayant jusqu'à 4 atomes de carbone compris, et l'autre, ainsi que $R_e$ représentent un hydrogène, et/ou à chaque fois alc est défini comme il est dit dans la revendication 5, X représente un carbonyle ou un hydroxyméthylène et $Ar_2$ représente un phényle substitué en position p par un halogène de numéro atomique allant jusqu'à 35 compris, et leurs sels.

7. Composés selon la revendication 1 de formule (I), où $Ar_1$ représente d'une part un phénylène mono- ou polysubstitué en outre par un halogène de numéro atomique allant jusqu'à 35 compris, un cyano et/ou N-alcoyle inférieur-amino ayant jusqu'à 4 atomes de carbone compris dans la partie alcoyle inférieur ou représente d'autre par un pyridylène, et à chaque fois R représente un alcoxy inférieur ayant jusqu'à 4 atomes de carbone compris, alc est un éthylène, X représente un carbonyle, et $Ar_2$ représente un phényle substitué par un halogène de numéro atomique allant jusqu'à 35 compris.

8. Composés selon la revendication 1, de formule (I), où le groupement R–$Ar_1$– représente l'élément de structure de formule (Ib) selon la revendication 5, où $R_a$ représente le radical et celui-ci représente un alcoxy inférieur ayant jusqu'à 4 atomes de carbone compris et $R_b$ et $R_e$ représentent un hydrogène, $R_c$ représente un alcoyle inférieur-amino ayant jusqu'à 4 atomes de carbone compris, et $R_d$ représente un halogène de numéro atomique allant jusqu'à 35 compris ou $R_c$ représente un hydrogène ou un halogène de numéro atomique allant jusqu'à 35 compris et $R_d$ est un cyano, ou bien où d'autre part, $R_a$, $R_d$ et $R_e$ représentent un hydrogène, $R_b$ représente un halogène de numéro atomique allant jusqu'à 35 compris et $R_c$ représente le radical R et celui-ci représente un alcoxy inférieur ayant jusqu'à 4 atomes de carbone compris, et à chaque fois alc représente un éthylène, X représente un carbonyle ou un hydroxyméthylène, et $Ar_2$ représente un 4-fluorophényle et leurs sels.

9. Composés selon la revendication 1, de formule (I) où le groupement R–$Ar_1$– représente l'élément structural de formule (Ib) où l'un des radicaux $R_a$ et $R_b$ représente un hydrogène ou un halogène de numéro atomique allant jusqu'à 35 compris, et l'autre représente un hydrogène, $R_c$ représente le radical R et celui-ci représente un halogène de numéro atomique allant jusqu'à 35 compris et $R_d$ et $R_e$ représente à chaque fois un hydrogène, et alc est un éthylène, X représente un carbonyle et $Ar_2$ représente un p-fluorophényle, et leurs sels.

10. Composés selon la revendication 1, de formule (I) où le groupement R–$Ar_1$– représente l'élément structural de formule (Ib) où $R_a$, $R_d$ et $R_e$ représentent un hydrogène, $R_b$ représente un hydrogène ou un halogène de numéro atomique allant jusqu'à 35 compris et $R_c$ représente le radical R et celui-ci un halogène de numéro atomique allant jusqu'à 35 compris, alc représente un éthylène, X représente un carbonyle et $Ar_2$ représente un 4-fluorophényle, et leurs sels.

11. Composés selon la revendication 1, de formule (I), où le groupement R–$Ar_1$– représente l'élément structurel de formule (Ib), où $R_a$ représente le radical R et celui-ci représente un alcoxy inférieur ayant jusqu'à 4 atomes de carbone compris, $R_b$, $R_c$ et $R_e$ représentent un hydrogène et $R_d$ est un cyano, alc représente un éthylène, X représente un carbonyle et $Ar_2$ représente un 4-fluorophényl, et leurs sels.

12. 5-cyano-N-[2-[4-(p-fluorobenzoyl)-pipéridinyl]-éthyl]-2-méthoxybenzamide
ou un de ses sels.

13. 4-chloro-5-cyano-N-[2-[4-(p-fluorobenzoyl)-pipéridinyl]-éthyl]-2-méthoxy-benzamide
ou un de ses sels.

14. 5-bromo-4-chloro-N-[-2[4-(p-fluorobenzoyl)-pipéridinyl]-éthyl]-2-méthoxy-benzamide
ou un de ses sels.
N-[2-[4-(p-fluorobenzoyl)-pipéridinyl]-éthyl]-4-fluoro-2-méthoxy-benzamine
ou un de ses sels,
5-bromo-N-[2-[4-(p-fluorobenzoyl)-pipéridinyl]-éthyl]-4-fluoro-2-méthoxy-benzamide
ou un de ses sels.
N-[2-[4-(p-fluorobenzoyl)-pipéridinyl]-éthyl]-2-méthoxy-5-trifluoro-méthyl-benzamide
ou un de ses sels,
2-méthoxy-5-sulfamoyl-N-[2-[4-(p-fluoro-benzoyl)-pipéridinyl]-éthyl]-benzamide
ou un de ses sels,
5-chloro-N-[2-[4-(p-fluorobenzoyl)-pipéridinyl]-éthyl]-2-méthoxy-4-méthylamino-sulfamoyl-benzamide
ou un de ses sels,
N-[2-[4-(p-fluorobenzoyl)-pipéridinyl]-éthyl]-2-méthoxy-5-diméthylsulfamoyl-benzamide
ou un de ses sels,
3-bromo-N-[2-[4-(p-fluorobenzoyl)-pipéridinyl]-éthyl]-4-méthoxy-benzamide
ou un de ses sels,
N-[2-[4-(p-fluorobenzoyl)-pipéridinyl]-éthyl]-2-méthoxy-benzamide
ou un de ses sels,
5-cyano-N-[2-[4-[(4-(p-fluorophényl]-hydroxyméthylène]-pipéridinyl]-éthyl]-2-méthoxy-benzamide
ou un de ses sels.

15. 3-bromo-4-fluoro-N-[2-[4-(p-fluorobenzoyl)-pipéridinyl]-éthyl]-benzamide
ou un de ses sels,
2-bromo-4-fluoro-N-[2-[4-(p-fluorobenzoyl)-pipéridinyl]-éthyl]-benzamide
ou un de ses sels,
N-[2-[4-(p-fluorobenzoyl)-pipéridinyl]-éthyl]-2,6-diméthoxy-benzamide
ou un de ses sels,
4-fluoro-N-[2-[4-(p-fluorobenzoyl)-pipéridinyl]-éthyl]-benzamide
ou un de ses sels,
5-bromo-N-[2-[4-(p-fluorobenzoyl)-pipéridinyl]-éthyl]-2-méthoxy-benzamide
ou un de ses sels,
3-cyano-N-[2-[4-(p-fluorobenzoyl)-pipéridinyl]-éthyl]-4-méthoxy-benzamide
ou un de ses sels.

16. 3,5-dichloro-N-[2-[4-(p-fluorobenzoyl)-pipéridinyl]-éthyl]-2-méthoxy-4-méthyl-benzamide
ou un de ses sels,
N-[2-[4-(p-fluorobenzoyl)-pipéridinyl]-éthyl]-2-méthoxy-5-méthyl-mercapto-benzamide
ou un de ses sels,
N-[2-[4-(p-fluorobenzoyl)-pipéridinyl]-éthyl]-2-méthoxy-5-méthanesulfonyl-benzamide
ou un de ses sels,
5-cyano-N-[2-[4-(p-fluorobenzyl)-pipéridinyl]-éthyl]-2-méthoxy-benzamide
ou un de ses sels,
N-[2-[4-(p-fluorobenzoyl)-pipéridinyl]-éthyl]-2-méthoxy-5-méthoxycarbonyl-benzamide
ou un de ses sels,
5-carbamoyl-N-[2-[4-(p-fluorobenzoyl)-pipéridinyl]-éthyl-2-méthoxy-benzamide
ou un de ses sels,
N-[2-[4-(p-fluorobenzoyl)-pipéridinyl]-éthyl]-2-méthoxy-5-méthane, sulfinyl-benzamide
ou un de ses sels,
5-cyano-2-méthoxy-N-[2-[4-(2-thénoyl)-pipéridinyl]-éthyl]-benzamide
ou un de ses sels,
4-fluoro-N-[2-[4-(2-thénoyl)-pipéridinyl]-éthyl]-benzamide
ou un de ses sels,
N-[2-[4-(p-chlorobenzoyl)-pipéridinyl]-éthyl]-5-cyano-2-méthoxy-benzamide
ou un de ses sels,
N-[2-[4-(p-chlorobenzoyl)-pipéridinyl]-éthyl-4-fluoro-benzamide
ou un de ses sels,
4-fluoro-N-[3-[4-(p-fluorobenzoyl)-pipéridinyl]-2-méthylpropyl]-benzamide
ou un de ses sels,
5-cyano-N-[3-[4-(p-fluorobenzoyl)-pipéridinyl]-propyl]-2-méthoxy-benzamide
ou un de ses sels,
Amide de l'acide N-[2-[4-(p-fluorobenzoyl)-pipéridinyl]-éthyl-2,6-diméthoxynicotinique
ou un de ses sels.

17. Composés aux fins d'application dans le traitement prophylactique ou thérapeutique du corps humain ou animal de formule générale (I)

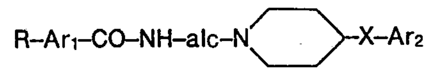 (I)

où R, $Ar_1$, alc, X et $Ar_2$ ont à chaque fois l'une des significations données dans les revendications 2 à 11, y compris les composés de formule (I) où le groupement $R–Ar_1–$ représente le radical de formule

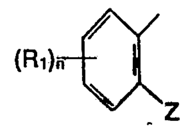 (Ia)

où $R_1$ est choisi dans le groupe constitué par alcoyle inférieur, trifluorométhyle, halogène, alcoxy inférieur, nitro et cyano, au moins l'un des radicaux $R_1$ représente un halogène ou un alcoxy inférieur, n représente un nombre entier allant de 1 à 3 et Z représente un nitro, amino, ou alcoyle inférieur amino, alc et X ont les significations indiquées et $Ar_2$ représente un phényle, thiényle ou pyridyle non substitué ou un phényle substitué par 1, 2 ou 3 substituants choisis dans le groupe constitué par halogène, alcoyle inférieur, alcoxy inférieur, trifluorométhyle et amino où les radicaux décrits comme «inférieurs» doivent comprendre ceux qui comportent jusqu'à 7 atomes de carbone compris, ou un sel pharmaceutiquement acceptable de ces corps.

18. Composés selon l'une des revendications 12 à 16 où un sel pharmaceutiquement acceptable de ce corps aux fins d'applications dans le traitement prophylactique ou thérapeutique du corps humain ou animal.

19. Composés selon l'une des revendications 17 à 18 de formule (I) aux fins d'application comme antipsychotiques.

20. Préparations pharmaceutiques contenant un composé de formule (I) selon l'une des revendications 17 à 18.

21. Application de composés de formule (I) selon l'une des revendications 17 à 18 aux fins de préparation d'antipsychotiques.

22. Procédé de préparation de N-(pipéridinyl-alcoyl)-carboxamides, selon la revendication 1 et de leurs sels, caractérisé en ce que

a) on condense un composé de formule (IIa)

$$R–Ar_1–X_1 \qquad (IIa)$$

où $X_1$ représente un carboxy ou un carboxy réactif fonctionnellement modifié, avec un composé de formule (IIb)

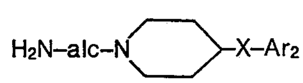 (IIb)

ou un de ses sels, ou

b) on condense de façon intermoléculaire des composés de formule (IIIa) et (IIIb)

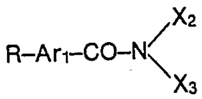

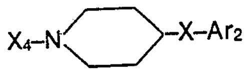

(IIIa) (IIIb)

où $X_2$ représente un hydrogène, l'un des radicaux $X_3$ et $X_4$ représente un hydrogène et l'autre radical représente un groupe de formule –alc–$X_5$ et $X_5$ représente un hydroxy estérifié réactif, des composés de formules (IIIa) et (IIIb) où $X_2$ et $X_3$ représentent ensemble alc′ ou alc′ représente un alcoylene à 2 ou 3 chaînons comportant de 2 à 7 atomes de carbone compris, et $X_4$ est un hydrogène ou leurs sels, ou

c) on condense de façon intramoléculaire un composé de formule (IIIc)

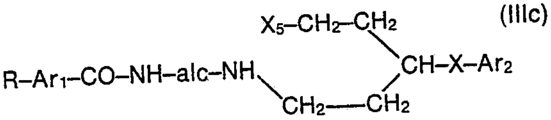

(IIIc)

où $X_5$ représente un hydroxy estérifié réactif ou

d) on fait réagir un composé de formule (IVa)

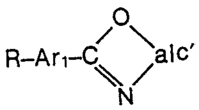

(IVa)

où alc′ représente un groupe alcoylène séparant l'atome de carbone et l'atome d'azote par de 2 à 3 atomes de carbone et comportant de 2 à 7 atomes de carbone compris, avec un composé de formule (IVb)

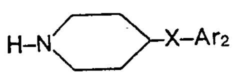

(IVb)

ou

e) dans un composé de formule (V)

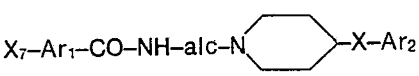

(V)

ou un de ses sels où $X_7$ représente un radical transformable en R, on transforme $X_7$ en R, et si on le désire, on transforme un composé que l'on peut obtenir selon le procédé ou d'une autre manière en un autre composé selon l'invention et/ou on transforme un sel que l'on peut obtenir selon le procédé en le composé libre ou en un autre sel, et/ou on transforme un composé libre que l'on peut obtenir selon le procédé et ayant des propriétés salificatrices en un sel et/ou on sépare un mélange d'isomères que l'on peut obtenir selon le procédé pour obtenir les isomères isolés.

23. Procédé de préparation de préparations pharmaceutiques caractérisé en ce qu'on transforme un composé selon l'une des revendications 17 à 19, éventuellement en ajoutant au mélange des additifs et supports habituels, en préparations pharmaceutiques.

**Revendications pour l'Etat contractant: AT**

1. Procédé préparation de N-(pipéridinyl-alcoyl-carboxamides de formule générale (I)

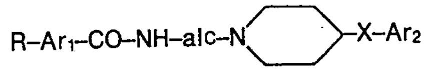

(I)

où

R signifie un hydroxy, alcoxy inférieur, alcénYloxy inférieur ou halogène,

$Ar_1$ représente un phénylène non substitué ou mono- ou polysubstitué en outre par un alcoyle inférieur, alcényle inférieur, alcadiényle inférieur, halogènalcoyle inférieur, hydroxy, alcanoyloxy inférieur, halogène alcoxy inférieur, alcényloxy inférieur, halogènalcoxy inférieur, alcanoyle inférieur, nitro, cyano carbamoyle, N-alcoyle inférieur-carbamoyle, N,N-dialcoyle inférieur-carbamoyle, carboxy, alcoxy inférieur-carbonyle, amino, N-alcoyle inférieur-amino, N,N-dialcoyle inférieur-amino, sulfamoyle, N-alcoyle inférieur-sulfamoyle, N-N-dialcoyle inférieur-sulfamoyle, alcoyle inférieur-thio, halogènalcoyle inférieur-thio, alcane inférieur-sulfinyle, alcane inférieur-sulfonyle et/ou halogènalcane inférieur sulfonyle ou un azaarylène monocyclique lié par l'intermédiaire d'un atome de carbone et ayant jusqu'à et y compris 3 atomes d'azote, alc représente les alcoylènes séparant les 2 atomes de N par 2 à 3 atomes de C et ayant de 2 jusqu'à et y compris 7 atomes de carbone,

X représente un carbonyle, dialcoxy inférieur-méthylène, alcoylène inférieur-dioxyméthylène, hydroxyméthylène, alcanoyloxy inférieur-méthylène ou méthylène, et

$Ar_2$ représente un radical phényle non substitué ou mono- ou polysubstitué par un alcoyle inférieur, alcényle inférieur, alcadiényle inférieur, halogènalcoyle inférieur, hydroxy, alcanoyloxy inférieur, halogène, alcoxy inférieur, alcényloxy inférieur, halogènalcoxy inférieur, alcanoyle inférieur, nitro, cyano, carbamoyle, N-alcoyle inférieur-carbamoyle, N,N-dialcoyle inférieur-carbamoyle, carboxy, alcoxy inférieur-carbonyle, amino, N-alcoyle inférieur-amino, N,N-dialcoyle inférieur-amino, sulfamoyle, N-alcoyle inférieur-sulfamoyle, N,N-dialcoyle inférieur-sulfamoyle, alcoyle inférieur-thio, halogènalcoyle inférieur-thio, alcane inférieur-sulfinyle, halogènalcane inférieur-sulfinyle, alcane inférieur-sulfonyle et/ou halogènalcane inférieur-sulfonyle, ou un radical monooxa-, monoaza- ou monothiaaryle monocyclique, et leurs sels, avec cette précision que le groupement R–$Ar_1$ est différent du radical de formule (Ia)

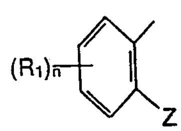

(Ia)

où $R_1$ est choisi dans le groupe constitué par alcoyle inférieur, trifluorométhyle, halogène, alcoxy inférieur, nitro et cyano et au moins l'un des radicaux $R_1$ représente un halogène ou un alcoxy inférieur, n représente un nombre entier allant de

1 à 3 et Z représente un nitro, amino ou alcoyle inférieur-amino lorsque alc et X ont les significations indiquées, et $Ar_2$ représente un phényle, thiényle ou pyridyle non substitué ou un phényle substitué par 1, 2 ou 3 substituants choisis dans le groupe constitué par halogène, alcoyle inférieur, alcoxy inférieur, trifluorométhyle et amino, où les radicaux décrits comme «inférieurs» doivent être compris comme ceux qui comportent jusqu'à 7 atomes de carbone compris

caractérisé en ce que

a) on condense un composé de formule (IIa)

$$R\text{–}Ar_1\text{–}X_1 \qquad (IIa)$$

où $X_1$ représente un carboxy ou un carboxy réactif fonctionnellement modifié, avec un composé de formule (IIb)

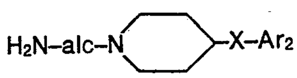 (IIb)

ou un de ses sels, ou

b) on condense de façon intermoléculaire des composés de formule (IIIa) et (IIIb)

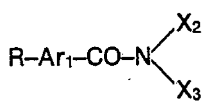 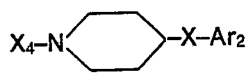

(IIIa) (IIIb)

où $X_2$ représente un hydrogène, l'un des radicaux $X_3$ et $X_4$ représente un hydrogène et l'autre radical représente un groupe de formule –alc–$X_5$ et $X_5$ représente un hydroxy estérifié réactif, des composés de formules (IIIa) et (IIIb) où $X_2$ et $X_3$ représentent ensemble alc′ ou alc′ représente un alcoylène à 2 ou 3 chaînons comportant de 2 à 7 atomes de carbone compris, et $X_4$ est un hydrogène ou leurs sels, ou

c) on condense de facon intramoléculaire un composé de formule (IIIc)

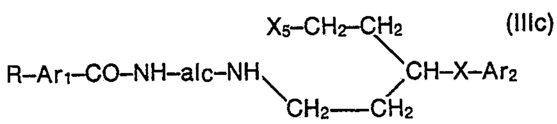 (IIIc)

où $X_5$ représente un hydroxy estérifié réactif ou

d) on fait réagir un composé de formule (IVa)

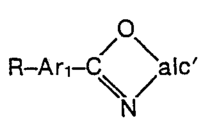 (IVa)

où alc′ représente un groupe alcoylène séparant l'atome de carbone et l'atome d'azote par de 2 à 3 atomes de carbone et comportant de 2 à 7 atomes de carbone compris, avec un composé de formule (IVb)

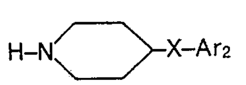 (IVb)

e) dans un composé de formule (V)

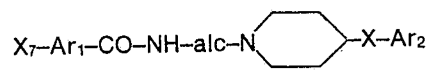 (V)

ou un de ses sels où $X_7$ représente un radical transformable en R, on transforme $X_7$ en R, et si on le désire, on transforme un composé que l'on peut obtenir selon le procédé ou d'une autre manière en un autre composé selon l'invention et/ou on transforme un sel que l'on peut obtenir selon le procédé en le composé libre ou en un autre sel, et/ou on transforme un composé libre que l'on peut obtenir selon le procédé et ayant des propriétés salificatrices en un sel et/ou on sépare un mélange d'isomères que l'on peut obtenir selon le procédé pour obtenir les isomères isolés.

2. Procédé selon la revendication 1, caractérisé en ce qu'on prépare des composés de formule (I) où R représente un alcoxy inférieur, $Ar_1$ représente un phénylène ou pyridylène non substitué ou mono- ou polysubstitué en outre par un alcoyle inférieur, halogènalcoyle inférieur, halogène, alcoxy inférieur, cyano, carbamoyle, amino, N-alcoyle inférieur-amino, N,N-dialcoyle inférieur-amino, sulfamoyle, N,N-dialcoyle inférieur sulfamoyle, halogènalcoyle inférieur-thio, alcane inférieur-sulfonyle et/ou halogène-alcane inférieur sulfonyle, alc représente un alcoylène séparant les 2 atomes de N par de 2 à 3 atomes de C et ayant 2 ou 3 atomes de carbone, X représente un carbonyle, hydroxyméthylène ou méthylène ou méthylène et $Ar_2$ représente un phényle substitué par un halogène ou un thiényl non substitué et leurs sels.

3. Procédé selon la revendication 1, caractérisé en ce qu'on prépare des composés de formule (I) où d'une part R représente un alcoxy inférieur ou halogène et $Ar_1$ représente un phénylène non substitué ou en outre mono- ou polysubstitué par un alcoyle inférieur, halogènalcoyle inférieur, hydroxy, halogène, alcoxy inférieur, cyano, carbamoyle, alcoxy inférieur-carbonyle, amino, alcoyle inférieur-amino, dialcoyle inférieur-amino, sulfamoyle, N,N-dialcoyle inférieur-sulfamoyle, alcoyle inférieur-thio, alcane inférieur sulfinyle et/ou alcane inférieur-sulfonyle, ou bien où R représente un alcoxy inférieur et $Ar_1$ représente un pyridylène non substitué ou substitué par un alcoxy inférieur, et à chaque fois alc représente un alcoylène séparant les 2 atomes de N par de 2 à 3 atomes de C et ayant de 2 à 7 atomes de carbone compris, X représente un carbonyle, hydroxyméthylène ou méthylène et $Ar_2$ représente un phényle substitué par un halogène ou un thiényle non substitué, et leurs sels.

4. Procédé selon la revendication 1, caractérisé en ce qu'on prépare des composés de formule (I) où

R représente à chaque fois un alcoxy inférieur,

$Ar_1$ représente d'une part un radical phényle non substitué ou mono- ou polysubstitué en outre par un halogènalcoyle inférieur, halogène, alcoxy inférieur, cyano carbamoyle, N-alcoyle inférieur-amino, sulfamoyle, N,N-dialcoyle inférieur sulfamoyle, halogènalcoyle inférieur-thio et/ou halogène alcane inférieur-sulfonyle, d'autre part un radical pyridylène mono- ou polysubstitué en outre par un alcoxy inférieur et/ou un halogène, alc représente un éthylène, ou un 1,3-propylène, X représente un carbonyle, hydroxyméthylène ou méthylène, et

$Ar_2$ représente un radical phényle substitué par un halogène, et leurs sels.

5. Procédé selon la revendication 1, caractérisé en ce qu'on prépare des composés de formule (I) où le groupement R–$Ar_1$ représente l'élément structurel de formule (Ib)

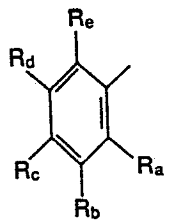

(Ib)

où l'un des radicaux $R_a$ et $R_c$ représentent le radical R et celui-ci représente un alcoxy inférieur ou un halogène et l'autre représente un hydrogène, alcoyle inférieur, halogène ou alcoyle inférieur-amino, et les radicaux $R_b$, $R_d$ et $R_e$ représentent indépendamment l'un de l'autre un hydrogène, alcoyle inférieur, halogènalcoyle inférieur, hydroxy, halogène, alcoxy inférieur, cyano, carbamoyle, alcoxy inférieur carbonyle, amino, sulfamoyle, N,N-dialcoyle inférieur-sulfamoyle, alcoyle inférieur-thio, alcane inférieur sulfinyle, et/ou alcane inférieur sulfonyle, et où alc représente un alcoylène séparant les 2 atomes de N par de 2 à 3 atomes de C et ayant de 2 à 4 atomes de carbone compris, X représente un carbonyle, hydroxyméthylène ou méthylène, et $Ar_2$ représente un phényle substitué par un halogène ou un thiényle non substitué, et leurs sels.

6. Procédé selon la revendication 1, caractérisé en ce qu'on prépare des composés de formule (I) où le groupement R–$Ar_1$– représente l'élément structurel de formule (Ib) selon la revendication 5, où d'une part le radical $R_a$ représente le radical R et celui-ci représente un alcoxy inférieur ayant jusqu'à 4 atomes de carbone compris, et $R_c$ représente un hydrogène, halogène de numéro atomique allant jusqu'à 35 compris ou alcoyle inférieur-amino ayant jusqu'à 4 atomes de carbone compris, ou bien où $R_a$ est un hydrogène et $R_c$ représente le radical R et celui-ci représente un alcoxy ayant jusqu'à 4 atomes de carbone compris ou bien où d'autre part le radical $R_c$ représente le radical R et celui-ci représente un halogène de numéro atomique allant jusqu'à 35 compris et $R_a$ représente un hydrogène ou un halogène de numéro atomique allant jusqu'à 35 compris, et l'un des radicaux $R_b$ et $R_d$ représente un hydrogène, un alcoyle inférieur ayant jusqu'à 4 atomes de carbone compris, un halogènalcoyle inférieur de numéro atomique allant jusqu'à 35 compris et ayant jusqu'à 4 atomes de carbone, un halogène de numéro atomique allant jusqu'à 35 compris, un alcoxy inférieur ayant jusqu'à 4 atomes de carbone compris, un cyano, carbamoyle, alcoxy inférieur-carbonyle ayant de 2 à 5 atomes de carbone compris, un alcoyle inférieur-thio ayant jusqu'à 4 atomes de carbone compris, un alcane inférieur-sulfinyle ayant jusqu'à 4 atomes de carbone compris ou un alcane inférieur sulfonyle ayant jusqu'à 4 atomes de carbone compris, et l'autre ainsi que R représentent un hydrogène et où à chaque fois alc est défini selon la revendication 5, X représente un carbonyle ou un hydroxyméthylène et $Ar_2$ représente un phényle substitué en position p par un halogène de numéro atomique allant jusqu'à 35 compris et leurs sels.

7. Procédé selon la revendication 1, caractérisé en ce qu'on prépare des composés de formule (I) où $Ar_1$ représente d'une part un phénylène mono- ou polysubstitué en outre par un halogène de numéro atomique allant jusqu'à 35 compris, un cyano et/ou un N-alcoyle inférieur amino ayant jusqu'à 4 atomes de carbone compris dans la fraction alcoyle inférieur, ou d'autre part représente un pyridylène, et à chaque fois R représente un alcoxy inférieur ayant jusqu'à 4 atomes de carbone, alc est un éthylène X représente un carbonyle et $Ar_2$ représente un phényle substitué par un halogène de numéro atomique allant jusqu'à 35 compris, et leurs sels.

8. Procédé selon la revendication 1 caractérisé en ce qu'on prépare des composés de formule (I) où le groupement R–$Ar_1$– représente l'élément structurel de formule (Ib) selon la revendication 5, où d'une part $R_a$ représente le radical R et celui-ci représente un alcoxy inférieur ayant jusqu'à 4 atomes de carbone compris, $R_b$ et $R_e$ représentent un hydrogène, $R_c$ représente un alcoyle inférieur-amino ayant jusqu'à 4 atomes de carbone compris et $R_d$ représente un halogène de numéro atomique allant jusqu'à 35 compris ou bien $R_c$ représente un hydrogène ou un halogène ayant un numéro atomique allant jusqu'à 35 compris, et $R_d$ est un cyano ou bien où $R_a$, $R_d$ et $R_e$ représentent un hydrogène, $R_b$ représente un halogène de numéro atomique allant jusqu'à 35 compris et $R_c$ représente le radical R et celui-ci représente un alcoxy inférieur ayant jusqu'à 4 atomes de carbone compris, et à chaque fois alc représente un éthylène, X représente un carbonyle ou un hydroxyméthylène, et $Ar_2$ représente un 4-fluorophényle et leurs sels.

9. Procédé selon la revendication 1, caractérisé en ce qu'on prépare des composés de formule (I) où le groupement R–$Ar_1$– représente l'élément structurel de formule (Ib) où l'un des radicaux $R_a$ et $R_b$ représentent un hydrogène ou un halogène de numéro atomique allant jusqu'à 35 compris, et l'autre représente un hydrogène, $R_c$ représente le radical R et celui-ci représente un halogène de numéro atomique allant jusqu'à 35 compris et $R_d$ et $R_e$ représentent à chaque fois un hydrogène, et alc est un éthylène, X représente un carbonyle et $Ar_2$ représente un p-fluorophényle et leurs sels.

10. Procédé selon la revendication 1, caractérisé en ce qu'on prépare des composés de formule (I) où le groupement R–$Ar_1$– représente l'élément structurel de formule (Ib) où $R_a$, $R_b$ et $R_e$

représenten un hydrogène ou un halogène de numéro atomique allant jusqu'à 35 compris et $R_c$ représente le radical R et celui-ci représente un halogène de numéro atomique allant jusqu'à 35 compris, alc représente un éthylène, X représente un carbonyle et $Ar_2$ représente un 4-fluorophényle et leurs sels.

11. Procédé selon la revendication 1, caractérisé en ce qu'on prépare des composés de formule (I) où le groupement $R-Ar_1-$ représente l'élément structurel de formule (Ib) où $R_a$ représente un radical R et celui-ci représente un alcoxy inférieur ayant jusqu'à 4 atomes de carbone compris, $R_b$, $R_c$ et $R_e$ représentent un hydrogène et $R_d$ est un cyano, alc représente un éthylène, X représente un carbonyle et $Ar_2$ représente un 4-fluorophényle et leurs sels.

12. Procédé selon la revendication 1, caractérisé en ce qu'on prépare le
5-cyano-N-[2-[4-(p-fluorobenzoyl)-pipéridinyl]-éthyl]-2-méthoxy-benzamide
ou un de ses sels.

13. Procédé selon la revendication 1, caractérisé en ce qu'on prépare le
4-chloro-5-cyano-N-[2-[4-(p-fluorobenzoyl)-pipéridinyl]-éthyl]-2-méthoxy-benzamide
ou un de ses sels.

14. Procédé selon la revendication 1, caractérisé en ce qu'on prépare le
5-bromo-4-chloro-N-[2-[4-(p-fluorobenzoyl)-pipéridinyl]-éthyl]-2-méthoxy-benzamide
N-[2-[4-(p-fluorobenzoyl)-pipéridinyl]-éthyl]-4-fluoro-2-méthoxy-benzamide
5-bromo-N-[2-[4-(p-fluorobenzoyl)-pipéridinyl]-éthyl]-4-fluoro-2-méthoxy-benzamide,
N-[2-[4-(p-fluorobenzoyl)-pipéridinyl]-éthyl]-2-méthoxy-5-trifluoro-méthyl-benzamide,
2-méthoxy-5-sulfamoyl-N-[2-[4-(p-fluorobenzoyl)-pipéridinyl]-éthyl]-benzamide,
5-chloro-N-[2-[4-(p-fluorobenzoyl)-pipéridinyl]-éthyl]-2-méthoxy-4-méthylaminosulfamoyl-benzamide,
N-[2-[4-(p-fluorobenzoyl)-pipéridinyl]-éthyl]-2-méthoxy-5-diméthyl-sulfamoyl-benzamide,
3-bromo-N-[2-[4-(p-fluorobenzoyl)-pipéridinyl]-éthyl]-4-méthoxy-benzamide,
N-[2-[4-(p-fluorobenzoyl)-pipéridinyl]-éthyl]-2-méthoxy-benzamide,
5-cyano-N-[2-[4-(fluorophényl)-hydroxyméthylène]-pipéridinyl]-éthyl]-2-méthoxy-benzamide
ou un de leurs sels.

15. Procédé selon la revendication 1, caractérisé en ce qu'on prépare
3-bromo-4-fluoro-N-[2-[4-(p-fluorobenzoyl)-pipéridinyl]-éthyl]-benzamide,
2-bromo-4-fluoro-N-[2-[4-(p-fluorobenzoyl)-pipéridinyl]-éthylbenzamide,
N-[2-[4-(p-fluorobenzoyl)-pipéridinyl]-éthyl]-2,6-diméthoxy-benzamide,
4-fluoro-N-[2-[4-(p-fluorobenzoyl)-pipéridinyl]-éthyl]-benzamide,
5-bromo-N-[2-[4-(p-fluorobenzoyl)-pipéridinyl]-éthyl]-2-méthoxy-benzamide,
3-cyano-N-[2-[4-(p-fluorobenzoyl)-pipéridinyl]-éthyl]-4-méthoxy-benzamide
ou un de leurs sels.

16. Procédé selon la revendication 1, caractérisé en ce qu'on prépare
3,5-dichloro-N-[2-[4-(p-fluorobenzoyl)-pipéridinyl]-éthyl]-2-méthoxy-4-méthyl-benzamide,
N-[2-[4-(p-fluorobenzoyl)-pipéridinyl]-éthyl]-2-méthoxy-5-méthyl-mercapto-benzamide,
N-[2-[4-(p-fluorobenzoyl)-pipéridinyl]-éthyl]-2-méthoxy-5-méthane-sulfonyl-benzamide,
5-cyano-N-[2-[4-(p-fluorobenzyl)-pipéridinyl]-éthyl]-2-méthoxy-benzamide,
N-[2-[4-(p-fluorobenzoyl)-pipéridinyl]-éthyl)-2-méthoxy-5-méthoxycarbonyl-benzamide,
5-carbamoyl-N-[2-[4-(p-fluorobenzoyl)-pipéridinyl]-éthyl]-2-méthoxy-benzamide,
N-[2-[4-(p-fluorobenzoyl)-pipéridinyl]-éthyl]-2-méthoxy-5-méthane-sulfinyl-benzamide,
5-cyano-2-méthoxy-N-[2-[4-(2-thénoyl)-pipéridinyl]-éthyl]-benzamide,
4-fluoro-N-[2-[4-(2-thénoyl)-pipéridinyl]-éthyl]-benzamide,
N-[2-[4-(p-fluorobenzoyl)-pipéridinyl]-éthyl]-5-cyano-2-méthoxy-benzamide,
N-[2-[4-(p-chlorobenzoyl)-pipéridinyl]-éthyl]-4-fluoro-benzamide,
4-fluoro-N-[3-[4-(p-fluorobenzoyl)-pipéridinyl]-2-méthylpropyl]-benzamide,
5-cyano-N-[3-[4-(p-fluorobenzoyl)-pipéridinyl]-propyl]-2-méthoxy-benzamide,
Amide de l'acide N-[2-[4-(p-fluorobenzoyl)-pipéridinyl]-éthyl]-2,6-diméthoxynicotinique
ou leurs sels.

17. Procédé de préparation de préparations pharmaceutiques caractérisé en ce qu'on transforme un composé selon l'une des revendications 1–16 éventuellement en ajoutant au mélange des additifs et supports habituels, pour obtenir des préparations pharmaceutiques.